(19) Europäisches Patentamt / European Patent Office / Office européen des brevets

(11) EP 1 185 691 B1

(12) **EUROPEAN PATENT SPECIFICATION**

(45) Date of publication and mention of the grant of the patent:
**29.04.2009 Bulletin 2009/18**

(21) Application number: **00910392.0**

(22) Date of filing: **08.03.2000**

(51) Int Cl.:
***C12Q 1/68*** *(2006.01)* ***C12N 15/11*** *(2006.01)*
***C07K 14/22*** *(2006.01)*

(86) International application number:
**PCT/US2000/005928**

(87) International publication number:
**WO 2000/066791 (09.11.2000 Gazette 2000/45)**

(54) **NEISSERIA GENOMIC SEQUENCES AND METHODS OF THEIR USE**

GENOMISCHE SEQUENZEN VON NEISSERIA UND VERFAHREN ZU IHRER VERWENDUNG

SEQUENCES GENOMIQUES DE NEISSERIA ET PROCEDES D'UTILISATION CORRESPONDANTS

(84) Designated Contracting States:
**AT BE CH CY DE DK ES FI FR GB GR IE IT LI LU MC NL PT SE**

(30) Priority: **30.04.1999 US 132068 P**
**08.10.1999 PCT/US99/23573**
**28.02.2000 GB 0004695**

(43) Date of publication of application:
**13.03.2002 Bulletin 2002/11**

(60) Divisional application:
**05075284.9 / 1 605 061**

(73) Proprietors:
- **Novartis Vaccines and Diagnostics, Inc.**
  **Emeryville, CA 94608 (US)**
- **J. Craig Venter Institute, Inc.**
  **Rockville, MD 20850 (US)**

(72) Inventors:
- **PIZZA, Mariagrazia**
  **I-53100 Siena (IT)**
- **HICKEY, Erin**
  **Emeryville, CA 94608-2916 (US)**
- **PETERSON, Jeremy**
  **Emeryville, CA 94608-2916 (US)**
- **TETTELIN, Herve**
  **Emeryville, CA 94608-2916 (US)**
- **VENTER, J., Craig**
  **Emeryville, CA 94608-2916 (US)**
- **MASIGNANI, Vega**
  **I-53100 Siena (IT)**
- **GALEOTTI, Cesira**
  **I-53100 Siena (IT)**
- **MORA, Marirosa**
  **I-53100 Siena (IT)**
- **RATTI, Giulio**
  **I-53100 Siena (IT)**
- **SCARSELLI, Maria**
  **I-53100 Siena (IT)**
- **SCARLATO, Vincenzo**
  **I-53100 Siena (IT)**
- **RAPPUOLI, Rino**
  **I-53100 Siena (IT)**
- **FRAZER, Claire, M.**
  **Emeryville, CA 94608 (US)**
- **GRANDI, Guido**
  **I-53100 Siena (IT)**

(74) Representative: **Hallybone, Huw George et al**
**Carpmaels & Ransford**
**43-45 Bloomsbury Square**
**London WC1A 2RA (GB)**

(56) References cited:
**EP-A- 0 467 714** **WO-A-98/17805**

- **FLEISCHMANN R D ET AL: "WHOLE-GENOME RANDOM SEQUENCING AND ASSEMBLY OF HAEMOPHILUS INFLUENZAE RD" SCIENCE,US, AMERICAN ASSOCIATION FOR THE ADVANCEMENT OF SCIENCE,, vol. 269, no. 5223, 28 July 1995 (1995-07-28), pages 496-498,507-51, XP000517090 ISSN: 0036-8075**
- **TETTELIN H ET AL: "Complete genome sequence of Neisseria meningitidis serogroup B strain MC58 [see comments]." SCIENCE, (2000 MAR 10) 287 (5459) 1809-15., XP000914963**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PIZZA M ET AL: "Identification of vaccine candidates against serogroup B meningococcus by whole- genome sequencing [see comments]." SCIENCE, (2000 MAR 10) 287 (5459) 1816-20., XP000914964**
- **PARKHILL J ET AL: "Complete DNA sequence of a serogroup A strain of Neisseria meningitidis Z2491 [see comments]." NATURE, (2000 MAR 30) 404 (6777) 502-6., XP000918875**

## Description

This invention relates to methods of obtaining antigens and immunogens, the antigens and immunogens so obtained, and nucleic acids from the bacterial species: *Neisseria meningitidis.* In particular, it relates to genomic sequences from the bacterium; more particularly its "B" serogroup.

BACKGROUND

*Neisseria meningitidis* is a non-motile, gram negative diplococcus human pathogen. It colonizes the pharynx, causing meningitis and, occasionally, septicaemia in the absence of meningitis. It is closely related to *N. gonorrhoea,* although one feature that clearly differentiates meningococcus from gonococcus is the presence of a polysaccharide capsule that is present in all pathogenic meningococci.

*N. meningitidis* causes both endemic and epidemic disease. In the United States the attack rate is 0.6-1 per 100,000 persons per year, and it can be much greater during outbreaks. (see Lieberman et al. (1996) Safety and Immunogenicity of a Serogroups A/C Neisseria meningitidis Oligosaccharide-Protein Conjugate Vaccine in Young Children. JAMA 275(19):1499-1503; Schuchat et al (1997) Bacterial Meningitis in the United States in 1995. N Engl J Med 337(14):970-976). In developing countries, endemic disease rates are much higher and during epidemics incidence rates can reach 500 cases per 100,000 persons per year. Mortality is extremely high, at 10-20% in the United States, and much higher in developing countries. Following the introduction of the conjugate vaccine against *Haemophilus influenzae, N. meningitidis* is the major cause of bacterial meningitis at all ages in the United States (Schuchat *et al* (1997) *supra).*

Based on the organism's capsular polysaccharide, 12 serogroups of *N. meningitidis* have been identified. Group A is the pathogen most often implicated in epidemic disease in sub-Saharan Africa. Serogroups B and C are responsible for the vast majority of cases in the United States and in most developed countries. Serogroups W135 and Y are responsible for the rest of the cases in the United States and developed countries. The meningococcal vaccine currently in use is a tetravalent polysaccharide vaccine composed of serogroups A, C, Y and W135. Although efficacious in adolescents and adults, it induces a poor immune response and short duration of protection, and cannot be used in infants (e.g., Morbidity and Mortality weekly report, Vol. 46, No. RR-5 (1997)). This is because polysaccharides are T-cell independent antigens that induce a weak immune response that cannot be boosted by repeated immunization. Following the success of the vaccination against *H. influenzae,* conjugate vaccines against serogroups A and C have been developed and are at the final stage of clinical testing (Zollinger WD "New and Improved Vaccines Against Meningococcal Disease". In: New Generation Vaccines, supra, pp. 469-488; Lieberman *et al* (1996) *supra;* Costantino *et al* (1992) Development and phase I clinical testing of a conjugate vaccine against meningococcus A (menA) and C (menC) *(*Vaccine 10:691-698)).

Meningococcus B (MenB) remains a problem, however. This serotype currently is responsible for approximately 50% of total meningitis in the United States, Europe, and South America. The polysaccharide approach cannot be used because the MenB capsular polysaccharide is a polymer of α(2-8)-linked N-acetyl neuraminic acid that is also present in mammalian tissue. This results in tolerance to the antigen; indeed, if an immune response were elicited, it would be anti-self, and therefore undesirable. In order to avoid induction of autoimmunity and to induce a protective immune response, the capsular polysaccharide has, for instance, been chemically modified substituting the *N*-acetyl groups with N-propionyl groups, leaving the specific antigenicity unaltered (Romero & Outschoorn (1994) Current status of Meningococcal group B vaccine candidates: capsular or non-capsular? Clin Microbiol Rev 7(4):559-575).

Alternative approaches to MenB vaccines have used complex mixtures of outer membrane proteins (OMPs), containing either the OMPs alone, or OMPs enriched in porins, or deleted of the class 4 OMPs that are believed to induce antibodies that block bactericidal activity. This approach produces vaccines that are not well characterized. They are able to protect against the homologous strain, but are not effective at large where there are many antigenic variants of the outer membrane proteins. To overcome the antigenic variability, multivalent vaccines containing up to nine different porins have been constructed (e.g., Poolman JT (1992) Development of a meningococcal vaccine. Infect. Agents Dis. 4:13-28). Additional proteins to be used in outer membrane vaccines have been the opa and opc proteins, but none of these approaches have been able to overcome the antigenic variability (e.g., Ala'Aldeen & Borriello (1996) The meningococcal transferrin-binding proteins 1 and 2 are both surface exposed and generate bactericidal antibodies capable of killing homologous and heterologous strains. Vaccine 14(1):49-53).

A certain amount of sequence data is available for meningococcal and gonococcal genes and proteins (e.g., EP-A-0467714, WO96/29412), but this is by no means complete. An N. meningitidis protein is disclosed under the EMBL accession number X77920. The provision of further sequences could provide an opportunity to identify secreted or surface-exposed proteins that are presumed targets for the immune system and which are not antigenically variable or at least are more antigenically conserved than other and more variable regions. Thus, those antigenic sequences that are more highly conserved are preferred sequences. Those sequences specific to *Neisseria meningitidis* or *Neisseria*

*gonorrhoeae* that are more highly conserved are further preferred sequences. For instance, some of the identified proteins could be components of efficacious vaccines against meningococcus B, some could be components of vaccines against all meningococcal serotypes, and others could be components of vaccines against all pathogenic *Neisseriae.* The identification of sequences from the bacterium will also facilitate the production of biological probes, particularly organism-specific probes.

It is thus an object of the invention is to provide Neisserial DNA sequences which (1) encode proteins predicted and/or shown to be antigenic or immunogenic, (2) can be used as probes or amplification primers, and (3) can be analyzed by bioinformatics.

BRIEF DESCRIPTION OF THE DRAWINGS

Figure which do not relate specifically to the claimed invention are for illustration only. Fig. 1 illustrates the products of protein expression and purification of the predicted ORF 919 as cloned and expressed in *E. coli.*

Fig. 2 illustrates the products of protein expression and purification of the predicted ORF 279 as cloned and expressed in *E. coli.*

Fig. 3 illustrates the products of protein expression and purification of the predicted ORF 576-1 as cloned and expressed in *E. coli.*

Fig. 4 illustrates the products of protein expression and purification of the predicted ORF 519-1 as cloned and expressed in *E. coli.*

Fig. 5 illustrates the products of protein expression and purification of the predicted ORF 121-1 as cloned and expressed in *E. coli.*

Fig. 6 illustrates the products of protein expression and purification of the predicted ORF 128-1 as cloned and expressed in *E. coli.*

Fig. 7 illustrates the products of protein expression and purification of the predicted ORF 206 as cloned and expressed in *E. coli.*

Fig. 8 illustrates the products of protein expression and purification of the predicted ORF 287 as cloned and expressed in *E. coli.*

Fig. 9 illustrates the products of protein expression and purification of the predicted ORF 406 as cloned and expressed in *E. coli.*

Fig. 10 illustrates the hydrophilicity plot, antigenic index and AMPHI regions of the products of protein expression the predicted ORF 919 as cloned and expressed in *E. coli.*

Fig. 11 illustrates the hydrophilicity plot, antigenic index and AMPHI regions of the products of protein expression the predicted ORF 279 as cloned and expressed in *E. coli.*

Fig. 12 illustrates the hydrophilicity plot, antigenic index and AMPHI regions of the products of protein expression the predicted ORF 576-1 as cloned and expressed in *E. coli.*

Fig. 13 illustrates the hydrophilicity plot, antigenic index and AMPHI regions of the products of protein expression the predicted ORF 519-1 as cloned and expressed in *E. coli.*

Fig. 14 illustrates the hydrophilicity plot, antigenic index and AMPHI regions of the products of protein expression the predicted ORF 121-1 as cloned and expressed in *E. coli.*

Fig. 15 illustrates the hydrophilicity plot, antigenic index and AMPHI regions of the products of protein expression the predicted ORF 128-1 as cloned and expressed in *E. coli.*

Fig. 16 illustrates the hydrophilicity plot, antigenic index and AMPHI regions of the products of protein expression the predicted ORF 206 as cloned and expressed in *E. coli.*

Fig. 17 illustrates the hydrophilicity plot, antigenic index and AMPHI regions of the products of protein expression the predicted ORF 287 as cloned and expressed in *E. coli.*

Fig. 18 illustrates the hydrophilicity plot, antigenic index and AMPHI regions of the products of protein expression the predicted ORF 406 as cloned and expressed in *E. coli.*

THE INVENTION

The invention provides a protein consisting of the amino acid sequence encoded by nucleotides 1363680 to 1364114 of SEQ ID NO: 1.

The invention also provides a protein consisting of an amino acid sequence differing from the amino acid sequence encoded by nucleotides 1363680 to 1364114 of SEQ ID NO: 1, which sequence has 95 % or greater identity to one amino acid sequence encoded by nucleotides 1363680 to 1364114 of SEQ ID NO : 1.

The invention also provides an antibody, a nucleic acid and a composition as defined in the claims.

Aspects of the following disclosure that do not relate specifically to the claimed invention are included for comparison and illustration and comparison.

The first complete sequence of the genome of *N. meningitidis* was disclosed as 961 partial contiguous nucleotide sequences, shown as SEQ ID NOs:1-961 of co-owned PCT/US99/23573 (the ’573 application), filed 8 October 1999 (to be published April 2000). A single sequence full length genome of *N. meningitidis* was also disclosed as SEQ ID NO. 1068 of the ’573 application. The disclosure is based on a full length genome of *N. meningitidis* which appears as SEQ ID NO. 1 in the present application as Appendix A hereto. The 961 sequences of the ’573 application represent substantially the whole genome of serotype B of *N. meningitidis* (>99.98%). There is partial overlap between some of the 961 contiguous sequences ("contigs") shown in the 961 sequences, which overlap was used to construct the single full length sequence shown in SEQ ID NO. 1 in Appendix A hereto, using the TIGR Assembler [G.S. Sutton et al., TIGR Assembler: A New Tool for Assembling Large Shotgun Sequencing Projects, Genome Science and Technology, 1:9-19 (1995)]. Some of the nucleotides in the contigs had been previously released. (See ftp:11ftp.tigr.org/pub/data/n_meningitidis on the world-wide web or "WWW"). The coordinates of the 2508 released sequences in the present contigs are presented in Appendix A of the ’573 application. These data include the contig number (or i.d.) as presented in the first column; the name of the sequence as found on WWW is in the second column; with the coordinates of the contigs in the third and fourth columns, respectively. The sequences of certain MenB ORFs presented in Appendix B of the ’573 application feature in International Patent Application filed by Chiron SpA on October 9, 1998 (PCT/IB98/01665) and January 14, 1999 (PCT/IB99/00103) respectively. Appendix B hereto provides a listing of 2158 open reading frames contained within the full length sequence found in SEQ ID NO. 1 in Appendix A hereto. The information set forth in Appendix B hereto includes the "NMB" name of the sequence, the putative translation product, and the beginning and ending nucleotide positions within SEQ ID NO. 1 which comprise the open reading frames. These open reading frames are referred to herein as the "NMB open reading frames".

In a first aspect, the disclosure provides nucleic acid including the *N. meningitidis* nucleotide sequence shown in SEQ ID NO. 1 in Appendix A hereto. It also discloses nucleic acid comprising sequences having sequence identity to the nucleotide sequence disclosed herein. Depending on the particular sequence, the degree of sequence identity is preferably greater than 50% (e.g., 60%, 70%, 80%, 90%, 95%, 99% or more). These sequences include, for instance, mutants and allelic variants. The degree of sequence identity cited herein is determined across the length of the sequence determined by the Smith-Waterman homology search algorithm as implemented in MPSRCH program (Oxford Molecular) using an affine gap search with the following parameters: gap open penalty 12, gap extension penalty 1.

The disclosure also provides nucleic acid including a fragment of one or more of the nucleotide sequences set out herein, including the NMB open reading frames shown in Appendix B hereto. The fragment should comprise at least n consecutive nucleotides from the sequences and, depending on the particular sequence, n is 10 or more (e.g., 11, 12, 13, 14, 15, 16, 17, 18, 19, 20, 21, 22, 23, 24, 25, 30, 35, 40, 45, 50, 60, 75, 100 or more). Preferably, the fragment is unique to the genome of *N. meningitidis,* that is to say it is not present in the genome of another organism. More preferably, the fragment is unique to the genome of strain B of *N. meningitidis.* The disclosure also provides nucleic acid that hybridizes to those provided herein. Conditions for hybridizing are disclosed herein.

The disclosure also provides nucleic acid including sequences complementary to those described above (e.g., for antisense, for probes, or for amplification primers).

Nucleic acid according to the disclosure can, of course, be prepared in many ways (e.g., by chemical synthesis, from DNA libraries, from the organism itself, etc.) and can take various forms (e.g., single-stranded, double-stranded, vectors, probes, primers, etc.). The term "nucleic acid" includes DNA and RNA, and also their analogs, such as those containing modified backbones, and also peptide nucleic acid (PNA) etc.

It will be appreciated that, as SEQ ID NOs:1-961 of the ’573 application represent the substantially complete genome of the organism, with partial overlap, references to SEQ ID NOs:1-961 of the ’573 application include within their scope references to the complete genomic sequence, that is, SEQ ID NO. 1 hereof. For example, where two SEQ ID NOs overlap, the disclosure encompasses the single sequence which is formed by assembling the two overlapping sequences, which full sequence will be found in SEQ ID NO. 1 hereof. Thus, for instance, a nucleotide sequence which bridges two SEQ ID NOs but is not present in its entirety in either SEQ ID NO is still within the scope of the disclosure. Such a sequence will be present in its entirety in the single full length sequence of SEQ ID NO. 1 of the present application.

The disclosure also provides vectors including nucleotide sequences of the disclosure (e.g., expression vectors, sequencing vectors, cloning vectors, etc.) and host cells transformed with such vectors.

According to a further aspect, the disclosure provides a protein including an amino acid sequence encoded within a *N. meningitidis* nucleotide sequence set out herein. It also discloses proteins comprising sequences having sequence identity to those proteins. Depending on the particular sequence, the degree of sequence identity is preferably greater than 50% (e.g., 60%, 70%, 80%, 90%, 95%, 99% or more). Sequence identity is determined as above disclosed. These homologous proteins include mutants and allelic variants, encoded within the *N. meningitidis* nucleotide sequence set out herein.

The disclosure further provides proteins including fragments of an amino acid sequence encoded within a *N.*

*meningitidis* nucleotide sequence set out in the sequence listing. The fragments should comprise at least *n* consecutive amino acids from the sequences and, depending on the particular sequence, *n* is 7 or more (e.g., 8, 10, 12, 14, 16, 18,20 or more). Preferably, the fragments comprise an epitope from the sequence.

The proteins of the disclosure can, of course, be prepared by various means (e.g., recombinant expression, purification from cell culture, chemical synthesis, *etc.)* and in various forms (e.g. native, fusions *etc.).* They are preferably prepared in substantially isolated form (*i.e.*, substantially free from other *N. meningitidis* host cell proteins).

Various tests can be used to assess the *in vivo* immunogenicity of the proteins of the disclosure. For example, the proteins can be expressed recombinantly or chemically synthesized and used to screen patient sera by immunoblot. A positive reaction between the protein and patient serum indicates that the patient has previously mounted an immune response to the protein in question; i.e., the protein is an immunogen. This method can also be used to identify immunodominant proteins.

The disclosure also provides nucleic acid encoding a protein of the invention.

In a further aspect, the disclosure provides a computer, a computer memory, a computer storage medium (e.g., floppy disk, fixed disk, CD-ROM, etc.), and/or a computer database containing the nucleotide sequence of nucleic acid according to the disclosure. Preferably, it contains one or more of the *N. meningitidis* nucleotide sequences set out herein.

This may be used in the analysis of the *N. meningitidis* nucleotide sequences set out herein. For instance, it may be used in a search to identify open reading frames (ORFs) or coding sequences within the sequences.

In a further aspect, the disclosure provides a method for identifying an amino acid sequence, comprising the step of searching for putative open reading frames or protein-coding sequences within a *N. meningitidis* nucleotide sequence set out herein. Similarly, the disclosure provides the use of a *N. meningitidis* nucleotide sequence set out herein in a search for putative open reading frames or protein-coding sequences.

Open-reading frame or protein-coding sequence analysis is generally performed on a computer using standard bioinformatic techniques. Typical algorithms or program used in the analysis include ORFFINDER (NCBI), GENMARK [Borodovsky & McIninch (1993) Computers Chem 17:122-133], and GLIMMER [Salzberg et al. (1998) Nucl Acids Res 26:544-548].

A search for an open reading frame or protein-coding sequence may comprise the steps of searching a *N. meningitidis* nucleotide sequence set out herein for an initiation codon and searching the upstream sequence for an in-frame termination codon. The intervening codons represent a putative protein-coding sequence. Typically, all six possible reading frames of a sequence will be searched.

An amino acid sequence identified in this way can be expressed using any suitable system to give a protein. This protein can be used to raise antibodies which recognize epitopes within the identified amino acid sequence. These antibodies can be used to screen *N. meningitidis* to detect the presence of a protein comprising the identified amino acid sequence.

Furthermore, once an ORF or protein-coding sequence is identified, the sequence can be compared with sequence databases. Sequence analysis tools can be found at NCBI (http://www.ncbi.nlm.nih.gov) e.g., the algorithms BLAST, BLAST2, BLASTn, BLASTp, tBLASTn, BLASTx, & tBLASTx [see also Altschul et al. (1997) Gapped BLAST and PSI-BLAST: new generation of protein database search programs. Nucleic Acids Research 25:2289-3402]. Suitable databases for comparison include the nonredundant GenBank, EMBL, DDBJ and PDB sequences, and the nonredundant GenBank CDS translations, PDB, SwissProt, Spupdate and PIR sequences. This comparison may give an indication of the function of a protein.

Hydrophobic domains in an amino acid sequence can be predicted using algorithms such as those based on the statistical studies of Esposti et al. [Critical evaluation of the hydropathy of membrane proteins (1990) Eur JBiochem 190:207-219]. Hydrophobic domains represent potential transmembrane regions or hydrophobic leader sequences, which suggest that the proteins may be secreted or be surface-located. These properties are typically representative of good immunogens.

Similarly, transmembrane domains or leader sequences can be predicted using the PSORT algorithm (http://www.psort.nibb.ac.jp), and functional domains can be predicted using the MOTIFS program (GCG Wisconsin & PROSITE).

The disclosure also provides nucleic acid including an open reading frame or protein-coding sequence present in a *N. meningitidis* nucleotide sequence set out herein. Furthermore, the disclosure provides a protein including the amino acid sequence encoded by this open reading frame or protein-coding sequence.

According to a further aspect, the disclosure provides antibodies which bind to these proteins. These may be polyclonal or monoclonal and may be produced by any suitable means known to those skilled in the art.

The antibodies of the disclosure can be used in a variety of ways, e.g., for confirmation that a protein is expressed, or to confirm where a protein is expressed. Labeled antibody (e.g., fluorescent labeling for FACS) can be incubated with intact bacteria and the presence of label on the bacterial surface confirms the location of the protein, for instance.

According to a further aspect, the disclosure provides compositions including protein, antibody, and/or nucleic acid according to the invention. These compositions may be suitable as vaccines, as immunogenic compositions, or as diagnostic reagents.

The disclosure also provides nucleic acid, protein, or antibody according to the disclosure for use as medicaments (e.g., as vaccines) or as diagnostic reagents. It also provides the use of nucleic acid, protein, or antibody according to the disclosure in the manufacture of (I) a medicament for treating or preventing infection due to Neisserial bacteria (ii) a diagnostic reagent for detecting the presence of Neisserial bacteria or of antibodies raised against Neisserial bacteria. Said Neisserial bacteria may be any species or strain (such as *N. gonorrhoeae)* but are preferably *N. meningitidis,* especially strain A, strain B or strain C.

In still yet another aspect, the present disclosure provides for compositions including proteins, nucleic acid molecules, or antibodies. More preferable aspects of the present disclosure are drawn to immunogenic compositions of proteins. Further preferable aspects of the present disclosure contemplate pharmaceutical immunogenic compositions of proteins or vaccines and the use thereof in the manufacture of a medicament for the treatment or prevention of infection due to Neisserial bacteria, preferably infection of MenB.

The disclosure provides a method of treating a patient, comprising administering to the patient a therapeutically effective amount of nucleic acid, protein, and/or antibody according to the invention.

According to further aspects, the disclosure provides various processes.

A process for producing proteins of the disclosure is provided, comprising the step of culturing a host cell according to the disclosure under conditions which induce protein expression. A process which may further include chemical synthesis of proteins and/or chemical synthesis (at least in part) of nucleotides.

A process for detecting polynucleotides of the disclosure is provided, comprising the steps of: (a) contacting a nucleic probe according to the disclosure with a biological sample under hybridizing conditions to form duplexes; and (b) detecting said duplexes.

A process for detecting proteins of the disclosure is provided, comprising the steps of: (a) contacting an antibody according to the disclosure with a biological sample under conditions suitable for the formation of an antibody-antigen complexes; and (b) detecting said complexes.

Another aspect of the present disclosure provides for a process for detecting antibodies that selectably bind to antigens or polypeptides or proteins specific to any species or strain of Neisserial bacteria and preferably to strains of *N. gonorrhoeae* but more preferably to strains of *N. meningitidis,* especially strain A, strain B or strain C, more preferably MenB, where the process comprises the steps of: (a) contacting antigen or polypeptide or protein according to the disclosure with a biological sample under conditions suitable for the formation of an antibody-antigen complexes; and (b) detecting said complexes.

Having now generally described the disclosure, the same will be more readily understood through reference to the following examples which are provided by way of illustration.

<u>Methodology - Summary of standard procedures and techniques.</u>

General

This disclosure provides *Neisseria meningitidis* MenB nucleotide sequences, amino acid sequences encoded therein. With these disclosed sequences, nucleic acid probe assays and expression cassettes and vectors can be produced. The proteins can also be chemically synthesized. The expression vectors can be transformed into host cells to produce proteins. The purified or isolated polypeptides can be used to produce antibodies to detect MenB proteins. Also, the host cells or extracts can be utilized for biological assays to isolate agonists or antagonists. In addition, with these sequences one can search to identify open reading frames and identify amino acid sequences. The proteins may also be used in immunogenic compositions and as vaccine components.

The practice of the present disclosure will employ, unless otherwise indicated, conventional techniques of molecular biology, microbiology, recombinant DNA, and immunology, which are within the skill of the art. Such techniques are explained fully in the literature e.g., Sambrook Molecular Cloning; A Laboratory Manual, Second Edition (1989); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C.C. Blackwell eds 1986).

Standard abbreviations for nucleotides and amino acids are used in this specification.

Expression systems

The *Neisseria* MenB nucleotide sequences can.be expressed in a variety of different expression systems; for example those used with mammalian cells, plant cells, baculoviruses, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (e.g., structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation (Sambrook et al. (1989) "Expression of Cloned Genes in Mammalian Cells." In Molecular Cloning: A Laboratory Manual, 2nd ed*.).*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallothionein gene, also provide useful promoter sequences. Expression may be either constitutive or regulated (inducible). Depending on the promoter selected, many promotes may be inducible using known substrates, such as the use of the mouse mammary tumor virus (MMTV) promoter with the glucocorticoid responsive element (GRE) that is induced by glucocorticoid in hormone-responsive transformed cells (see for example, U.S. Patent 5,783,681).

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter (Maniatis et al. (1987) Science 236:1237*;* Alberts et al. (1989) Molecular Biology of the Cell, 2nd ed.). Enhancer elements derived from viruses may be particularly useful, because they usually have a broader host range. Examples include the SV40 early gene enhancer (Dijkema et al (1985) EMBO J. 4:761) and the enhancer/promoters derived from the long terminal repeat (LTR) of the Rous Sarcoma Virus (Gorman et al. (1982b) Proc. Natl. Acad. Sci. 79:6777) and from human cytomegalovirus (Boshart et al. (1985) Cell 41:521). Additionally, some enhancers are regulatable and become active only in the presence of an inducer, such as a hormone or metal ion (Sassone-Corsi and Borelli (1986) Trends Genet. 2:215; Maniatis et al. (1987) Science 236:1237).

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus tripartite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation (Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105). These sequences direct the transcription of an mRNA which can be translated into the polypeptide encoded by the DNA. Examples of transcription terminator/polyadenylation signals include those derived from SV40 (Sambrook et al (1989) "Expression of cloned genes in cultured mammalian cells." In Molecular Cloning: A Laboratory Manual)*.*

Usually, the above-described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g., plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 (Gluzman (1981) Cell 23:175) or polyomavirus, replicate to extremely high copy number in the presence of the appropriate viral T antigen. Additional examples of mammalian replicons include those derived from bovine papillomavirus and Epstein-Barr virus. Additionally, the replicon may have two replication systems, thus allowing it to be maintained, for example, in mammalian cells for expression and in a prokaryotic host for cloning and amplification. Examples of such mammalian-bacteria shuttle vectors include pMT2 (Kaufman et al. (1989) Mol. Cell. Biol. 9:946) and pHEBO (Shimizu et al. (1986) Mol. Cell. Biol. 6:1074).

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

ii. Plant Cellular Expression Systems

There are many plant cell culture and whole plant genetic expression systems known in the art. Exemplary plant cellular genetic expression systems include those described in patents, such as: U.S. 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolin B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, *Texas Agricultural Experiment Station Bulletin No. 1555* (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g., plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance *(amp)* gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g., structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human (alpha) $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by cotransfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91. The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 μm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. Current Protocols in Microbiology Vol. 2 (Ausubel et al. eds) at 16.8 (Supp. 10, 1990); Summers and Smith, *supra;* Miller et al. (1989).

Recombinant baculovirus expression vectors have been developed for infection into several insect cells. For example, recombinant baculoviruses have been developed for, *inter alia: Aedes aegypti , Autographa californica, Bombyx mori, Drosophila melanogaster, Spodoptera frugiperda,* and *Trichoplusia ni* (PCT Pub. No. WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See,* e.g., Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g., HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g., proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iv. Bacterial Systems

Bacterial expression techniques are known in the art. A bacterial promoter is any DNA sequence capable of binding bacterial RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli *(E. coli)* (Raibaud et al. (1984) Annu. Rev. Genet. 18:173). Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose *(lac)* (Chang et al. (1977) Nature 198:1056), and maltose. Additional examples include promoter sequences derived from biosyhthetic enzymes such as tryptophan *(trp)* (Goeddel et al. (1980) Nuc. Acids Res. 8:4057; Yelverton et al. (1981) Nucl. Acids Res. 9:731; U.S. Patent 4,738,921; EPO Publ. Nos. 036 776 and 121 775). The beta-lactamase *(bla)* promoter system (Weissmann (1981) "The cloning of interferon and other mistakes." In *Interferon 3* (ed. I. Gresser)), bacteriophage lambda PL (Shimatake et al. (1981) Nature 292:128) and T5 (U.S. Patent 4,689,406) promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter (U.S. Patent 4,551,433). For example, the *tac* promoter is a hybrid *trp-lac* promoter comprised of both *trp* promoter and *lac* operon sequences that is regulated by the *lac* repressor (Amann et al. (1983) Gene 25:167; de Boer et al. (1983) Proc. Natl. Acad. Sci. 80:21). Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system (Studier et al. (1986) J. Mol. Biol. 189:113; Tabor et al. (1985) Proc Natl. Acad. Sci. 82:1074). In addition, a hybrid promoter can also be comprised of a bacteriophage promoter and an *E. coli* operator region (EPO Publ. No. 267 851).

In addition to a functioning promoter sequence, an efficient ribosome binding site is also useful for the expression of foreign genes in prokaryotes. In *E. coli,* the ribosome binding site is called the Shine-Dalgamo (SD) sequence and includes an initiation codon (ATG) and a sequence 3-9 nucleotides in length located 3-11 nucleotides upstream of the initiation codon (Shine et al. (1975) Nature 254:34). The SD sequence is thought to promote binding of mRNA to the ribosome by the pairing of bases between the SD sequence and the 3' end of *E. coli* 16S rRNA (Steitz et al. (1979) "Genetic signals and nucleotide sequences in messenger RNA." In Biological Regulation and Development: Gene Expression (ed. R.F. Goldberger)). To express eukaryotic genes and prokaryotic genes with weak ribosome-binding site, it is often necessary to optimize the distance between the SD sequence and the ATG of the eukaryotic gene (Sambrook et al. (1989) "Expression of cloned genes in Escherichia coli." In Molecular Cloning: A Laboratory Manual).

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* or *in vitro* incubation with a bacterial methionine N-terminal peptidase (EPO Publ. No. 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene (Nagai et al. (1984) Nature 309:810). Fusion proteins can also be made with sequences from the *lacZ* (Jia et al. (1987) Gene 60:197), *trpE* (Allen et al. (1987) J. Biotechnol. 5:93; Makoff et al. (1989) J. Gen. Microbiol. 135:11), and *Chey* (EPO Publ. No. 324 647) genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (e.g. ubiquitin specific processing-protease) to cleave the ubiquitin from the foreign protein. Through this method, native foreign protein can be isolated (Miller et al. (1989) BiolTechnology 7:698).

Alternatively, foreign proteins can also be secreted from the cell by creating chimeric DNA molecules that encode a fusion protein comprised of a signal peptide sequence fragment that provides for secretion of the foreign protein in bacteria (U.S. Patent 4,336,336). The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene *(ompA)* (Masui et al. (1983), in: Experimental Manipulation of Gene Expression; Ghrayeb et al. (1984) EMBO J. 3:2437) and the *E. coli* alkaline phosphatase signal sequence *(phoA)* (Oka et al. (1985) Proc. Natl. Acad. Sci. 82:7212). As an additional example, the signal sequence of the alpha-amylase gene from various Bacillus strains can be used to secrete heterologous proteins from *B. subtilis* (Palva et al. (1982) Proc. Natl. Acad. Sci. USA 79:5582; EPO Publ. No. 244 042).

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (e.g., plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EPO Publ. No. 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline (Davies et al. (1978) Annu. Rev. Microbiol. 32:469). Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis (Palva et al. (1982) Proc. Natl. Acad. Sci. USA 79:5582; EPO Publ. Nos. 036 259 and 063 953; PCT Publ. No. WO 84/04541), Escherichia coli (Shimatake et al. (1981) Nature 292:128; Amann et al. (1985) Gene 40:183; Studier et al. (1986) J. Mol. Biol. 189:113; EPO Publ. Nos. 036 776, 136 829 and 136 907), Streptococcus cremoris (Powell et al. (1988) Appl. Environ. Microbiol. 54:655); Streptococcus lividans (Powell et al. (1988) Appl. Environ. Microbiol. 54:655), Streptomyces lividans (U.S. Patent 4,745,056).

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. (See e.g., use of *Bacillus:* Masson et al. (1989) FEMS Microbiol. Lett. 60:273*;* Palva et al. (1982) Proc. Natl. Acad. Sci. USA 79:5582; EPO Publ. Nos. 036 259 and 063 953; PCT Publ. No. WO 84/04541; use *of Campylobacter:* Miller et al. (1988) Proc. Natl. Acad. Sci. 85:856; and Wang et al. (1990) J. Bacteriol. 172:949*;* use of *Escherichia coli:* Cohen et al. (1973) Proc. Natl. Acad. Sci. 69:2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; use of *Lactobacillus:* Chassy et al. (1987) FEMS Microbiol. Lett. 44:173; use of *Pseudomonas:* Fiedler et al. (1988) Anal. Biochem 170:38*;* use of *Staphylococcus:* Augustin et al. (1990) FEMS Microbiol. Lett. 66:203; use of *Streptococcus:* Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412.

v. Yeast Expression

Yeast expression systems are also known to one of ordinary skill in the art. A yeast promoter is any DNA sequence capable of binding yeast RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EPO Publ. No. 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO Publ. No. 329 203). The yeast *PHO5* gene, encoding acid phosphatase, also provides useful promoter sequences (Myanohara et al. (1983) Proc. Natl. Acad. Sci. USA 80:1).

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (U.S. Patent Nos. 4,876,197 and 4,880,734). Other examples of hybrid promoters include promoters which consist of the regulatory sequences of either *the ADH2, GAL4, GAL10, OR PHO5* genes, combined with the transcriptional activation region of a glycolytic enzyme gene such as GAP or PyK (EPO Publ. No. 164 556). Furthermore, a yeast promoter can include naturally occurring promoters of non-yeast origin that have the ability to bind yeast RNA polymerase and initiate transcription. Examples of such promoters include, *inter alia,* (Cohen et al. (1980) Proc. Natl. Acad Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;).

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, plant, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g., EPO Publ. No. 196056. Another example is a ubiquitin fusion protein. Such a fusion protein is made with the ubiquitin region that preferably retains a site for a processing enzyme (e.g. ubiquitin-specific processing protease) to cleave the ubiquitin from the foreign protein. Through this method, therefore, native foreign protein can be isolated (e.g., WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EPO Publ. No. 012 873; JPO Publ. No. 62:096,086) and the A-factor gene (U.S. Patent 4,588,684). Alternatively, leaders of non-yeast origin, such as an interferon leader, exist that also provide for secretion in yeast (EPO Publ. No. 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (U.S. Patent Nos. 4,546,083 and 4,870,008; EPO Publ. No. 324 274). Additional leaders employing an alpha-factor leader fragment that provides for secretion include hybrid alpha-factor leaders made with a presequence of a first yeast, but a pro-region from a second yeast alpha factor. (See e.g., PCT Publ. No. WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 (Botstein et al. (1979) Gene 8:17-24), pCl/l (Brake et al. (1984) Proc. Natl. Acad. Sci USA 81:4642-4646), and YRp17 (Stinchcomb et al. (1982) J. Mol. Biol. 158:157). In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See e.g., Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome (Orr-Weaver et al. (1983) Methods in Enzymol. 101:228-245). An integrating vector may be directed to a specific locus in yeast by selecting the appropriate homologous sequence for inclusion in the vector. See Orr-Weaver *et al., supra.* One or more expression construct may integrate, possibly affecting levels of recombinant protein produced (Rine et al. (1983) Proc. Natl. Acad. Sci. USA 80:6750). The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRPI,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions (Butt et al. (1987) Microbiol, Rev. 51:351).

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors and methods of introducing exogenous DNA into yeast hosts have been developed for, *inter alia,* the following yeasts: *Candida albicans* (Kurtz, et al. (1986) Mol. Cell. Biol. 6:142); *Candida maltosa* (Kunze, et al. (1985) J. Basic Microbiol. 25:141); *Hansenula polymorpha* (Gleeson, et al. (1986) J. Gen. Microbiol. 132:3459; Roggenkamp et al. (1986) Mol. Gen. Genet. 202:302*); Kluyveromyces fragilis* (Das, et al. (1984) J. Bacteriol. 158:1165); *Kluyveromyces lactis* (De Louvencourt et al. (1983) J. Bacteriol. 154: 737; Van den Berg et al. (1990) Bio/Technology 8:135); *Pichia guillerimondii* (Kunze et al. (1985) J. Basic Microbiol. 25: 141)*; Pichia pastoris* (Cregg, et al. (1985) Mol. Cell. Biol. 5:3376; U.S. Patent Nos. 4,837,148 and 4,929,555); *Saccharomyces cerevisiae* (Hinnen et al. (1978) Proc. Natl. Acad. Sci. USA 75:1929; Ito et al. (1983) J. Bacteriol. 153:163); *Schizosaccharomyces pombe* (Beach and Nurse (1981) Nature 300:706); and *Yarrowia lipolytica* (Davidow, et al. (1985) Curr. Genet. 10:380471 Gaillardin, et al. (1985) Curr. Genet. 10:49).

Methods of introducing exogenous DNA into yeast hosts are well-known in the art, and usually include either the transformation of spheroplasts or of intact yeast cells treated with alkali cations. Transformation procedures usually vary with the yeast species to be transformed. See e.g., [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5: 3376; Kunze et.al. (1985) J. Basic Microbiol. 25:141; U.S. Patent Nos. 4,837,148 and 4,929,555; Pichia]; [Hinnen et al. (1978) Proc. Natl. Acad. Sci. USA 75;1929; Ito et al. (1983) J. Bacteriol. 153:163 Saccharomyces]; [Beach and Nurse (1981) Nature 300:706; Schizosaccharomyces]; [Davidow et al. (1985) Curr. Genet. 10:39; Gaillardin et al. (1985) Curr. Genet. 10:49; Yarrowia].

Definitions

A composition containing X is "substantially free of' Y when at least 85% by weight of the total X+Y in the composition is X. Preferably, X comprises at least about 90% by weight of the total of X+Y in the composition, more preferably at least about 95% or even 99% by weight.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as a DNA, RNA or amino acid sequence differing from but having homology with the native or disclosed sequence. Depending on the particular sequence, the degree of homology between the native or disclosed sequence and the mutant sequence is preferably greater than 50% (e.g., 60%, 70%, 80%, 90%, 95%, 99% or more) which is calculated as described above. As used herein, an "allelic variant" of a nucleic acid molecule, or region, for which nucleic acid sequence is provided herein is a nucleic acid molecule, or region, that occurs at essentially the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions. (see, for example, U.S. Patent 5,753,235).

Antibodies

As used herein, the term "antibody" refers to a polypeptide or group of polypeptides composed of at least one antibody combining site. An "antibody combining site" is the three-dimensional binding space with an internal surface shape and charge distribution complementary to the features of an epitope of an antigen, which allows a binding of the antibody with the antigen. "Antibody" includes, for example, vertebrate antibodies, hybrid antibodies, chimeric antibodies, humanized antibodies, altered antibodies, univalent antibodies, Fab proteins, and single domain antibodies.

Antibodies against the proteins of the invention are useful for affinity chromatography, immunoassays, and distinguishing/identifying *Neisseria* MenB proteins. Antibodies elicited against the proteins of the present invention bind to antigenic polypeptides or proteins or protein fragments that are present and specifically associated with strains of *Neisseria meningitidis* MenB. In some instances, these antigens may be associated with specific strains, such as those antigens specific for the MenB strains. The antibodies of the invention may be immobilized to a matrix and utilized in an immunoassay or on an affinity chromatography column, to enable the detection and/or separation of polypeptides, proteins or protein fragments or cells comprising such polypeptides, proteins or protein fragments. Alternatively, such polypeptides, proteins or protein fragments may be immobilized so as to detect antibodies bindably specific thereto.

Antibodies to the proteins of the invention, both polyclonal and monoclonal, may be prepared by conventional methods. In general, the protein is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to *in vivo* immunization. Polyclonal antisera is obtained by bleeding the immunized animal into a glass or plastic container, incubating the blood at 25°C for one hour, followed by incubating at 4°C for 2-18 hours. The serum is recovered by centrifugation (e.g., 1,000g for 10 minutes). About 20-50 ml per bleed may be obtained from rabbits.

Monoclonal antibodies are prepared using the standard method of Kohler & Milstein (Nature (1975) 256:495-96), or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the protein antigen. B-cells that express membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected MAb-secreting hybridomas are then cultured either *in vitro* (e.g., in tissue culture bottles or hollow fiber reactors), or *in vivo* (as ascites in mice).

If desired, the antibodies (whether polyclonal or monoclonal) may be labeled using conventional techniques. Suitable labels include fluorophores, chromophores, radioactive atoms (particularly $^{32}P$ and $^{125}I$), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, $^{125}I$ may serve as a radioactive label or as an electron-dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with $^{125}I$, or with an anti-biotin MAb labeled with HRP. Other permutations and possibilities will be readily apparent to those of ordinary skill in the art, and are considered as equivalents within the scope of the instant invention.

Antigens, immunogens, polypeptides, proteins or protein fragments of the present invention elicit formation of specific binding partner antibodies. These antigens, immunogens, polypeptides, proteins or protein fragments of the present invention comprise immunogenic compositions of the present invention. Such immunogenic compositions may further comprise or include adjuvants, carriers, or other compositions that promote or enhance or stabilize the antigens, polypeptides, proteins or protein fragments of the present invention. Such adjuvants and carriers will be readily apparent to those of ordinary skill in the art.

Pharmaceutical Compositions

Pharmaceutical compositions can include either polypeptides, antibodies, or nucleic acid of the disclosure. The pharmaceutical compositions will comprise a therapeutically effective amount of either polypeptides, antibodies, or polynucleotides of the disclosure invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature, when given to a patient that is febrile. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present disclosure, an effective dose will be from about 0.01 mg/kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

Delivery Methods

Once formulated, the compositions of the disclosure can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal and transcutaneous applications, needles, and gene guns or hyposprays. Dosage treatment may be a single dose schedule or a multiple dose schedule.

Vaccines

Vaccines according to the disclosure may either be prophylactic (i.e., to prevent infection) or therapeutic (i.e., to treat disease after infection).

Such vaccines comprise immunizing antigen(s) or immunogen(s), immunogenic polypeptide, protein(s) or protein fragments, or nucleic acids (e.g., ribonucleic acid or deoxyribonucleic acid), usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the immunogen or antigen may be conjugated to a bacterial toxoid, such as a toxoid from diphtheria, tetanus, cholera, *H. pylori,* etc. pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (e.g., IL-1, IL-2, IL-4, IL-5, IL-6, IL-7, IL-12, *etc.),* interferons (e.g., gamma interferon), macrophage colony stimulating factor (M-CSF), tumor necrosis factor (TNF), etc; (6) detoxified mutants of a bacterial ADP-ribosylating toxin such as a cholera toxin (CT), a pertussis toxin (PT), or an E. coli heat-labile toxin (LT), particularly LT-K63, LT-R72, CT-S109, PT-K9/G129; see, e.g., WO 93/13302 and WO 92/19265; and (7) other substances that act as immunostimulating agents to enhance the effectiveness of the composition. Alum and MF59 are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-huydroxyphosphoryloxy)-ethylamine (MTP-PE), *etc.*

The vaccine compositions comprising immunogenic compositions (e.g., which may include the antigen, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles. Alternatively, vaccine compositions comprising immunogenic compositions may comprise an antigen, polypeptide, protein, protein fragment or nucleic acid in a pharmaceutically acceptable carrier.

More specifically, vaccines comprising immunogenic compositions comprise an immunologically effective amount of the immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (e.g., nonhuman primate, primate, *etc.),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

Typically, the vaccine compositions or immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

The immunogenic compositions are conventionally administered parenterally, e.g., by injection, either subcutaneously or intramuscularly. Additional formulations suitable for other modes of administration include oral and pulmonary formulations, suppositories, and transdermal and transcutaneous applications. Dosage treatment may be a single dose schedule or a multiple dose schedule. The vaccine may be administered in conjunction with other immunoregulatory agents.

As an alternative to protein-based vaccines, DNA vaccination may be employed (e.g., Robinson & Torres (1997) Seminars in Immunology 9:271-283; Donnelly et al. (1997) Annu Rev Immunol 15:617-648).

Gene Delivery Vehicles

Gene therapy, vehicles for delivery of constructs, including a coding sequence of a therapeutic of the disclosure, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The disclosure includes gene delivery vehicles capable of expressing the contemplated nucleic acid sequences.

The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

Retroviral vectors are well known in the art, including B, C and D type retroviruses, xenotropic retroviruses (for example, NZB-X1, NZB-X2 and NZB9-1 (see O'Neill (1985) J. Virol. 53:160) polytropic retroviruses e.g., MCF and MCF-MLV (see Kelly (1983) J. Virol. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells (e.g., HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25*),* Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this disclosure include those described in patent applications GB2200651, EP0415731, EP0345242, EP0334301, WO89/02468; WO89/05349, WO89/09271, WO90/02806, WO90/07936, WO94/03622, WO93/25698, WO93/25234, WO93/11230, WO93/10218, WO91/02805, WO91/02825, WO95/07994, US 5,219,740, US 4,405,712, US 4,861,719, US 4,980,289, US 4,777,127, US 5,591,624. See also Vile (1993) Cancer Res 53:3860-3864; Vile (1993) Cancer Res 53:962-967; Ram (1993) Cancer Res 53 (1993) 83-88; Takamiya (1992) J Neurosci Res 33:493-503; Baba (1993) J Neurosurg 79:729-735; Mann (1983) Cell 33:153; Cane (1984) Proc Natl Acad Sci 81:6349; and Miller (1990) Human Gene Therapy 1.

Human adenoviral gene therapy vectors are also known in the art and employable in this disclosure. See, for example, Berkner (1988) Biotechniques 6:616 and Rosenfeld (1991) Science 252:431, and WO93/07283, WO93/06223, and WO93/07282. Exemplary known adenoviral gene therapy vectors employable in this disclosure include those described in the above referenced documents and in WO94/12649, WO93/03769, WO93/19191, WO94/28938, WO95/11984 WO95/00655 WO95/27071, WO95/29993, WO95/34671, WO96/05320, WO94/08026, WO94/11506, WO93/06223, WO94/24299, WO95/14102, WO95/24297, WO95/02697, WO94/2815, WO94/24299, WO95/09241, WO95/25807, WO95/05835, WO94/18922 and WO95/09654. Alternatively, administration of DNA linked to killed adenovirus as described in Curiel (1992) Hum. Gene Ther. 3:147-154 may be employed. The gene delivery vehicles of the disclosure also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (i.e., there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) Gene 124:257-262. Another example of such an AAV vector is psub201 (see Samulski (1987) J. virol. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver. Its structure and construction are disclosed in Su (1996) Human Gene Therapy 7:463-470. Additional AAV gene therapy vectors are described in US 5,354,678, US 5,173,414, US 5,139,941, and US 5,252,479.

The gene therapy vectors comprising sequences of the disclosure also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar Institute), pHSVlac described in Geller (1988) Science 241: 1667-1669 and in WO90/09441 and WO92/07945, HSV Us3::pgC-lacZ described in Fink (1992) Human Gene Therapy 3:11-19 and HSV 7134, 2 RH 105 and GAL4 described in EP 0453242 (Breakefield), and those deposited with the ATCC as accession numbers ATCC VR-977 and ATCC VR-260.

Also contemplated are alpha virus gene therapy vectors that can be employed in this disclosure. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in U.S. Serial No. 08/405,627, filed March 15, 1995, WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukarytic layered expression systems are also useful for expressing the nucleic acids of the disclosure. SeeWO95/07994 for a detailed description of eukaryotic layered expression systems. Preferably, the eukaryotic layered expression systems of the disclosure are derived from alphavirus vectors and most preferably from Sindbis viral vectors.

Other viral vectors suitable for use in the present disclosure include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US.4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Virol. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this disclosure into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14: 2411-2418 and in Woffendin (1994) Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin.

Naked DNA may also be employed to transform a host cell. Exemplary naked DNA introduction methods are described in WO 90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in U.S. 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc. Natl. Acad Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in U.S. 5,149,655; use of ionizing radiation for activating transferred gene, as described in U.S. 5,206,152 and WO92/11033

Exemplary liposome and polycationic gene delivery vehicles are those described in US 5,422,120 and 4,762,915; inWO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprise a therapeutically effective amount of a gene therapy vehicle, as the term is defined above. For purposes of the present disclosure, an effective dose will be from about 0.01 mg/ kg to 50 mg/kg or 0.05 mg/kg to about 10 mg/kg of the DNA constructs in the individual to which it is administered.

Delivery Methods

Once formulated, the polynucleotide compositions of the disclosure can be administered (1) directly to the subject; (2) delivered ex vivo, to cells derived from the subject; or (3) in vitro for expression of recombinant proteins. The subjects to be treated can be mammals or birds. Also, human subjects can be treated.

Direct delivery of the compositions will generally be accomplished by injection, either subcutaneously, intraperitoneally, transdermally or transcutaneously, intravenously or intramuscularly or delivered to the interstitial space of a tissue. The compositions can also be administered into a tumor or lesion. Other modes of administration include oral and pulmonary administration, suppositories, and transdermal applications, needles, and gene guns or hyposprays. Dosage treatment may be a single dose schedule or a multiple dose schedule. See WO98/20734.

Methods for the *ex vivo* delivery and reimplantation of transformed cells into a subject are known in the art and described in e.g., WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo and in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Polynucleotide and Polypeptide pharmaceutical compositions

In addition to the pharmaceutically acceptable carriers and salts described above, the following additional agents can be used with polynucleotide and/or polypeptide compositions.

A. Polypeptides

One example are polypeptides which include, without limitation: asialoorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B. Hormones, Vitamins, Etc.

Other groups that can be included in a pharmaceutical composition include, for example: hormones, steroids, androgens, estrogens, thyroid hormone, or vitamins, folic acid.

C. Polyalkylenes, Polysaccharides, *etc.*

Also, polyalkylene glycol can be included in a pharmaceutical compositions with the desired polynucleotides and/or polypeptides. In a preferred embodiment, the polyalkylene glycol is polyethlylene glycol. In addition, mono-, di-, or polysaccarides can be included. In a preferred embodiment of this aspect, the polysaccharide is dextran or DEAE-dextran. Also, chitosan and poly(lactide-co-glycolide) may be included in a pharmaceutical composition.

D. Lipids, and Liposomes

The desired polynucleotide or polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid or polypeptide. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present disclosure include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86: 6077-6081); and purified transcription factors (Debs (1990) J. Biol. Chem. 265:10189-10192), in functional form.

Cationic liposomes are readily available. For example, N(1-2,3-dioleyloxy)propyl)-N,N,N-triethylammonium (DOTMA) liposomes are available under the trademark Lipofectin, from GIBCO BRL, Grand Island, NY. (See, also, Felgner *supra).* Other commercially available liposomes include transfectace (DDAB/DOPE) and DOTAP/DOPE (Boerhinger). Other cationic liposomes can be prepared from readily available materials using techniques well known in the art. See, e.g., Szoka (1978) Proc. Natl. Acad. Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See e.g., Straubinger (1983) Meth. Immunol. 101:512-527; Szoka (1978) Proc. Natl. Acad Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E. Lipoproteins

In addition, lipoproteins can be included with the polynucleotide or polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, and E; over time these lipoproteins lose A and acquire C and E apoproteins. VLDL comprises A, B, C, and E apoproteins, LDL comprises apoprotein B; and HDL comprises apoproteins A, C, and E.

The amino acid sequences of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phopholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in *Meth. Enzymol. (supra);* Pitas (1980) J. Biochem. 255:5454-5460 and Mahey (1979) J Clin. Invest 64: 743-750.

Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443.

Lipoproteins can also be purchased from commercial suppliers, such as Biomedical Technologies, Inc., Stoughton, Massachusetts, USA.

Further description of lipoproteins can be found in Zuckermann et al., PCT. Appln. No. US97/14465.

F. Polycationic Agents

Polycationic agents can be included, with or without lipoprotein, in a composition with the desired polynucleotide and/or polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.

The following are examples of useful polypeptides as polycationic agents: polylysine, polyarginine, polyornithine, and protamine. Other examples of useful polypeptides include histones, protamines; human serum albumin, DNA binding proteins, non-histone chromosomal proteins, coat proteins from DNA viruses, such as ΦX174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

G. Synthetic Polycationic Agents

Synthetic polycationic agents which are useful in pharmaceutical compositions include, for example, DEAE-dextran, polybrene. Lipofectin™, and lipofectAMINE™ are monomers that form polycationic complexes when combined with polynucleotides or polypeptides. Immunodiagnostic Assays

*Neisseria* MenB antigens, or antigenic fragments thereof, of the disclosure can be used in immunoassays to detect antibody levels (or, conversely, *anti-Neisseria* MenB antibodies can be used to detect antigen levels). Immunoassays based on well defined, recombinant antigens can be developed to replace invasive diagnostics methods. Antibodies to *Neisseria* MenB proteins or fragments thereof within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, *etc.)* required for the conduct of the assay, as well as suitable set of assay instructions.

Nucleic Acid Hybridization

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al. (supra)* Volume 2, chapter 9, pages 9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1μg for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 μg of yeast DNA, blotting for two hours, and hybridizing for 4-8 hours with a probe of $10^8$ cpm/μg. For a single-copy mammalian gene a conservative approach would start with 10 μg of DNA, blot overnight, and hybridize overnight in the presence of 10% dextran sulfate using a probe of greater than $10^8$ cpm/μg, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation: $Tm = 81 + 16.6(\log_{10}Ci) + 0.4(\%(G + C)) - 0.6(\%formamide) - 600/n - 1.5(\%mismatch)$ where Ci is the salt concentration (monovalent ions) and n is the length of the hybrid in base pairs (slightly modified from Meinkoth & Wahl (1984) Anal. Biochem. 138:267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (i.e., stringency), it becomes less likely for hybridization to occur between strands that are nonhomologous, and as a result, background decreases. If the radiolabeled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

Nucleic Acid Probe Assays

Methods such as PCR, branched DNA probe assays, or blotting techniques utilizing nucleic acid probes according to the disclosure can determine the presence of cDNA or mRNA. A probe is said to "hybridize" with a sequence of the disclosure if it can form a duplex or double stranded complex, which is stable enough to be detected. The nucleic acid probes will hybridize to the Neisserial nucleotide sequences of the disclosure (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) -- some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably at least 30 nucleotides, although it may be shorter than this. Short primers generally require cooler temperatures to form sufficiently stable hybrid complexes with the template.

Probes may be produced by synthetic procedures, such as the triester method of Matteucci et al. (J. Am. Chem. Soc. (1981) 103:3185), or according to Urdea et al. (Proc. Natl. Acad. Sci. USA (1983) 80: 7461), or using commercially available automated oligonucleotide synthesizers.

The chemical nature of the probe can be selected according to preference. For certain applications, DNA or RNA are appropriate. For other applications, modifications may be incorporated e.g., backbone modifications, such as phosphorothioates or methylphosphonates, can be used to increase *in vivo* half-life, alter RNA affinity, increase nuclease resistance *etc.* (e.g., see Agrawal & Iyer (1995) Curr Opin Biotechnol 6:12-19; Agrawal (1996) TIBTECH 14:376-387); analogues such as peptide nucleic acids may also be used (e.g., see Corey (1997) TIBTECH 15:224-229; Buchardt et al. (1993) TIBTECH 11:384-386).

One example of a nucleotide hybridization assay is described by Urdea *et al.* in international patent application WO92/02526 (see also U.S. Patent 5,124,246).

Alternatively, the polymerase chain reaction (PCR) is another well-known means for detecting small amounts of target nucleic acids. The assay is described in: Mullis et al. (Meth. Enzymol. (1987) 155: 335-350); US patent 4,683,195; and US patent 4,683,202. Two "primer" nucleotides hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labeled probe will hybridize to the Neisserial sequence (or its complement).

Also, mRNA or cDNA can be detected by traditional blotting techniques described in Sambrook *et al (supra).* mRNA, or cDNA generated from mRNA using a polymerase enzyme, can be purified and separated using gel electrophoresis. The nucleic acids on the gel are then blotted onto a solid support, such as nitrocellulose. The solid support is exposed to a labeled probe and then washed to remove any unhybridized probe. Next, the duplexes containing the labeled probe are detected. Typically, the probe is labeled with a radioactive moiety.

## EXAMPLES

Aspects of the following examples that do not relate specifically to the claimed invention are included for illustration and comparison.

The disclosure is based on the 961 nucleotide sequences from the genome of *N. meningitidis* set out in Appendix C, SEQ ID NOs:1-961 of the '573 application, which together represent substantially the complete genome of serotype *B of N. meningitidis,* as well as the full length genome sequence shown in Appendix D, SEQ ID NO 1068 of the '573 application, and the full length genome sequence shown in Appendix A hereto, SEQ ID NO. 1.

It will be self-evident to the skilled person how this sequence information can be utilized according to the disclosure, as above described.

The standard techniques and procedures which may be employed in order to perform the disclosure (*e.g.* to utilize the disclosed sequences to predict polypeptides useful for vaccination or diagnostic purposes) were summarized above. This summary is not a limitation on the disclosure but, rather, gives examples that may be used, but are not required.

These sequences are derived from contigs shown in Appendix C (SEQ ID NOs 1-961) and from the full length genome sequence shown in Appendix D (SEQ ID NO 1068), which were prepared during the sequencing of the genome *of N. meningitidis* (strain B). The full length sequence was assembled using the TIGR Assembler as described by G.S. Sutton et al., TIGR Assembler: A New Tool for Assembling Large Shotgun Sequencing Projects, Genome Science and Technology, 1:9-19 (1995) [*see also* R. D. Fleischmann, et al., Science 269, 496-512 (1995); C. M. Fraser, et al., Science 270, 397-403 (1995); C. J. Bult, et al., Science 273, 1058-73 (1996); C. M. Fraser, et. al, Nature 390, 580-586 (1997); J.-F. Tomb, et. al., Nature 388, 539-547 (1997); H. P. Klenk, et al., Nature 390, 364-70 (1997); C. M. Fraser, et al., Science 281, 375-88 (1998); M. J. Gardner, et al., Science 282, 1126-1132-(1998); K. E. Nelson, et al., Nature 399, 323-9 (1999)]. Then, using the above-described methods, putative translation products of the sequences were determined. Computer analysis of the translation products were determined based on database comparisons. Corresponding gene and protein sequences, if any, were identified in Neisseria meningitidis (Strain A) and Neisseria gonorrhoeae. Then the proteins were expressed, purified, and characterized to assess their antigenicity and immunogenicity.

In particular, the following methods were used to express, purify, and biochemically characterize the proteins.

Chromosomal DNA Preparation

*N. meningitidis* strain 2996 was grown to exponential phase in 100 ml of GC medium, harvested by centrifugation, and resuspended in 5 ml buffer (20% Sucrose, 50 mM Tris-HCl, 50 mM EDTA, adjusted to pH 8.0). After 10 minutes incubation on ice, the bacteria were lysed by adding 10 ml lysis solution (50 mM NaCl, 1% Na-Sarkosyl, 50 μg/ml Proteinase K), and the suspension was incubated at 37°C for 2 hours. Two phenol extractions (equilibrated to pH 8) and one $ChCl_3$/isoamylalcohol (24:1) extraction were performed. DNA was precipitated by addition of 0.3M sodium acetate and 2 volumes ethanol, and was collected by centrifugation. The pellet was washed once with 70% ethanol and redissolved in 4 ml buffer (10 mM Tris-HCl, 1mM EDTA, pH 8). The DNA concentration was measured by reading the OD at 260 nm.

**Oligonucleotide design**

Synthetic oligonucleotide primers were designed on the basis of the coding sequence of each ORF, using (a) the meningococcus B sequence when available, or (b) the gonococcus/meningococcus A sequence, adapted to the codon preference usage of meningococcus. Any predicted signal peptides were omitted, by deducing the 5'-end amplification primer sequence immediately downstream from the predicted leader sequence.

For most ORFs, the 5' primers included two restriction enzyme recognition sites *(BamH*I-*Nde*I, *BamHI-Nhe*I, or *EcoR*I-*Nhe*I, depending on the gene's restriction pattern); the 3' primers included a *Xho*I restriction site. This procedure was established in order to direct the cloning of each amplification product (corresponding to each ORF) into two different expression systems: pGEX-KG (using either *BamH*I-*Xho*I or *EcoR*I-*Xho*I), and pET21b+ (using either *Nde*I-*Xho*I or *Nhe*I-*Xho*I).

| | | |
|---|---|---|
| 5'-end primer tail: | CGCGGATCCCATATG | (*BamH*I-*Nde*I) |
| | CGCGGATCCGCTAGC | (*BamH*I-*Nhe*I) |
| | CCGGAATTCTAGCTAGC | (*EcoR*I-*Nhe*I) |
| 3'-end primer tail: | CCCGCTCGAG | (*Xho*I) |

For some ORFs, two different amplifications were performed to clone each ORF in the two expression systems. Two different 5' primers were used for each ORF; the same 3' *Xho*I primer was used as before:

| | | |
|---|---|---|
| 5'-end primer tail: | GGAATTCCATATGGCCATGG | (*Nde*I) |
| 5'-end primer tail: | CGGGATCC | (*BamH*I) |

Other ORFs were cloned in the pTRC expression vector and expressed as an amino-terminus His-tag fusion. The predicted signal peptide may be included in the final product. *Nhe*I-*BamH*I restriction sites were incorporated using primers:

| | | |
|---|---|---|
| 5'-end primer tail: | GATCAGCTAGCCATATG | (*Nhe*I) |
| 3'-end primer tail: | CGGGATCC | (*BamH*I) |

As well as containing the restriction enzyme recognition sequences, the primers included nucleotides which hybridizeed to the sequence to be amplified. The number of hybridizing nucleotides depended on the melting temperature of the whole primer, and was determined for each primer using the formulae:

$$T_m = 4\ (G+C) + 2\ (A+T) \qquad \text{(tail excluded )}$$

(continued)

$$T_m = 64.9 + 0.41\ (\%\ GC) - 600/N \quad \text{(whole primer)}$$

The average melting temperature of the selected oligos were 65-70°C for the whole oligo and 50-55°C for the hybridising region alone.

Oligos were synthesized by a Perkin Elmer 394 DNA/RNA Synthesizer, eluted from the columns in 2 ml $NH_4$-OH, and deprotected by 5 hours incubation at 56°C. The oligos were precipitated by addition of 0.3M Na-Acetate and 2 volumes ethanol. The samples were then centrifuged and the pellets resuspended in either 100μl or 1ml of water. $OD_{260}$ was determined using a Perkin Elmer Lambda Bio spectophotometer and the concentration was determined and adjusted to 2-10 pmol/μl.

Table 1 shows the forward and reverse primers used for each amplification. In certain cases, it might be noted that the sequence of the primer does not exactly match the sequence in the ORF. When initial amplifications are performed, the complete 5' and/or 3' sequence may not be known for some meningococcal ORFs, although the corresponding sequences may have been identified in gonoccus. For amplification, the gonococcal sequences could thus be used as the basis for primer design, altered to take account of codon preference. In particular, the following codons may be changed: ATA→ATT; TCG→TCT; CAG→CAA; AAG→AAA; GAG→GAA; CGA and CGG→CGC; GGG→GGC.

## Amplification

The standard PCR protocol was as follows: 50-200 ng of genomic DNA were used as a template in the presence of 20-40 μM of each oligo, 400-800 μM dNTPs solution, 1x PCR buffer (including 1.5 mM $MgCl_2$), 2.5 units *TaqI* DNA polymerase (using Perkin-Elmer AmpliTaQ, GIBCO Platinum, Pwo DNA polymerase, or Tahara Shuzo Taq polymerase).

In some cases, PCR was optimsed by the addition of 10μl DMSO or 50 μl 2M betaine.

After a hot start (adding the polymerase during a preliminary 3 minute incubation of the whole mix at 95°C), each sample underwent a double-step amplification: the first 5 cycles were performed using as the hybridization temperature the one of the oligos excluding the restriction enzymes tail, followed by 30 cycles performed according to the hybridization temperature of the whole length oligos. The cycles were followed by a final 10 minute extension step at 72°C.

The standard cycles were as follows:

| | Denaturation | Hybridisation | Elongation |
|---|---|---|---|
| First 5 cycles | 30 seconds 95°C | 30 seconds 50-55°C | 30-60 seconds 72°C |
| Last 30 cycles | 30 seconds 95°C | 30 seconds 65-70°C | 30-60 seconds 72°C |

The elongation time varied according to the length of the ORF to be amplified.

The amplifications were performed using either a 9600 or a 2400 Perkin Elmer GeneAmp PCR System. To check the results, 1/10 of the amplification volume was loaded onto a 1-1.5% agarose gel and the size of each amplified fragment compared with a DNA molecular weight marker.

The amplified DNA was either loaded directly on a 1% agarose gel or first precipitated with ethanol and resuspended in a suitable volume to be loaded on a 1% agarose gel. The DNA fragment corresponding to the right size band was then eluted and purified from gel, using the Qiagen Gel Extraction Kit, following the instructions of the manufacturer. The final volume of the DNA fragment was 30μl or 50μl of either water or 10mM Tris, pH 8.5.

## Digestion of PCR fragments

The purified DNA corresponding to the amplified fragment was split into 2 aliquots and double-digested with:

NdeI/XhoI or *Nhe*I/*Xho*I for cloning into pET-21b+ and further expression of the protein as a C-terminus His-tag fusion
BamHI/XhoI or *EcoR*I/*Xho*I for cloning into pGEX-KG and further expression of the protein as a GST N-terminus fusion.
For ORF 76, *Nhe*I/*BamH*I for cloning into pTRC-HisA vector and further expression of the protein as N-terminus His-tag fusion.

Each purified DNA fragment was incubated (37°C for 3 hours to overnight) with 20 units of each restriction enzyme (New England Biolabs) in a either 30 or 40 μl final volume in the presence of the appropriate buffer. The digestion product was then purified using the QIAquick PCR purification kit, following the manufacturer's instructions, and eluted in a final volume of 30 (or 50) µl of either water or 10mum Tris-HCl, pH 8.5. The final DNA concentration was determined by 1% agarose gel electrophoresis in the presence of titrated molecular weight marker.

**Digestion of the cloning vectors (pET22B, pGEX-KG and pTRC-His A)**

10 µg plasmid was double-digested with 50 units of each restriction enzyme in 200 µl reaction volume in the presence of appropriate buffer by overnight incubation at 37°C. After loading the whole digestion on a 1% agarose gel, the band corresponding to the digested vector was purified from the gel using the Qiagen QIAquick Gel Extraction Kit and the DNA was eluted in 50 µl of 10 mM Tris-HCl, pH 8.5. The DNA concentration was evaluated by measuring $OD_{260}$ of the sample, and adjusted to 50 µg/µl. 1 µl of plasmid was used for each cloning procedure.

**Cloning**

The fragments corresponding to each ORF, previously digested and purified, were ligated in both pET22b and pGEX-KG. In a final volume of 20 µl, a molar ratio of 3:1 fragment/vector was ligated using 0.5 µl of NEB T4 DNA ligase (400 units/µl), in the presence of the buffer supplied by the manufacturer. The reaction was incubated at room temperature for 3 hours. In some experiments, ligation was performed using the Boheringer "Rapid Ligation Kit", following the manufacturer's instructions.

In order to introduce the recombinant plasmid in a suitable strain, 100 µl *E. coli* DH5 competent cells were incubated with the ligase reaction solution for 40 minutes on ice, then at 37°C for 3 minutes, then, after adding 800 µl LB broth, again at 37°C for 20 minutes. The cells were then centrifuged at maximum speed in an Eppendorf microfuge and resuspended in approximately 200 µl of the supernatant. The suspension was then plated on LB ampicillin (100 mg/ml).

The screening of the recombinant clones was performed by growing 5 randomly-chosen colonies overnight at 37 °C in either 2 ml (pGEX or pTC clones) or 5ml (pET clones) LB broth + 100 µg/ml ampicillin. The cells were then pelletted and the DNA extracted using the Qiagen prep Spin Miniprep Kit, following the manufacturer's instructions, to a final volume of 30 µl. 5 µl of each individual miniprep (approximately 1g) were digested with either *Nde*I/*Xho*I or *BamH*I/*Xho*I and the whole digestion loaded onto a 1-1.5% agarose gel (depending on the expected insert size), in parallel with the molecular weight marker (1Kb DNA Ladder, GIBCO). The screening of the positive clones was made on the base of the correct insert size.

**Cloning**

Certain ORFs may be cloned into the pGEX-HIS vector using *EcoR*I-*Pst*I, *EcoR*I-*Sal*I, or *Sal*I-*Pst*I cloning sites. After cloning, the recombinant plasmids may be introduced in the *E*.coli host W3110.

**Expression**

Each ORF cloned into the expression vector may then be transformed into the strain suitable for expression of the recombinant protein product. 1 µl of each construct was used to transform 30 µl of *E.coli* BL21 (pGEX vector), *E.coli* TOP 10 (pTRC vector) or *E.coli* BL21-DE3 (pET vector), as described above. In the case of the pGEX-His vector, the same *E.coli* strain (W3110) was used for initial cloning and expression. Single recombinant colonies were inoculated into 2ml LB+Amp (100 µg/ml), incubated at 37°C overnight, then diluted 1:30 in 20 ml of LB+Amp (100 µg/ml) in 100 ml flasks, making sure that the $OD_{600}$ ranged between 0.1 and 0.15. The flasks were incubated at 30°C into gyratory water bath shakers until OD indicated exponential growth suitable for induction of expression (0.4-0.8 OD for pET and pTRC vectors; 0.8-1 OD for pGEX and pGEX-His vectors). For the pET, pTRC and pGEX-His vectors, the protein expression was induced by addiction of 1mM IPTG, whereas in the case of pGEX system the final concentration of IPTG was 0.2 mM. After 3 hours incubation at 30°C, the final concentration of the sample was checked by OD. In order to check expression, 1ml of each sample was removed, centrifuged in a microfuge, the pellet resuspended in PBS, and analysed by 12% SDS-PAGE with Coomassie Blue staining. The whole sample was centrifuged at 6000g and the pellet resuspended in PBS for further use.

**GST-fusion proteins large-scale purification.**

A single colony was grown overnight at 37°C on LB+Amp agar plate. The bacteria were inoculated into 20 ml of LB+Amp liquid colture in a water bath shaker and grown overnight. Bacteria were diluted 1:30 into 600 ml of fresh medium and allowed to grow at the optimal temperature (20-37°C) to $OD_{550}$ 0.8-1. Protein expression was induced with 0.2mM IPTG followed by three hours incubation. The culture was centrifuged at 8000 rpm at 4°C. The supernatant was discarded and the bacterial pellet was resuspended in 7.5 ml cold PBS. The cells were disrupted by sonication on ice for 30 sec at 40W using a Branson sonifier B-15, frozen and thawed two times and centrifuged again. The supernatant was collected and mixed with 150μl Glutatione-Sepharose 4B resin (Pharmacia) (previously washed with PBS) and incubated at room temperature for 30 minutes. The sample was centrifuged at 700g for 5 minutes at 4C. The resin was washed twice with 10 ml cold PBS for 10 minutes, resuspended in 1ml cold PBS, and loaded on a disposable column. The resin was washed twice with 2ml cold PBS until the flow-through reached $OD_{280}$ of 0.02-0.06. The GST-fusion protein was eluted by addition of 700μl cold Glutathione elution buffer 10mM reduced glutathione, 50mM Tris-HCl) and fractions collected until the $OD_{280}$ was 0.1. 21μl of each fraction were loaded on a 12% SDS gel using either Biorad SDS-PAGE Molecular weight standard broad range (M1) (200, 116.25, 97.4, 66.2, 45, 31, 21.5, 14.4, 6.5 kDa) or Amersham Rainbow Marker (M") (220, 66, 46, 30, 21.5, 14.3 kDa) as standards. As the MW of GST is 26kDa, this value must be added to the MW of each GST-fusion protein.

**His-fusion soluble proteins large-scale purification.**

A single colony was grown overnight at 37°C on a LB + Amp agar plate. The bacteria were inoculated into 20ml of LB+Amp liquid culture and incubated overnight in a water bath shaker. Bacteria were diluted 1:30 into 600ml fresh medium and allowed to grow at the optimal temperature (20-37°C) to $OD_{550}$ 0.6-0.8. Protein expression was induced by addition of 1 mM IPTG and the culture further incubated for three hours. The culture was centrifuged at 8000 rpm at 4°C, the supernatant was discarded and the bacterial pellet was resuspended in 7.5ml cold 10mM imidazole buffer (300 mM NaCl, 50 mM phosphate buffer, 10 mM imidazole, pH 8). The cells were disrupted by sonication on ice for 30 sec at 40W using a Branson sonifier B-15, frozen and thawed two times and centrifuged again. The supernatant was collected and mixed with 150μl $Ni^{2+}$-resin (Pharmacia) (previously washed with 10mM imidazole buffer) and incubated at room temperature with gentle agitation for 30 minutes. The sample was centrifuged at 700g for 5 minutes at 4°C. The resin was washed twice with 10 ml cold 10mM imidazole buffer for 10 minutes, resuspended in 1ml cold 10mM imidazole buffer and loaded on a disposable column. The resin was washed at 4°C with 2ml cold 10mM imidazole buffer until the flow-through reached the $O.D_{280}$ of 0.02-0.06. The resin was washed with 2ml cold 20mM imidazole buffer (300 mM NaCl, 50 mM phosphate buffer, 20 mM imidazole, pH 8) until the flow-through reached the $O.D_{280}$ of 0.02-0.06. The His-fusion protein was eluted by addition of 700μl cold 250mM imidazole buffer (300 mM NaCl, 50 mM phosphate buffer, 250 mM imidazole, pH 8) and fractions collected until the $O.D_{280}$ was 0.1. 21μl of each fraction were loaded on a 12% SDS gel.

**His-fusion insoluble proteins large-scale purification.**

A single colony was grown overnight at 37 °C on a LB + Amp agar plate. The bacteria were inoculated into 20 ml of LB+Amp liquid culture in a water bath shaker and grown overnight. Bacteria were diluted 1:30 into 600ml fresh medium and let to grow at the optimal temperature (37°C) to $O.D_{550}$ 0.6-0.8. Protein expression was induced by addition of 1 mM IPTG and the culture further incubated for three hours. The culture was centrifuged at 8000rpm at 4°C. The supernatant was discarded and the bacterial pellet was resuspended in 7.5 ml buffer B (urea 8M, 10mM Tris-HCl, 100mM phosphate buffer, pH 8.8). The cells were disrupted by sonication on ice for 30 sec at 40W using a Branson sonifier B-15, frozen and thawed twice and centrifuged again. The supernatant was stored at -20°C, while the pellets were resuspended in 2 ml guanidine buffer (6M guanidine hydrochloride, 100mM phosphate buffer, 10 mM Tris-HCl, pH 7.5) and treated in a homogenizer for 10 cycles. The product was centrifuged at 13000 rpm for 40 minutes. The supernatant was mixed with 150μl $Ni^{2+}$-resin (Pharmacia) (previously washed with buffer B) and incubated at room temperature with gentle agitation for 30 minutes. The sample was centrifuged at 700 g for 5 minutes at 4°C. The resin was washed twice with 10 ml buffer B for 10 minutes, resuspended in 1ml buffer B, and loaded on a disposable column. The resin was washed at room temperature with 2ml buffer B until the flow-through reached the $OD_{280}$ of 0.02-0.06. The resin was washed with 2ml buffer C (urea 8M, 10mM Tris-HCl, 100mM phosphate buffer, pH 6.3) until the flow-through reached the $O.D_{280}$ of 0.02-0.06. The His-fusion protein was eluted by addition of 700μl elution buffer (urea 8M, 10mM Tris-HCl, 100mM phosphate buffer, pH 4.5) and fractions collected until the $OD_{280}$ was 0.1. 21μl of each fraction were loaded on a 12% SDS gel.

**His-fusion proteins renaturation**

10% glycerol was added to the denatured proteins. The proteins were then diluted to 20μg/ml using dialysis buffer I (10% glycerol, 0.5M arginine, 50mM phosphate buffer, 5mM reduced glutathione, 0.5mM oxidised glutathione, 2M urea, pH 8.8) and dialysed against the same buffer at 4°C for 12-14 hours. The protein was further dialysed against dialysis buffer II (10% glycerol, 0.5M arginine, 50mM phosphate buffer, 5mM reduced glutathione, 0.5mM oxidised glutathione, pH 8.8) for 12-14 hours at 4°C. Protein concentration was evaluated using the formula:

$$\text{Protein (mg/ml)} = (1.55 \times OD_{280}) - (0.76 \times OD_{260})$$

**Mice immunisations**

20μg of each purified protein were used to immunise mice intraperitoneally. In the case of some ORFs, Balb-C mice were immunised with $Al(OH)_3$ as adjuvant on days 1, 21 and 42, and immune response was monitored in samples taken on day 56. For other ORFs, CD1 mice could be immunised using the same protocol. For other ORFs, CD1 mice could be immunised using Freund's adjuvant, and the same immunisation protocol was used, except that the immune response was measured on day 42, rather than 56. Similarly, for still other ORFs, CD1 mice could be immunised with Freund's adjuvant, but the immune response was measured on day 49.

**ELISA assay (sera analysis)**

The acapsulated MenB M7 strain was plated on chocolate agar plates and incubated overnight at 37°C. Bacterial colonies were collected from the agar plates using a sterile dracon swab and inoculated into 7ml of Mueller-Hinton Broth (Difco) containing 0.25% Glucose. Bacterial growth was monitored every 30 minutes by following $OD_{620}$. The bacteria were let to grow until the OD reached the value of 0.3-0.4. The culture was centrifuged for 10 minutes at 10000 rpm. The supernatant was discarded and bacteria were washed once with PBS, resuspended in PBS containing 0.025% formaldehyde, and incubated for 2 hours at room temperature and then overnight at 4°C with stirring. 100μl bacterial cells were added to each well of a 96 well Greiner plate and incubated overnight at 4°C. The wells were then washed three times with PBT washing buffer (0.1% Tween-20 in PBS). 200 μl of saturation buffer (2.7% Polyvinylpyrrolidone 10 in water) was added to each well and the plates incubated for 2 hours at 37°C. Wells were washed three times with PBT. 200 μl of diluted sera (Dilution buffer: 1% BSA, 0.1% Tween-20, 0.1% $NaN_3$ in PBS) were added to each well and the plates incubated for 90 minutes at 37°C. Wells were washed three times with PBT. 100 μl of HRP-conjugated rabbit anti-mouse (Dako) serum diluted 1:2000 in dilution buffer were added to each well and the plates were incubated for 90 minutes at 37°C. Wells were washed three times with PBT buffer. 100 μl of substrate buffer for HRP (25 ml of citrate buffer pH5, 10 mg of O-phenildiamine and 10 μl of $H_2O$) were added to each well and the plates were left at room temperature for 20 minutes. 100 μl $H_2SO_4$ was added to each well and $OD_{490}$ was followed. The ELISA was considered positive when OD490 was 2.5 times the respective pre-immune sera.

**FACScan bacteria Binding Assay procedure.**

The acapsulated MenB M7 strain was plated on chocolate agar plates and incubated overnight at 37°C. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000 rpm. The supernatant was discarded and the pellet was resuspended in blocking buffer (1% BSA, 0.4% $NaN_3$) and centrifuged for 5 minutes at 4000 rpm. Cells were resuspended in blocking buffer to reach $OD_{620}$ of 0.07. 100μl bacterial cells were added to each well of a Costar 96 well plate. 100μl of diluted (1:200) sera (in blocking buffer) were added to each well and plates incubated for 2 hours at 4°C. Cells were centrifuged for 5 minutes at 4000 rpm, the supernatant aspirated and cells washed by addition of 200μl/well of blocking buffer in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/well of PBS, 0.25% formaldehyde. Samples were transferred to FACScan tubes and read. The condition for FACScan setting were: FL1 on, FL2 and FL3 off; FSC-H Treshold:92; FSC PMT Voltage: E 02; SSC PMT: 474; Amp. Gains 7.1; FL-2 PMT: 539. Compensation values: 0.

**OMV preparations**

Bacteria were grown overnight on 5 GC plates, harvested with a loop and resuspended in 10 ml 20mM Tris-HCl. Heat inactivation was performed at 56°C for 30 minutes and the bacteria disrupted by sonication for 10' on ice ( 50% duty cycle, 50% output ). Unbroken cells were removed by centrifugation at 5000g for 10 minutes and the total cell envelope fraction recovered by centrifugation at 50000g at 4°C for 75 minutes. To extract cytoplasmic membrane proteins from the crude outer membranes, the whole fraction was resuspended in 2% sarkosyl (Sigma) and incubated at room temperature for 20 minutes. The suspension was centrifuged at 10000g for 10 minutes to remove aggregates, and the supernatant further ultracentrifuged at 50000g for 75 minutes to pellet the outer membranes. The outer membranes were resuspended in 10mM Tris-HCl, pH8 and the protein concentration measured by the Bio-Rad Protein assay, using BSA as a standard.

**Whole Extracts preparation**

Bacteria were grown overnight on a GC plate, harvested with a loop and resuspended in 1ml of 20mM Tris-HCl. Heat inactivation was performed at 56°C for 30' minutes.

**Western blotting**

Purified proteins (500ng/lane), outer membrane vesicles (5 μg) and total cell extracts (25μg) derived from MenB strain 2996 were loaded on 15% SDS-PAGE and transferred to a nitrocellulose membrane. The transfer was performed for 2 hours at 150mA at 4°C, in transferring buffer (0.3 % Tris base, 1.44 % glycine, 20% methanol). The membrane was saturated by overnight incubation at 4°C in saturation buffer (10% skimmed milk, 0.1% Triton X100 in PBS). The membrane was washed twice with washing buffer (3% skimmed milk, 0.1% Triton X100 in PBS) and incubated for 2 hours at 37°C with 1:200 mice sera diluted in washing buffer. The membrane was washed twice and incubated for 90 minutes with a 1:2000 dilution of horseradish peroxidase labeled anti-mouse Ig. The membrane was washed twice with 0.1% Triton X100 in PBS and developed with the Opti-4CN Substrate Kit (Bio-Rad). The reaction was stopped by adding water.

**Bactericidal assay**

MC58 strain was grown overnight at 37°C on chocolate agar plates. 5-7 colonies were collected and used to inoculate 7ml Mueller-Hinton broth. The suspension was incubated at 37°C on a nutator and let to grow until $OD_{620}$ was in between 0.5-0.8. The culture was aliquoted into sterile 1.5ml Eppendorf tubes and centrifuged for 20 minutes at maximum speed in a microfuge. The pellet was washed once in Gey's buffer (Gibco) and resuspended in the same buffer to an $OD_{620}$ of 0.5, diluted 1:20000 in Gey's buffer and stored at 25°C.

50μl of Gey's buffer/1% BSA was added to each well of a 96-well tissue culture plate. 25μl of diluted (1:100) mice sera (dilution buffer: Gey's buffer/0.2% BSA) were added to each well and the plate incubated at 4°C. 25μl of the previously described bacterial suspension were added to each well. 25μl of either heat-inactivated (56°C waterbath for 30 minutes) or normal baby rabbit complement were added to each well. Immediately after the addition of the baby rabbit complement, 22μl of each sample/well were plated on Mueller-Hinton agar plates (time 0). The 96-well plate was incubated for 1 hour at 37°C with rotation and then 22μl of each sample/well were plated on Mueller-Hinton agar plates (time 1). After overnight incubation the colonies corresponding to time 0 and time 1h were counted.

The following DNA and amino acid sequences are identified by titles of the following form: [g, m, or a] [#].[seq or pep], where "g" means a sequence from *N. gonorrhoeae,* "m" means a sequence from *N. meningitidis B,* and "a" means a sequence from *N. meningitidis A;* "#" means the number of the sequence; "seq" means a DNA sequence, and "pep" means an amino acid sequence. For example, "g001.seq" refers to an *N. gonorrohoeae* DNA sequence, number 1. The presence of the suffix "-1" or "-2" to these sequences indicates an additional sequence found for the same ORF. Further, open reading frames are identified as ORF #, where "#" means the number of the ORF, corresponding to the number of the sequence which encodes the ORF, and the ORF designations may be suffixed with ".ng" or ".a", indicating that the ORF corresponds to a *N. gonorrhoeae* sequence or a *N. meningitidis A* sequence, respectively. Computer analysis was performed for the comparisons that follow between "g", "m", and "a" peptide sequences; and therein the "pep" suffix is implied where not expressly stated.

EXAMPLE 1

The following ORFs were predicted from the contig sequences and/or the full length sequences using the methods herein described.

**Localization of the ORFs**

| ORF: | contig: |
|---|---|
| 279 | gnm4.seq |

The following partial DNA sequence was identified in *N. meningitidis* <SEQ ID 2>:

```
m279.seq
  1  ATAACGCGGA TTTGCGGCTG CTTGATTTCA ACGGTTTTCA GGGCTTCGGC
 51  AAGTTTGTCG GCGGCGGGTT TCATCAGGCT GCAATGGGAA GGTACGGACA
101  CGGGCAGCGG CAGGGCGCGT TTGGCACCGG CTTCTTTGGC GGCAGCCATG
151  GCGCGTCCGA CGGCGGCGGC GTTGCCTGCA ATCACGATTT GTCCGGGTGA
201  GTTGAAGTTG ACGGCTTCGA CCACTTCGCT TTGGGCGGCT TCGGCACAAA
251  TGGCTTTAAC CTGCTCATCT TCCAAGCCGA GAATCGCCGC CATTGCGCCC
301  ACGCCTTGCG GTACGGCGGA CTGCATCAGT TCGGCGCGCA GGCGCACGAG
351  TTTGACCGCG TCGGCAAAAT TCAATGCGCC GGCGGCAACG AGTGCGGTGT
401  ATTCGCCGAG GCTGTGTCCG GCAACGGCGG CAGGCGTTTT GCCGCCCGCT
451  TCTAAATAG
```

This corresponds to the amino acid sequence <SEQ ID 3; ORF 279>:

```
m279.pep
  1  ITRICGCLIS TVFRASASLS AAGFIRLQWE GTDTGSGRAR LAPASLAAAM
 51  ARPTAAALPA ITICPGELKL TASTTSLWAA SAQMALTCSS SKPRIAAIAP
101  TPCGTADCIS SARRRTSLTA SAKFNAPAAT SAVYSPRLCP ATAAGVLPPA
151  SK*
```

The following partial DNA sequence was identified in *N.gonorrhoeae* <SEQ ID 4>:

```
g279.seq
  1  atgacgcgga tttgcggctg cttgatttca acggttttga gtgtttcggc
 51  aagtttgtcg gcggcgggtt tcatcaggct gcaatgggaa ggaacggata
101  ccggcagcgg cagggcgcgt ttggctccgg cttctttggc ggcagccatg
151  gtgcgtccga cggcggcggc gttgcctgca atcacgactt gtccgggcga
201  gttgaagttg acggcttcga ccacttcgcc ctgtgcggat tcggcacaaa
251  tctgcctgac ctgttcatct tccaaaccca aaatggccgc cattgcgcct
301  acgccttgcg gtacggcgga ctgcatcagt tcggcgcgca ggcggacgag
351  tttgacggca tcggcaaaat ccaatgcttc ggcggcgaca agcgcggtgt
401  attcgccgag gctgtgtccg gcaacggcgg caggcgtttt gccgcccact
451  tccaaatag
```

This corresponds to the amino acid sequence <SEQ ID 5; ORF 279.ng>:

```
g279.pep
      1  MTRICGCLIS TVLSVSASLS AAGFIRLQWE GTDTGSGRAR LAPASLAAAM
     51  VRPTAAALPA ITTCPGELKL TASTTSPCAD SAQICLTCSS SKPKMAAIAP
    101  TPCGTADCIS SARRRTSLTA SAKSNASAAT SAVYSPRLCP ATAAGVLPPT
    151  SK*
```

ORF 279 shows 89.5% identity over a 152 aa overlap with a predicted ORF (ORF 279.ng) from *N. gonorrhoeae:*

```
                       10        20        30        40        50        60
m279.pep     ITRICGCLISTVFRASASLSAAGFIRLQWEGTDTGSGRARLAPASLAAAMARPTAAALPA
             :||||||||||:  :||||||||||||||||||||||||||||||||||||:|||||||||
g279         MTRICGCLISTVLSVSASLSAAGFIRLQWEGTDTGSGRARLAPASLAAAMVRPTAAALPA
                       10        20        30        40        50        60

                       70        80        90       100       110       120
m279.pep     ITICPGELKLTASTTSLWAASAQMALTCSSSKPRIAAIAPTPCGTADCISSARRRTSLTA
             || ||||||||||||   | |||: ||||||||::||||||||||||||||||||||||||
g279         ITTCPGELKLTASTTSPCADSAQICLTCSSSKPKMAAIAPTPCGTADCISSARRRTSLTA
                       70        80        90       100       110       120

                      130       140       150
m279.pep     SAKFNAPAATSAVYSPRLCPATAAGVLPPASKX
             ||| || ||||||||||||||||||||||:|||
g279         SAKSNASAATSAVYSPRLCPATAAGVLPPTSKX
                      130       140       150
```

The following partial DNA sequence was identified in *N. meningitidis* <SEQ ID 6>:

```
a279.seq
      1  ATGACNCNGA TTTGCGGCTG CTTGATTTCA ACGGTTTNNA GGGCTTCGGC
     51  GAGTTTGTCG GCGGCGGGTT TCATGAGGCT GCAATGGGAA GGTACNGACA
    101  CNGGCAGCGG CAGGGCGCGT TTGGCGCCGG CTTCTTTGGC GGCAAGCATA
    151  GCGCGCTCGA CGGCGGCGGC ATTGCCTGCA ATCACGACTT GTCCGGGCGA
    201  GTTGAAGTTG ACGGCTTCAA CCACTTCATC CTGTGCGGAT TCGGCGCAAA
    251  TTTGTTTTAC CTGTTCATCT TCCAAGCCGA GAATCGCCGC CATTGCGCCC
    301  ACGCCTTGCG GTACGGCGGA CTGCATCAGT TCGGCGCGCA NGCGCACGAG
    351  TTTGACCGCG TCGGCAAAAT CCAATGCGCC GGCGGCAACN AGTGCGGTGT


    401  ATTCGCCGAN GCTGTGTCCG GCAACGGCGG CAGGCGTTTT GCCGCCCGCT
    451  TCCGAATAG
```

This corresponds to the amino acid sequence <SEQ ID 7; ORF 279.a>:

```
a279.pep
      1  MTXICGCLIS TVXRASASLS AAGFMRLQWE GTDTGSGRAR LAPASLAASI
     51  ARSTAAALPA ITTCPGELKL TASTTSSCAD SAQICFTCSS SKPRIAAIAP
    101  TPCGTADCIS SARXRTSLTA SAKSNAPAAT SAVYSPXLCP ATAAGVLPPA
    151  SE*
```

m279/a279 ORFs 279 and 279.a showed a 88.2% identity in 152 aa overlap

```
                    10        20        30        40        50        60
m279.pep    ITRICGCLISTVFRASASLSAAGFIRLQWEGTDTGSGRARLAPASLAAAMARPTAAALPA
            :| |||||||| ||||||||||:|||||||||||||||||||||||::|| |||||||
a279        MTXICGCLISTVXRASASLSAAGFMRLQWEGTDTGSGRARLAPASLAASIARSTAAALPA
                    10        20        30        40        50        60

                    70        80        90       100       110       120
m279.pep    ITICPGELKLTASTTSLWAASAQMALTCSSSKPRIAAIAPTPCGTADCISSARRRTSLTA
            || |||||||||||||   | |||: :|||||||||||||||||||||||||| ||||||
a279        ITTCPGELKLTASTTSSCADSAQICFTCSSSKPRIAAIAPTPCGTADCISSARXRTSLTA
                    70        80        90       100       110       120

                   130       140       150
m279.pep    SAKFNAPAATSAVYSPRLCPATAAGVLPPASKX
            ||| |||||||||||| ||||||||||||||:|
a279        SAKSNAPAATSAVYSPXLCPATAAGVLPPASEX
                   130       140       150
```

## 519 and 519-1 gnm7.seq

The following partial DNA sequence was identified in *N. meningitidis* <SEQ ID 8>:

```
m519.seq  (partial)
      1  ..TCCGTTATCG GGCGTATGGA GTTGGACAAA ACGTTTGAAG AACGCGACGA
     51    AATCAACAGT ACTGTTGTTG CGGCTTTGGA CGAGGCGGCC GGGgCTTgGG
    101    GTGTGAAGGT TTTGCGTTAT GAGATTAAAG ACTTGGTTCC GCCGCAAGAA
    151    ATCCTTCGCT CAATGCAGGC GCAAATTACT GCCGAACGCG AAAAACGCGC
    201    CCGTATCGCC GAATCCGAAG GTCGTAAAAT CGAACAAATC AACCTTGCCA
    251    GTGGTCAGCG CGAAGCCGAA ATCCAACAAT CCGAAGGCGA GGCTCAGGCT
    301    GCGGTCAATG CGTCAAATGC CGAGAAAATC GCCCGCATCA ACCGCGCCAA
    351    AGGTGAAGCG GAATCCTTGC GCCTTGTTGC CGAAGCCAAT GCCGAAGCCA
    401    TCCGTCAAAT TGCCGCCGCC CTTCAAACCC AAGGCGGTGC GGATGCGGTC
    451    AATCTGAAGA TTGCGGAACA ATACGTCGCT GCGTTCAACA ATCTTGCCAA
    501    AGAAAGCAAT ACGCTGATTA TGCCCGCCAA TGTTGCCGAC ATCGGCAGCC
    551    TGATTTCTGC CGGTATGAAA ATTATCGACA GCAGCAAAAC CGCCAAaTAA
```

This corresponds to the amino acid sequence <SEQ ID 9; ORF 519>:

```
m519.pep  (partial)
      1  ..SVIGRMELDK TFEERDEINS TVVAALDEAA GAWGVKVLRY EIKDLVPPQE
     51    ILRSMQAQIT AEREKRARIA ESEGRKIEQI NLASGQREAE IQQSEGEAQA
    101    AVNASNAEKI ARINRAKGEA ESLRLVAEAN AEAIRQIAAA LQTQGGADAV
    151    NLKIAEQYVA AFNNLAKESN TLIMPANVAD IGSLISAGMK IIDSSKTAK*
```

The following partial DNA sequence was identified in *N. gonorrhoeae* <SEQ ID 10>:

```
g519.seq
      1  atggaatttt tcattatctt gttggcagcc gtcgccgttt tcggcttcaa
     51  atcctttgtc gtcatccccc agcaggaagt ccacgttgtc gaaaggctcg
```

```
101  ggcgtttcca tcgcgccctg acggccggtt tgaatatttt gattcccttt
151  atcgaccgcg tcgcctaccg ccattcgctg aaagaaatcc ctttagacgt
201  acccagccag gtctgcatca cgcgcgataa tacgcaattg actgttgacg
251  gcatcatcta tttccaagta accgatccca aactcgcctc atacggttcg
301  agcaactaca ttatggcaat tacccagctt gcccaaacga cgctgcgttc
351  cgttatcggg cgtatggagt tggacaaaac gtttgaagaa cgcgacgaaa
401  tcaacagtac cgtcgtctcc gccctcgatg aagccgccgg ggcttggggt
451  gtgaaagtcc tccgttacga aatcaaggat ttggttccgc cgcaagaaat
501  ccttcgcgca atgcaggcac aaattaccgc cgaacgcgaa aaacgcgccc
551  gtattgccga atccgaaggc cgtaaaatcg aacaaatcaa ccttgccagt
601  ggtcagcgtg aagccgaaat ccaacaatcc gaaggcgagg ctcaggctgc
651  ggtcaatgcg tccaatgccg agaaaatcgc ccgcatcaac cgcgccaaag
701  gcgaagcgga atccctgcgc cttgttgccg aagccaatgc cgaagccaac
751  cgtcaaattg ccgccgccct tcaaacccaa agcggggcgg atgcggtcaa
801  tctgaagatt gcgggacaat acgttaccgc gttcaaaaat cttgccaaag
851  aagacaatac gcggattaag cccgccaagg ttgccgaaat cgggaacccct
901  aattttcggc ggcatgaaaa attttcgcca gaagcaaaaa cggccaaata
951  a
```

This corresponds to the amino acid sequence <SEQ ID 11; ORF 519.ng>:

```
g519.pep
    1  MEFFIILLAA VAVFGFKSFV VIPQQEVHVV ERLGRFHRAL TAGLNILIPF
   51  IDRVAYRHSL KEIPLDVPSQ VCITRDNTQL TVDGIIYFQV TDPKLASYGS
  101  SNYIMAITQL AQTTLRSVIG RMELDKTFEE RDEINSTVVS ALDEAAGAWG
  151  VKVLRYEIKD LVPPQEILRA MQAQITAERE KRARIAESEG RKIEQINLAS
  201  GQREAEIQQS EGEAQAAVNA SNAEKIARIN RAKGEAESLR LVAEANAEAN
  251  RQIAAALQTQ SGADAVNLKI AGQYVTAFKN LAKEDNTRIK PAKVAEIGNP
  301  NFRRHEKFSP EAKTAK*
```

ORF 519 shows 87.5% identity over a 200aa overlap with a predicted ORF (ORF 519.ng) from *N.gonorrhoeae*:

```
m519/g519

                                                      10        20        30
m519.pep                                      SVIGRMELDKTFEERDEINSTVVAALDEAA
                                              |||||||||||||||||||||||:||||||
g519            YFQVTDPKLASYGSSNYIMAITQLAQTTLRSVIGRMELDKTFEERDEINSTVVSALDEAA
                  90       100       110       120       130       140

                        40        50        60        70        80        90
m519.pep        GAWGVKVLRYEIKDLVPPQEILRSMQAQITAEREKRARIAESEGRKIEQINLASGQREAE
                |||||||||||||||||||||||:||||||||||||||||||||||||||||||||||||
g519            GAWGVKVLRYEIKDLVPPQEILRAMQAQITAEREKRARIAESEGRKIEQINLASGQREAE
                 150       160       170       180       190       200

                       100       110       120       130       140       150
m519.pep        IQQSEGEAQAAVNASNAEKIARINRAKGEAESLRLVAEANAEAIRQIAAALQTQGGADAV
                ||||||||||||||||||||||||||||||||||||||||||| ||||||||||:|||||
g519            IQQSEGEAQAAVNASNAEKIARINRAKGEAESLRLVAEANAEANRQIAAALQTQSGADAV
                 210       220       230       240       250       260

                       160       170       180       190       200
m519.pep        NLKIAEQYVAAFNNLAKESNTLIMPANVADIGSL-ISAGMKIIDSSKTAK
                ||||| |||:||:|||||:|| | ||:||:||:  :    |:   :||||
g519            NLKIAGQYVTAFKNLAKEDNTRIKPAKVAEIGNPNFRRHEKFSPEAKTAK
                 270       280       290       300       310
```

The following partial DNA sequence was identified in *N. meningitidis* <SEQ ID 12>:

a519.seq

```
  1 ATGGAATTTT TCATTATCTT GCTGGCAGCC GTCGTTGTTT TCGGCTTCAA
 51 ATCCTTTGTT GTCATCCCAC AGCAGGAAGT CCACGTTGTC GAAAGGCTCG
101 GGCGTTTCCA TCGCGCCCTG ACGGCCGGTT TGAATATTTT GATTCCCTTT
151 ATCGACCGCG TCGCCTACCG CCATTCGCTG AAAGAAATCC CTTTAGACGT
201 ACCCAGCCAG GTCTGCATCA CGCGCGACAA TACGCAGCTG ACTGTTGACG
251 GTATCATCTA TTTCCAAGTA ACCGACCCCA AACTCGCCTC ATACGGTTCG
301 AGCAACTACA TTATGGCGAT TACCCAGCTT GCCCAAACGA CGCTGCGTTC
351 CGTTATCGGG CGTATGGAAT TGGACAAAAC GTTTGAAGAA CGCGACGAAA
401 TCAACAGCAC CGTCGTCTCC GCCCTCGATG AAGCCGCCGG AGCTTGGGGT
451 GTGAAGGTTT TGCGTTATGA GATTAAAGAC TTGGTTCCGC CGCAAGAAAT
501 CCTTCGCTCA ATGCAGGCGC AAATTACTGC TGAACGCGAA AAACGCGCCC
551 GTATCGCCGA ATCCGAAGGT CGTAAAATCG AACAAATCAA CCTTGCCAGT
601 GGTCAGCGCG AAGCCGAAAT CCAACAATCC GAAGGCGAGG CTCAGGCTGC
651 GGTCAATGCG TCAAATGCCG AGAAAATCGC CCGCATCAAC CGCGCCAAAG
701 GTGAAGCGGA ATCCTTGCGC CTTGTTGCCG AAGCCAATGC CGAAGCCATC
751 CGTCAAATTG CCGCCGCCCT TCAAACCCAA GGCGGTGCGG ATGCGGTCAA
801 TCTGAAGATT GCGGAACAAT ACGTCGCCGC GTTCAACAAT CTTGCCAAAG
851 AAAGCAATAC GCTGATTATG CCCGCCAATG TTGCCGACAT CGGCAGCCTG
901 ATTTCTGCCG GTATGAAAAT TATCGACAGC AGCAAAACCG CCAAATAA
```

This corresponds to the amino acid sequence <SEQ ID 13; ORF 519.a>:

a519.pep

```
  1 MEFFIILLAA VVVFGFKSFV VIPQQEVHVV ERLGRFHRAL TAGLNILIPF
 51 IDRVAYRHSL KEIPLDVPSQ VCITRDNTQL TVDGIIYFQV TDPKLASYGS
101 SNYIMAITQL AQTTLRSVIG RMELDKTFEE RDEINSTVVS ALDEAAGAWG
151 VKVLRYEIKD LVPPQEILRS MQAQITAERE KRARIAESEG RKIEQINLAS
201 GQREAEIQQS EGEAQAAVNA SNAEKIARIN RAKGEAESLR LVAEANAEAI
251 RQIAAALQTQ GGADAVNLKI AEQYVAAFNN LAKESNTLIM PANVADIGSL
301 ISAGMKIIDS SKTAK*
```

**m519/a519** **ORFs 519 and 519.a showed a** 99.5% identity in 199 aa overlap

```
                                                10        20        30
m519.pep                                SVIGRMELDKTFEERDEINSTVVAALDEAA
                                        ||||||||||||||||||||||:|||||||
a519            YFQVTDPKLASYGSSNYIMAITQLAQTTLRSVIGRMELDKTFEERDEINSTVVSALDEAA
                  90       100       110       120       130       140

                       40        50        60        70        80        90
m519.pep        GAWGVKVLRYEIKDLVPPQEILRSMQAQITAEREKRARIAESEGRKIEQINLASGQREAE
                ||||||||||||||||||||||||||||||||||||||||||||||||||||||||||||
a519            GAWGVKVLRYEIKDLVPPQEILRSMQAQITAEREKRARIAESEGRKIEQINLASGQREAE
                  150       160       170       180       190       200

                      100       110       120       130       140       150
m519.pep        IQQSEGEAQAAVNASNAEKIARINRAKGEAESLRLVAEANAEAIRQIAAALQTQGGADAV
                ||||||||||||||||||||||||||||||||||||||||||||||||||||||||||||
a519            IQQSEGEAQAAVNASNAEKIARINRAKGEAESLRLVAEANAEAIRQIAAALQTQGGADAV
                  210       220       230       240       250       260

                      160       170       180       190       200
m519.pep        NLKIAEQYVAAFNNLAKESNTLIMPANVADIGSLISAGMKIIDSSKTAKX
                ||||||||||||||||||||||||||||||||||||||||||||||||||
a519            NLKIAEQYVAAFNNLAKESNTLIMPANVADIGSLISAGMKIIDSSKTAKX
                  270       280       290       300       310
```

Further work revealed the following DNA sequence identified in *N. meningitidis* <SEQ ID 14>:

```
m519-1.seq

  1  ATGGAATTTT TCATTATCTT GTTGGTAGCC GTCGCCGTTT TCGGTTTCAA
 51  ATCCTTTGTT GTCATCCCAC AACAGGAAGT CCACGTTGTC GAAAGGCTGG
101  GGCGTTTCCA TCGCGCCCTG ACGGcCGGTT TGAATATTTT GATTCCCTTT
151  ATCGACCGCG TCGCCTACCG CCATTCGCTG AAAGAAATCC CTTTAGACGT
201  ACCCAGCCAG GTCTGCATCA CGCGCGACAA TACGCAGCTG ACTGTTGACG
251  GCATCATCTA TTTCCAAGTA ACCGACCCCA AACTCGCCTC ATACGGTTCG
301  AGCAACTACA TTATGGCGAT TACCCAGCTT GCCCAAACGA CGCTGCGTTC
351  CGTTATCGGG CGTATGGAGT TGGACAAAAC GTTTGAAGAA CGCGACGAAA
401  TCAACAGTAC TGTTGTTGCG GCTTTGGACG AGGCGGCCGG GGCTTGGGGT
451  GTGAAGGTTT TGCGTTATGA GATTAAAGAC TTGGTTCCGC CGCAAGAAAT
501  CCTTCGCTCA ATGCAGGCGC AAATTACTGC CGAACGCGAA AAACGCGCCC
551  GTATCGCCGA ATCCGAAGGT CGTAAAATCG AACAAATCAA CCTTGCCAGT
601  GGTCAGCGCG AAGCCGAAAT CCAACAATCC GAAGGCGAGG CTCAGGCTGC
651  GGTCAATGCG TCAAATGCCG AGAAAATCGC CCGCATCAAC CGCGCCAAAG
701  GTGAAGCGGA ATCCTTGCGC CTTGTTGCCG AAGCCAATGC CGAAGCCATC
751  CGTCAAATTG CCGCCGCCCT TCAAACCCAA GGCGGTGCGG ATGCGGTCAA
801  TCTGAAGATT GCGGAACAAT ACGTCGCTGC GTTCAACAAT CTTGCCAAAG
851  AAAGCAATAC GCTGATTATG CCCGCCAATG TTGCCGACAT CGGCAGCCTG
901  ATTTCTGCCG GTATGAAAAT TATCGACAGC AGCAAAACCG CCAAATAA
```

This corresponds to the amino acid sequence <SEQ ID 15; ORF 519-1>:

```
m519-1.
  1  MEFFIILLVA VAVFGFKSFV VIPQQEVHVV ERLGRFHRAL TAGLNILIPF
 51  IDRVAYRHSL KEIPLDVPSQ VCITRDNTQL TVDGIIYFQV TDPKLASYGS
101  SNYIMAITQL AQTTLRSVIG RMELDKTFEE RDEINSTVVA ALDEAAGAWG
151  VKVLRYEIKD LVPPQEILRS MQAQITAERE KRARIAESEG RKIEQINLAS
201  GQREAEIQQS EGEAQAAVNA SNAEKIARIN RAKGEAESLR LVAEANAEAI
251  RQIAAALQTQ GGADAVNLKI AEQYVAAFNN LAKESNTLIM PANVADIGSL
301  ISAGMKIIDS SKTAK*
```

The following DNA sequence was identified in *N. gonorrhoeae* <SEQ ID 16>:

```
g519-1.seq
  1  ATGGAATTTT TCATTATCTT GTTGGCAGCC GTCGCCGTTT TCGGCTTCAA
 51  ATCCTTTGTC GTCATCCCCC AGCAGGAAGT CCACGTTGTC GAAAGGCTCG
101  GGCGTTTCCA TCGCGCCCTG ACGGCCGGTT TGAATATTTT GATTCCCTTT
151  ATCGACCGCG TCGCCTACCG CCATTCGCTG AAAGAAATCC CTTTAGACGT
201  ACCCAGCCAG GTCTGCATCA CGCGCGATAA TACGCAATTG ACTGTTGACG
251  GCATCATCTA TTTCCAAGTA ACCGATCCCA AACTCGCCTC ATACGGTTCG
301  AGCAACTACA TTATGGCAAT TACCCAGCTT GCCCAAACGA CGCTGCGTTC
351  CGTTATCGGG CGTATGGAGT TGGACAAAAC GTTTGAAGAA CGCGACGAAA
401  TCAACAGTAC CGTCGTCTCC GCCCTCGATG AAGCCGCCGG GGCTTGGGGT
451  GTGAAAGTCC TCCGTTACGA AATCAAGGAT TTGGTTCCGC CGCAAGAAAT
501  CCTTCGCGCA ATGCAGGCAC AAATTACCGC CGAACGCGAA AAACGCGCCC
551  GTATTGCCGA ATCCGAAGGC CGTAAAATCG AACAAATCAA CCTTGCCAGT
601  GGTCAGCGTG AAGCCGAAAT CCAACAATCC GAAGGCGAGG CTCAGGCTGC
651  GGTCAATGCG TCCAATGCCG AGAAAATCGC CCGCATCAAC CGCGCCAAAG
701  GCGAAGCGGA ATCCCTGCGC CTTGTTGCCG AAGCCAATGC CGAAGCCATC
751  CGTCAAATTG CCGCCGCCCT TCAAACCCAA GGCGGGGCGG ATGCGGTCAA
801  TCTGAAGATT GCGGAACAAT ACGTAGCCGC GTTCAACAAT CTTGCCAAAG
851  AAAGCAATAC GCTGATTATG CCCGCCAATG TTGCCGACAT CGGCAGCCTG
901  ATTTCTGCCG GCATGAAAAT TATCGACAGC AGCAAAACCG CCAAATAA
```

This corresponds to the amino acid sequence <SEQ ID 17; ORF 519-1.ng>:

```
g519-1.pep
      1  MEFFIILLAA VAVFGFKSFV VIPQQEVHVV ERLGRFHRAL TAGLNILIPF
     51  IDRVAYRHSL KEIPLDVPSQ VCITRDNTQL TVDGIIYFQV TDPKLASYGS
    101  SNYIMAITQL AQTTLRSVIG RMELDKTFEE RDEINSTVVS ALDEAAGAWG
    151  VKVLRYEIKD LVPPQEILRA MQAQITAERE KRARIAESEG RKIEQINLAS
    201  GQREAEIQQS EGEAQAAVNA SNAEKIARIN RAKGEAESLR LVAEANAEAI
    251  RQIAAALQTQ GGADAVNLKI AEQYVAAFNN LAKESNTLIM PANVADIGSL
    301  ISAGMKIIDS SKTAK*
```

**m519-1/g519-1 ORFs 519-1 and 519-1.ng showed a** 99.0% identity in 315 aa overlap

```
                   10        20        30        40        50        60
g519-1.pep  MEFFIILLAAVAVFGFKSFVVIPQQEVHVVERLGRFHRALTAGLNILIPFIDRVAYRHSL
            ||||||||:|||||||||||||||||||||||||||||||||||||||||||||||||||
m519-1      MEFFIILLVAVAVFGFKSFVVIPQQEVHVVERLGRFHRALTAGLNILIPFIDRVAYRHSL
                   10        20        30        40        50        60

                   70        80        90       100       110       120
g519-1.pep  KEIPLDVPSQVCITRDNTQLTVDGIIYFQVTDPKLASYGSSNYIMAITQLAQTTLRSVIG
            ||||||||||||||||||||||||||||||||||||||||||||||||||||||||||||
m519-1      KEIPLDVPSQVCITRDNTQLTVDGIIYFQVTDPKLASYGSSNYIMAITQLAQTTLRSVIG
                   70        80        90       100       110       120

                  130       140       150       160       170       180
g519-1.pep  RMELDKTFEERDEINSTVVSALDEAAGAWGVKVLRYEIKDLVPPQEILRAMQAQITAERE
            |||||||||||||||||||:|||||||||||||||||||||||||||||:||||||||||
m519-1      RMELDKTFEERDEINSTVVAALDEAAGAWGVKVLRYEIKDLVPPQEILRSMQAQITAERE
                  130       140       150       160       170       180

                  190       200       210       220       230       240
g519-1.pep  KRARIAESEGRKIEQINLASGQREAEIQQSEGEAQAAVNASNAEKIARINRAKGEAESLR
            ||||||||||||||||||||||||||||||||||||||||||||||||||||||||||||
m519-1      KRARIAESEGRKIEQINLASGQREAEIQQSEGEAQAAVNASNAEKIARINRAKGEAESLR
                  190       200       210       220       230       240

                  250       260       270       280       290       300
g519-1.pep  LVAEANAEAIRQIAAALQTQGGADAVNLKIAEQYVAAFNNLAKESNTLIMPANVADIGSL
            ||||||||||||||||||||||||||||||||||||||||||||||||||||||||||||
m519-1      LVAEANAEAIRQIAAALQTQGGADAVNLKIAEQYVAAFNNLAKESNTLIMPANVADIGSL
                  250       260       270       280       290       300

                  310
g519-1.pep  ISAGMKIIDSSKTAKX
            ||||||||||||||||
m519-1      ISAGMKIIDSSKTAKX
                  310
```

The following DNA sequence was identified in *N. meningitidis* <SEQ ID 18>:

```
a519-1.seq
      1  ATGGAATTTT TCATTATCTT GCTGGCAGCC GTCGTTGTTT TCGGCTTCAA
     51  ATCCTTTGTT GTCATCCCAC AGCAGGAAGT CCACGTTGTC GAAAGGCTCG
    101  GGCGTTTCCA TCGCGCCCTG ACGGCCGGTT TGAATATTTT GATTCCCTTT
    151  ATCGACCGCG TCGCCTACCG CCATTCGCTG AAAGAAATCC CTTTAGACGT
    201  ACCCAGCCAG GTCTGCATCA CGCGCGACAA TACGCAGCTG ACTGTTGACG
    251  GTATCATCTA TTTCCAAGTA ACCGACCCCA AACTCGCCTC ATACGGTTCG
    301  AGCAACTACA TTATGGCGAT TACCCAGCTT GCCCAAACGA CGCTGCGTTC
    351  CGTTATCGGG CGTATGGAAT TGGACAAAAC GTTTGAAGAA CGCGACGAAA
    401  TCAACAGCAC CGTCGTCTCC GCCCTCGATG AAGCCGCCGG AGCTTGGGGT
    451  GTGAAGGTTT TGCGTTATGA GATTAAAGAC TTGGTTCCGC CGCAAGAAAT
    501  CCTTCGCTCA ATGCAGGCGC AAATTACTGC TGAACGCGAA AAACGCGCCC
    551  GTATCGCCGA ATCCGAAGGT CGTAAAATCG AACAAATCAA CCTTGCCAGT
    601  GGTCAGCGCG AAGCCGAAAT CCAACAATCC GAAGGCGAGG CTCAGGCTGC
    651  GGTCAATGCG TCAAATGCCG AGAAAATCGC CCGCATCAAC CGCGCCAAAG
    701  GTGAAGCGGA ATCCTTGCGC CTTGTTGCCG AAGCCAATGC CGAAGCCATC
    751  CGTCAAATTG CCGCCGCCCT TCAAACCCAA GGCGGTGCGG ATGCGGTCAA
    801  TCTGAAGATT GCGGAACAAT ACGTCGCCGC GTTCAACAAT CTTGCCAAAG
    851  AAAGCAATAC GCTGATTATG CCCGCCAATG TTGCCGACAT CGGCAGCCTG
    901  ATTTCTGCCG GTATGAAAAT TATCGACAGC AGCAAAACCG CCAAATAA
```

This corresponds to the amino acid sequence <SEQ ID 19; ORF 519-1.a>:

```
a519-1.pep.
      1  MEFFIILLAA VVVFGFKSFV VIPQQEVHVV ERLGRFHRAL TAGLNILIPF
     51  IDRVAYRHSL KEIPLDVPSQ VCITRDNTQL TVDGIIYFQV TDPKLASYGS
    101  SNYIMAITQL AQTTLRSVIG RMELDKTFEE RDEINSTVVS ALDEAAGAWG
    151  VKVLRYEIKD LVPPQEILRS MQAQITAERE KRARIAESEG RKIEQINLAS
    201  GQREAEIQQS EGEAQAAVNA SNAEKIARIN RAKGEAESLR LVAEANAEAI
    251  RQIAAALQTQ GGADAVNLKI AEQYVAAFNN LAKESNTLIM PANVADIGSL
    301  ISAGMKIIDS SKTAK*
```

**m519-1/a519-1** ORFs 519-1 and 519-1.a showed a 99.0% identity in 315 aa overlap

```
                           10        20        30        40        50        60
a519-1.pep        MEFFIILLAAVVVFGFKSFVVIPQQEVHVVERLGRFHRALTAGLNILIPFIDRVAYRHSL
                  ||||||||:||:||||||||||||||||||||||||||||||||||||||||||||||||
m519-1            MEFFIILLVAVAVFGFKSFVVIPQQEVHVVERLGRFHRALTAGLNILIPFIDRVAYRHSL
                           10        20        30        40        50        60

                           70        80        90       100       110       120
a519-1.pep        KEIPLDVPSQVCITRDNTQLTVDGIIYFQVTDPKLASYGSSNYIMAITQLAQTTLRSVIG
                  ||||||||||||||||||||||||||||||||||||||||||||||||||||||||||||
m519-1            KEIPLDVPSQVCITRDNTQLTVDGIIYFQVTDPKLASYGSSNYIMAITQLAQTTLRSVIG
                           70        80        90       100       110       120

                          130       140       150       160       170       180
a519-1.pep        RMELDKTFEERDEINSTVVSALDEAAGAWGVKVLRYEIKDLVPPQEILRSMQAQITAERE
                  |||||||||||||||||||:||||||||||||||||||||||||||||||||||||||||
m519-1            RMELDKTFEERDEINSTVVAALDEAAGAWGVKVLRYEIKDLVPPQEILRSMQAQITAERE
                          130       140       150       160       170       180

                          190       200       210       220       230       240
a519-1.pep        KRARIAESEGRKIEQINLASGQREAEIQQSEGEAQAAVNASNAEKIARINRAKGEAESLR
                  ||||||||||||||||||||||||||||||||||||||||||||||||||||||||||||
m519-1            KRARIAESEGRKIEQINLASGQREAEIQQSEGEAQAAVNASNAEKIARINRAKGEAESLR
                          190       200       210       220       230       240

                          250       260       270       280       290       300
a519-1.pep        LVAEANAEAIRQIAAALQTQGGADAVNLKIAEQYVAAFNNLAKESNTLIMPANVADIGSL
                  ||||||||||||||||||||||||||||||||||||||||||||||||||||||||||||
m519-1            LVAEANAEAIRQIAAALQTQGGADAVNLKIAEQYVAAFNNLAKESNTLIMPANVADIGSL
                          250       260       270       280       290       300

                          310
a519-1.pep        ISAGMKIIDSSKTAKX
                  ||||||||||||||||
m519-1            ISAGMKIIDSSKTAKX
                          310
```

## 576 and 576-1 gnm22.seq

The following partial DNA sequence was identified in *N. meningitidis* <SEQ ID 20>:

```
m576.seq.. (partial)
      1  ..ATGCAGCAGG CAAGCTATGC GATGGGCGTG GACATCGGAC GCTCCCTGAA
     51    GCAAATGAAG GAACAGGGCG CGGAAATCGA TTTGAAAGTC TTTACCGAAG
    101    CCATGCAGGC AGTGTATGAC GGCAAAGAAA TCAAAATGAC CGAAGAGCAG
    151    GCTCAGGAAG TCATGATGAA ATTCCTTCAG GAACAACAGG CTAAAGCCGT
    201    AGAAAAACAC AAGGCGGACG CGAAGGCCAA TAAAGAAAAA GGCGAAGCCT
```

```
251     TTCTGAAAGA AAATGCCGCC AAAGACGGCG TGAAGACCAC TGCTTCCGGC
301     CTGCAATACA AAATCACCAA ACAGGGCGAA GGCAAACAGC CGACCAAAGA
351     CGACATCGTT ACCGTGGAAT ACGAAGGCCG CCTGATTGAC GGTACGGTAT
401     TCGACAGCAG CAAAGCCAAC GGCGGCCCGG TCACCTTCCC TTTGAGCCAA
451     GTGATTCCGG GTTGGACCGA AGgCGTACAG CTTCTGAAAG AAGGCGGCGA
501     AGCCACGTTC TACATCCCGT CCAACCTTGC CTACCGCGAA CAGGGTGCGG
551     GCGACAAAAT CGGTCCGAAC GCCACTTTGG TATTTGATGT GAAACTGGTC
601     AAAATCGGCG CACCCGAAAA CGCGCCCGCC AAGCAGCCGG CTCAAGTCGA
651     CATCAAAAAA GTAAATTAA
```

This corresponds to the amino acid sequence <SEQ ID 21; ORF 576>:

```
m576.pep.. (partial)
  1   ..MQQASYAMGV DIGRSLKQMK EQGAEIDLKV FTEAMQAVYD GKEIKMTEEQ
 51     AQEVMMKFLQ EQQAKAVEKH KADAKANKEK GEAFLKENAA KDGVKTTASG
101     LQYKITKQGE GKQPTKDDIV TVEYEGRLID GTVFDSSKAN GGPVTFPLSQ
151     VIPGWTEGVQ LLKEGGEATF YIPSNLAYRE QGAGDKIGPN ATLVFDVKLV
201     KIGAPENAPA KQPAQVDIKK VN*
```

The following partial DNA sequence was identified in *N. gonorrhoeae* <SEQ ID 22>:

```
g576.seq..(partial)
  1   ..atgggcgtgg acatcggacg ctccctgaaa caaatgaagg aacagggcgc
 51     ggaaatcgat ttgaaagtct ttaccgatgc catgcaggca gtgtatgacg
101     gcaaagaaat caaaatgacc gaagagcagg cccaggaagt gatgatgaaa
151     ttcctgcagg agcagcaggc taaagccgta gaaaaacaca aggcggatgc
201     gaaggccaac aaagaaaaag gcgaagcctt cctgaaggaa aatgccgccg
251     aagacggcgt gaagaccact gcttccggtc tgcagtacaa aatcaccaaa
301     cagggtgaag gcaaacagcc gacaaaagac gacatcgtta ccgtggaata
351     cgaaggccgc ctgattgacg gtaccgtatt cgacagcagc aaagccaacg
401     gcggcccggc caccttccct ttgagccaag tgattccggg ttggaccgaa
451     ggcgtacggc ttctgaaaga aggcggcgaa gccacgttct acatcccgtc
501     caaccttgcc taccgcgaac agggtgcggg cgaaaaaatc ggtccgaacg
551     ccactttggt atttgacgtg aaactggtca aaatcggcgc acccgaaaac
601     gcgcccgcca agcagccgga tcaagtcgac atcaaaaaag taaattaa
```

This corresponds to the amino acid sequence <SEQ ID 23; ORF 576.ng>:

```
g576.pep..(partial)
  1   ..MGVDIGRSLK QMKEQGAEID LKVFTDAMQA VYDGKEIKMT EEQAQEVMMK
 51     FLQEQQAKAV EKHKADAKAN KEKGEAFLKE NAAEDGVKTT ASGLQYKITK
101     QGEGKQPTKD DIVTVEYEGR LIDGTVFDSS KANGGPATFP LSQVIPGWTE
151     GVRLLKEGGE ATFYIPSNLA YREQGAGEKI GPNATLVFDV KLVKIGAPEN
201     APAKQPDQVD IKKVN*
```

Computer analysis of this amino acid sequence gave the following results:
Homology with a predicted ORF from *N. gonorrhoeae*

**m576/g576** 97.2% identity in 215 aa overlap

```
                    10        20        30        40        50        60
m576.pep    MQQASYAMGVDIGRSLKQMKEQGAEIDLKVFTEAMQAVYDGKEIKMTEEQAQEVMMKFLQ
                   |||||||||||||||||||||||||||:||||||||||||||||||||||||||
g576               MGVDIGRSLKQMKEQGAEIDLKVFTDAMQAVYDGKEIKMTEEQAQEVMMKFLQ
                           10        20        30        40        50

                    70        80        90       100       110       120
m576.pep    EQQAKAVEKHKADAKANKEKGEAFLKENAAKDGVKTTASGLQYKITKQGEGKQPTKDDIV
            ||||||||||||||||||||||||||||||:|||||||||||||||||||||||||||||
g576        EQQAKAVEKHKADAKANKEKGEAFLKENAAEDGVKTTASGLQYKITKQGEGKQPTKDDIV
                  60        70        80        90       100       110

                   130       140       150       160       170       180
m576.pep    TVEYEGRLIDGTVFDSSKANGGPVTFPLSQVIPGWTEGVQLLKEGGEATFYIPSNLAYRE
            ||||||||||||||||||||||:|||||||||||||||||:|||||||||||||||||||
g576        TVEYEGRLIDGTVFDSSKANGGPATFPLSQVIPGWTEGVRLLKEGGEATFYIPSNLAYRE
                  120       130       140       150       160       170

                   190       200       210       220
m576.pep    QGAGDKIGPNATLVFDVKLVKIGAPENAPAKQPAQVDIKKVNX
            ||||:|||||||||||||||||||||||||||| |||||||||
g576        QGAGEKIGPNATLVFDVKLVKIGAPENAPAKQPDQVDIKKVNX
                  180       190       200       210
```

The following partial DNA sequence was identified in *N. meningitidis* <SEQ ID 24>:

```
a576.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCTGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGCCCGAAC GCCACTTTGG TATTTGATGT GAAACTGGTC
    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 25; ORF 576.a>:

```
a576.pep
      1  MNTIFKISAL TLSAALALSA CGKKEAAPAS ASEPAAASSA QGDTSSIGST
     51  MQQASYAMGV DIGRSLKQMK EQGAEIDLKV FTEAMQAVYD GKEIKMTEEQ
    101  AQEVMMKFLQ EQQAKAVEKH KADAKANKEK GEAFLKENAA KDGVKTTASG
    151  LQYKITKQGE GKQPTKDDIV TVEYEGRLID GTVFDSSKAN GGPVTFPLSQ
    201  VILGWTEGVQ LLKEGGEATF YIPSNLAYRE QGAGDKIGPN ATLVFDVKLV
    251  KIGAPENAPA KQPAQVDIKK VN*
```

```
m576/a576    ORFs 576 and 576.a showed a 99.5% identity in 222 aa overlap

                                                     10        20        30
m576.pep                                     MQQASYAMGVDIGRSLKQMKEQGAEIDLKV
                                             ||||||||||||||||||||||||||||||
a576         CGKKEAAPASASEPAAASSAQGDTSSIGSTMQQASYAMGVDIGRSLKQMKEQGAEIDLKV
                     30        40        50        60        70        80

                     40        50        60        70        80        90
m576.pep     FTEAMQAVYDGKEIKMTEEQAQEVMMKFLQEQQAKAVEKHKADAKANKEKGEAFLKENAA
             ||||||||||||||||||||||||||||||||||||||||||||||||||||||||||||
a576         FTEAMQAVYDGKEIKMTEEQAQEVMMKFLQEQQAKAVEKHKADAKANKEKGEAFLKENAA
                     90       100       110       120       130       140

                    100       110       120       130       140       150
m576.pep     KDGVKTTASGLQYKITKQGEGKQPTKDDIVTVEYEGRLIDGTVFDSSKANGGPVTFPLSQ
             ||||||||||||||||||||||||||||||||||||||||||||||||||||||||||||
a576         KDGVKTTASGLQYKITKQGEGKQPTKDDIVTVEYEGRLIDGTVFDSSKANGGPVTFPLSQ
                    150       160       170       180       190       200

                    160       170       180       190       200       210
m576.pep     VIPGWTEGVQLLKEGGEATFYIPSNLAYREQGAGDKIGPNATLVFDVKLVKIGAPENAPA
             || |||||||||||||||||||||||||||||||||||||||||||||||||||||||||
a576         VILGWTEGVQLLKEGGEATFYIPSNLAYREQGAGDKIGPNATLVFDVKLVKIGAPENAPA
                    210       220       230       240       250       260

                    220
m576.pep     KQPAQVDIKKVNX
             ||||||||||||
a576         KQPAQVDIKKVNX
                    270
```

Further work revealed the following DNA sequence identified in *N. meningitidis* <SEQ ID 26>:

```
m576-1.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCCGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGTCCGAAC GCCACTTTGG TATTTGATGT GAAACTGGTC
    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 27; ORF 576-1>:

```
m576-1.pep
       1  MNTIFKISAL TLSAALALSA CGKKEAAPAS ASEPAAASSA QGDTSSIGST
      51  MQQASYAMGV DIGRSLKQMK EQGAEIDLKV FTEAMQAVYD GKEIKMTEEQ
     101  AQEVMMKFLQ EQQAKAVEKH KADAKANKEK GEAFLKENAA KDGVKTTASG
     151  LQYKITKQGE GKQPTKDDIV TVEYEGRLID GTVFDSSKAN GGPVTFPLSQ
     201  VIPGWTEGVQ LLKEGGEATF YIPSNLAYRE QGAGDKIGPN ATLVFDVKLV
     251  KIGAPENAPA KQPAQVDIKK VN*
```

The following DNA sequence was identified in *N. gonorrhoeae* <SEQ ID 28>:

```
g576-1.seq
       1  ATGAACACCA TTTTCAAAAT CAGCGCACTG ACCCTTTCCG CCGCTTTGGC
      51  ACTTTCCGCC TGCGGCAAAA AAGAAGCCGC CCCCGCATCT GCATCCGAAC
     101  CTGCCGCCGC TTCTGCCGCG CAGGGCGACA CCTCTTCAAT CGGCAGCACG
     151  ATGCAGCAGG CAAGCTATGC AATGGGCGTG GACATCGGAC GCTCCCTGAA
     201  ACAAATGAAG GAACAGGGCG CGGAAATCGA TTTGAAAGTC TTTACCGATG
     251  CCATGCAGGC AGTGTATGAC GGCAAAGAAA TCAAAATGAC CGAAGAGCAG
     301  GCCCAGGAAG TGATGATGAA ATTCCTGCAG GAGCAGCAGG CTAAAGCCGT
     351  AGAAAAACAC AAGGCGGATG CGAAGGCCAA CAAAGAAAAA GGCGAAGCCT
     401  TCCTGAAGGA AAATGCCGCC AAAGACGGCG TGAAGACCAC TGCTTCCGGT
     451  CTGCAGTACA AAATCACCAA ACAGGGTGAA GGCAAACAGC CGACAAAAGA
     501  CGACATCGTT ACCGTGGAAT ACGAAGGCCG CCTGATTGAC GGTACCGTAT
     551  TCGACAGCAG CAAAGCCAAC GGCGGCCCGG CCACCTTCCC TTTGAGCCAA
     601  GTGATTCCGG GTTGGACCGA AGGCGTACGG CTTCTGAAAG AAGGCGGCGA
     651  AGCCACGTTC TACATCCCGT CCAACCTTGC CTACCGCGAA CAGGGTGCGG
     701  GCGAAAAAAT CGGTCCGAAC GCCACTTTGG TATTTGACGT GAAACTGGTC
     751  AAAATCGGCG CACCCGAAAA CGCGCCCGCC AAGCAGCCGG ATCAAGTCGA
     801  CATCAAAAAA GTAAATTAA
```

This corresponds to the amino acid sequence <SEQ ID 29; ORF 576-1.ng>:

```
g576-1.pep
      1  MNTIFKISAL TLSAALALSA CGKKEAAPAS ASEPAAASAA QGDTSSIGST
     51  MQQASYAMGV DIGRSLKQMK EQGAEIDLKV FTDAMQAVYD GKEIKMTEEQ
    101  AQEVMMKFLQ EQQAKAVEKH KADAKANKEK GEAFLKENAA KDGVKTTASG
    151  LQYKITKQGE GKQPTKDDIV TVEYEGRLID GTVFDSSKAN GGPATFPLSQ
    201  VIPGWTEGVR LLKEGGEATF YIPSNLAYRE QGAGEKIGPN ATLVFDVKLV
    251  KIGAPENAPA KQPDQVDIKK VN*
```

**g576-1/m576-1** ORFs 576-1 and 576-1.ng showed a 97.8% identity in 272 aa overlap

```
                    10        20        30        40        50        60
g576-1.pep  MNTIFKISALTLSAALALSACGKKEAAPASASEPAAASAAQGDTSSIGSTMQQASYAMGV
            ||||||||||||||||||||||||||||||||||||||:|||||||||||||||||||||
m576-1      MNTIFKISALTLSAALALSACGKKEAAPASASEPAAASSAQGDTSSIGSTMQQASYAMGV
                    10        20        30        40        50        60

                    70        80        90       100       110       120
g576-1.pep  DIGRSLKQMKEQGAEIDLKVFTDAMQAVYDGKEIKMTEEQAQEVMMKFLQEQQAKAVEKH
            |||||||||||||||||||||:||||||||||||||||||||||||||||||||||||||
m576-1      DIGRSLKQMKEQGAEIDLKVFTEAMQAVYDGKEIKMTEEQAQEVMMKFLQEQQAKAVEKH
                    70        80        90       100       110       120

                   130       140       150       160       170       180
g576-1.pep  KADAKANKEKGEAFLKENAAKDGVKTTASGLQYKITKQGEGKQPTKDDIVTVEYEGRLID
            ||||||||||||||||||||||||||||||||||||||||||||||||||||||||||||
m576-1      KADAKANKEKGEAFLKENAAKDGVKTTASGLQYKITKQGEGKQPTKDDIVTVEYEGRLID
                   130       140       150       160       170       180

                   190       200       210       220       230       240
g576-1.pep  GTVFDSSKANGGPATFPLSQVIPGWTEGVRLLKEGGEATFYIPSNLAYREQGAGEKIGPN
            |||||||||||||:|||||||||||||||:|||||||||||||||||||||||||:||||
m576-1      GTVFDSSKANGGPVTFPLSQVIPGWTEGVQLLKEGGEATFYIPSNLAYREQGAGDKIGPN
                   190       200       210       220       230       240

                   250       260       270
g576-1.pep  ATLVFDVKLVKIGAPENAPAKQPDQVDIKKVNX
            |||||||||||||||||||||||| |||||||||
m576-1      ATLVFDVKLVKIGAPENAPAKQPAQVDIKKVNX
                   250       260       270
```

The following DNA sequence was identified in *N. meningitidis* <SEQ ID 30>:

```
a576-1.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCTGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGCCCGAAC GCCACTTTGG TATTTGATGT GAAACTGGTC

    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 31; ORF 576-1.a>:

```
a576-1.pep
      1  MNTIFKISAL TLSAALALSA CGKKEAAPAS ASEPAAASSA QGDTSSIGST
     51  MQQASYAMGV DIGRSLKQMK EQGAEIDLKV FTEAMQAVYD GKEIKMTEEQ
    101  AQEVMMKFLQ EQQAKAVEKH KADAKANKEK GEAFLKENAA KDGVKTTASG
    151  LQYKITKQGE GKQPTKDDIV TVEYEGRLID GTVFDSSKAN GGPVTFPLSQ
    201  VILGWTEGVQ LLKEGGEATF YIPSNLAYRE QGAGDKIGPN ATLVFDVKLV
    251  KIGAPENAPA KQPAQVDIKK VN*
```

**a576-1/m576-1** ORFs 576-1 and 576-1.a 99.6% identity in 272 aa overlap

```
                        10        20        30        40        50        60
a576-1.pep      MNTIFKISALTLSAALALSACGKKEAAPASASEPAAASSAQGDTSSIGSTMQQASYAMGV
                ||||||||||||||||||||||||||||||||||||||||||||||||||||||||||||
m576-1          MNTIFKISALTLSAALALSACGKKEAAPASASEPAAASSAQGDTSSIGSTMQQASYAMGV
                        10        20        30        40        50        60

                        70        80        90       100       110       120
a576-1.pep      DIGRSLKQMKEQGAEIDLKVFTEAMQAVYDGKEIKMTEEQAQEVMMKFLQEQQAKAVEKH
                ||||||||||||||||||||||||||||||||||||||||||||||||||||||||||||
m576-1          DIGRSLKQMKEQGAEIDLKVFTEAMQAVYDGKEIKMTEEQAQEVMMKFLQEQQAKAVEKH
                        70        80        90       100       110       120

                       130       140       150       160       170       180
a576-1.pep      KADAKANKEKGEAFLKENAAKDGVKTTASGLQYKITKQGEGKQPTKDDIVTVEYEGRLID
                ||||||||||||||||||||||||||||||||||||||||||||||||||||||||||||
m576-1          KADAKANKEKGEAFLKENAAKDGVKTTASGLQYKITKQGEGKQPTKDDIVTVEYEGRLID
                       130       140       150       160       170       180

                       190       200       210       220       230       240
a576-1.pep      GTVFDSSKANGGPVTFPLSQVILGWTEGVQLLKEGGEATFYIPSNLAYREQGAGDKIGPN
                |||||||||||||||||||||| |||||||||||||||||||||||||||||||||||||
m576-1          GTVFDSSKANGGPVTFPLSQVIPGWTEGVQLLKEGGEATFYIPSNLAYREQGAGDKIGPN
                       190       200       210       220       230       240

                       250       260       270
a576-1.pep      ATLVFDVKLVKIGAPENAPAKQPAQVDIKKVNX
                |||||||||||||||||||||||||||||||||
m576-1          ATLVFDVKLVKIGAPENAPAKQPAQVDIKKVNX
                       250       260       270
```

## 919 and 919-2 gnm43.seq

The following partial DNA sequence was identified in *N.meningitidis* <SEQ ID 32>:

```
m919.seq
      1  ATGAAAAAAT ACCTATTCCG CGCCGCCCTG TACGGCATCG CCGCCGCCAT
     51  CCTCGCCGCC TGCCAAAGCA AGAGCATCCA AACCTTTCCG CAACCCGACA
    101  CATCCGTCAT CAACGGCCCG GACCGGCCGG TCGGCATCCC CGACCCCGCC
    151  GGAACGACGG TCGGCGGCGG CGGGGCCGTC TATACCGTTG TACCGCACCT
    201  GTCCCTGCCC CACTGGGCGG CGCAGGATTT CGCCAAAAGC CTGCAATCCT
    251  TCCGCCTCGG CTGCGCCAAT TTGAAAAACC GCCAAGGCTG GCAGGATGTG
    301  TGCGCCCAAG CCTTTCAAAC CCCCGTCCAT TCCTTTCAGG CAAAACAGTT
    351  TTTTGAACGC TATTTCACGC CGTGGCAGGT TGCAGGCAAC GGAAGCCTTG
```

```
 401  CCGGTACGGT TACCGGCTAT TACGAACCGG TGCTGAAGGG CGACGACAGG
 451  CGGACGGCAC AAGCCCGCTT CCCGATTTAC GGTATTCCCG ACGATTTTAT
 501  CTCCGTCCCC CTGCCTGCCG GTTTGCGGAG CGGAAAAGCC CTTGTCCGCA
 551  TCAGGCAGAC GGGAAAAAAC AGCGGCACAA TCGACAATAC CGGCGGCACA
 601  CATACCGCCG ACCTCTCCcG ATTCCCCATC ACCGCGCGCA CAACAGCAAT
 651  CAAAGGCAGG TTTGAAGGAA GCCGCTTCCT CCCCTACCAC ACGCGCAACC
 701  AAATCAACGG CGGCGCGCTT GACGGCAAAG CCCCGATACT CGGTTACGCC
 751  GAAGACCCTG TCGAACTTTT TTTTATGCAC ATCCAAGGCT CGGGCCGTCT
 801  GAAAACCCCG TCCGGCAAAT ACATCCGCAT CGGCTATGCC GACAAAAACG
 851  AACATCCyTA CGTTTCCATC GGACGCTATA TGGCGGATAA GGGCTACCTC
 901  AAACTCGGAC AAACCTCCAT GCAGGGCATT AAGTCTTATA TGCGGCAAAA
 951  TCCGCAACGC CTCGCCGAAG TTTTGGGTCA AAACCCCAGC TATATCTTTT
1001  TCCGCGAGCT TGCCGGAAGC AGCAATGACG GCCCTGTCGG CGCACTGGGC
1051  ACGCCGCTGA TGGGGGAATA TGCCGGCGCA GTCGACCGGC ACTACATTAC
1101  CTTGGGTGCG CCCTTATTTG TCGCCACCGC CCATCCGGTT ACCCGCAAAG
1151  CCCTCAACCG CCTGATTATG GCGCAGGATA CCGGCAGCGC GATTAAAGGC
1201  GCGGTGCGCG TGGATTATTT TTGGGGATAC GGCGACGAAG CCGGCGAACT
1251  TGCCGGCAAA CAGAAAACCA CGGGATATGT CTGGCAGCTC CTACCCAACG
1301  GTATGAAGCC CGAATACCGc CCGTAA
```

This corresponds to the amino acid sequence <SEQ ID 33; ORF 919>:

**m919.pep**

```
  1  MKKYLFRAAL YGIAAAILAA CQSKSIQTFP QPDTSVINGP DRPVGIPDPA
 51  GTTVGGGGAV YTVVPHLSLP HWAAQDFAKS LQSFRLGCAN LKNRQGWQDV
101  CAQAFQTPVH SFQAKQFFER YFTPWQVAGN GSLAGTVTGY YEPVLKGDDR
151  RTAQARFPIY GIPDDFISVP LPAGLRSGKA LVRIRQTGKN SGTIDNTGGT
201  HTADLSRFPI TARTTAIKGR FEGSRFLPYH TRNQINGGAL DGKAPILGYA
251  EDPVELFFMH IQGSGRLKTP SGKYIRIGYA DKNEHPYVSI GRYMADKGYL
301  KLGQTSMQGI KSYMRQNPQR LAEVLGQNPS YIFFRELAGS SNDGPVGALG
351  TPLMGEYAGA VDRHYITLGA PLFVATAHPV TRKALNRLIM AQDTGSAIKG
401  AVRVDYFWGY GDEAGELAGK QKTTGYVWQL LPNGMKPEYR P*
```

The following partial DNA sequence was identified in *N.meningitidis* <SEQ ID 34>:

**m919-2.seq**

```
   1  ATGAAAAAAT ACCTATTCCG CGCCGCCCTG TACGGCATCG CCGCCGCCAT
  51  CCTCGCCGCC TGCCAAAGCA AGAGCATCCA AACCTTTCCG CAACCCGACA
 101  CATCCGTCAT CAACGGCCCG GACCGGCCGG TCGGCATCCC CGACCCCGCC
 151  GGAACGACGG TCGGCGGCGG CGGGGCCGTC TATACCGTTG TACCGCACCT
 201  GTCCCTGCCC CACTGGGCGG CGCAGGATTT CGCCAAAAGC CTGCAATCCT
 251  TCCGCCTCGG CTGCGCCAAT TTGAAAAACC GCCAAGGCTG GCAGGATGTG
 301  TGCGCCCAAG CCTTTCAAAC CCCCGTCCAT TCCTTTCAGG CAAAACAGTT
 351  TTTTGAACGC TATTTCACGC CGTGGCAGGT TGCAGGCAAC GGAAGCCTTG
 401  CCGGTACGGT TACCGGCTAT TACGAACCGG TGCTGAAGGG CGACGACAGG
 451  CGGACGGCAC AAGCCCGCTT CCCGATTTAC GGTATTCCCG ACGATTTTAT
 501  CTCCGTCCCC CTGCCTGCCG GTTTGCGGAG CGGAAAAGCC CTTGTCCGCA
 551  TCAGGCAGAC GGGAAAAAAC AGCGGCACAA TCGACAATAC CGGCGGCACA
 601  CATACCGCCG ACCTCTCCCG ATTCCCCATC ACCGCGCGCA CAACAGCAAT
 651  CAAAGGCAGG TTTGAAGGAA GCCGCTTCCT CCCCTACCAC ACGCGCAACC
 701  AAATCAACGG CGGCGCGCTT GACGGCAAAG CCCCGATACT CGGTTACGCC
 751  GAAGACCCTG TCGAACTTTT TTTTATGCAC ATCCAAGGCT CGGGCCGTCT
 801  GAAAACCCCG TCCGGCAAAT ACATCCGCAT CGGCTATGCC GACAAAAACG
 851  AACATCCCTA CGTTTCCATC GGACGCTATA TGGCGGATAA GGGCTACCTC
 901  AAACTCGGAC AAACCTCCAT GCAGGGCATT AAGTCTTATA TGCGGCAAAA
 951  TCCGCAACGC CTCGCCGAAG TTTTGGGTCA AAACCCCAGC TATATCTTTT
1001  TCCGCGAGCT TGCCGGAAGC AGCAATGACG GCCCTGTCGG CGCACTGGGC
1051  ACGCCGCTGA TGGGGGAATA TGCCGGCGCA GTCGACCGGC ACTACATTAC
1101  CTTGGGTGCG CCCTTATTTG TCGCCACCGC CCATCCGGTT ACCCGCAAAG
1151  CCCTCAACCG CCTGATTATG GCGCAGGATA CCGGCAGCGC GATTAAAGGC
```

```
1201  GCGGTGCGCG TGGATTATTT TTGGGGATAC GGCGACGAAG CCGGCGAACT
1251  TGCCGGCAAA CAGAAAACCA CGGGATATGT CTGGCAGCTC CTACCCAACG
1301  GTATGAAGCC CGAATACCGC CCGTAA
```

This corresponds to the amino acid sequence <SEQ ID 35; ORF 919-2>:

```
m919-2.pep
  1  MKKYLFRAAL YGIAAAILAA CQSKSIQTFP QPDTSVINGP DRPVGIPDPA
 51  GTTVGGGGAV YTVVPHLSLP HWAAQDFAKS LQSFRLGCAN LKNRQGWQDV
101  CAQAFQTPVH SFQAKQFFER YFTPWQVAGN GSLAGTVTGY YEPVLKGDDR
151  RTAQARFPIY GIPDDFISVP LPAGLRSGKA LVRIRQTGKN SGTIDNTGGT
201  HTADLSRFPI TARTTAIKGR FEGSRFLPYH TRNQINGGAL DGKAPILGYA
251  EDPVELFFMH IQGSGRLKTP SGKYIRIGYA DKNEHPYVSI GRYMADKGYL
301  KLGQTSMQGI KSYMRQNPQR LAEVLGQNPS YIFFRELAGS SNDGPVGALG
351  TPLMGEYAGA VDRHYITLGA PLFVATAHPV TRKALNRLIM AQDTGSAIKG
401  AVRVDYFWGY GDEAGELAGK QKTTGYVWQL LPNGMKPEYR P*
```

The following partial DNA sequence was identified in *N.gonorrhoeae* <SEQ ID 36>:

```
g919.seq
   1  ATGAAAAAAC ACCTGCTCCG CTCCGCCCTG TACGGcatCG CCGCCgccAT
  51  CctcgCCGCC TGCCAAAgca gGAGCATCCA AACCTTTCCG CAACCCGACA
 101  CATCCGTCAT CAACGGCCCG GACCGGCCGG CCGGCATCCC CGACCCCGCC
 151  GGAACGACGG TTGCCGGCGG CGGGGCCGTC TATACCGTTG TGCCGCACCT
 201  GTCCATGCCC CACTGGGCGG CGCaggATTT TGCCAAAAGC CTGCAATCCT
 251  TCCGCCTCGG CTGCGCCAAT TTGAAAAACC GCCAAGGCTG GCAGGATGTG
 301  TGCGCCCAAG CCTTTCAAAC CCCCGTGCAT TCCTTTCAGG CAAAGcGgTT
 351  TTTTGAACGC TATTTCACGC cgtGGCaggt tgcaggcaAC GGAAGcCTTG
 401  Caggtacggt TACCGGCTAT TACGAACCGG TGCTGAAGGG CGACGGCAGG
 451  CGGACGGAAC GGGCCCGCTT CCCGATTTAC GGTATTCCCG ACGATTTTAT
 501  CTCCGTCCCG CTGCCTGCCG GTTTGCGGGG CGGAAAAAAC CTTGTCCGCA
 551  TCAGGCAGac ggGGAAAAAC AGCGGCACGA TCGACAATGC CGGCGGCACG
 601  CATACCGCCG ACCTCTCCCG ATTCCCCATC ACCGCGCGCA CAACGGcaat
 651  caaaGGCAGG TTTGAaggAA GCCGCTTCCT CCCTTACCAC ACGCGCAACC
 701  AAAtcaacGG CGGCgcgcTT GACGGCAAag cccCCATCCT CggttacgcC
 751  GAagaccCcG tcgaacttTT TTTCATGCAC AtccaaggCT CGGGCCGCCT
 801  GAAAACCCcg tccggcaaat acatCCGCAt cggaTacgcc gacAAAAACG
 851  AACAtccgTa tgtttccatc ggACGctaTA TGGCGGACAA AGGCTACCTC
 901  AAGctcgggc agACCTCGAT GCAGGgcatc aaagcCTATA TGCGGCAAAA
 951  TCCGCAACGC CTCGCCGAAG TTTTGGGTCA AAACCCCAGC TATATCTTTT
1001  TCCGCGAGCT TGCCGGAAGC GGCAATGAGG GCCCCGTCGG CGCACTGGGC
1051  ACGCCACTGA TGGGGGAATA CGCCGGCGCA ATCGACCGGC ACTACATTAC
1101  CTTGGGCGCG CCCTTATTTG TCGCCACCGC CCATCCGGTT ACCCGCAAAG
1151  CCCTCAACCG CCTGATTATG GCGCAGGATA CAGGCAGCGC GATCAAAGGC
1201  GCGGTGCGCG TGGATTATTT TTGGGGTTAC GGCGACGAAG CCGGCGAACT
1251  TGCCGGCAAA CAGAAAACCA CGGGATACGT CTGGCAGCTC CTGCCCAACG
1301  GCATGAAGCC CGAATACCGC CCGTGA
```

This corresponds to the amino acid sequence <SEQ ID 37; ORF 919.ng>:

```
g919.pep
      1  MKKHLLRSAL YGIAAAILAA CQSRSIQTFP QPDTSVINGP DRPAGIPDPA
     51  GTTVAGGGAV YTVVPHLSMP HWAAQDFAKS LQSFRLGCAN LKNRQGWQDV
    101  CAQAFQTPVH SFQAKRFFER YFTPWQVAGN GSLAGTVTGY YEPVLKGDGR
    151  RTERARFPIY GIPDDFISVP LPAGLRGGKN LVRIRQTGKN SGTIDNAGGT
    201  HTADLSRFPI TARTTAIKGR FEGSRFLPYH TRNQINGGAL DGKAPILGYA
    251  EDPVELFFMH IQGSGRLKTP SGKYIRIGYA DKNEHPYVSI GRYMADKGYL
    301  KLGQTSMQGI KAYMRQNPQR LAEVLGQNPS YIFFRELAGS GNEGPVGALG
    351  TPLMGEYAGA IDRHYITLGA PLFVATAHPV TRKALNRLIM AQDTGSAIKG
    401  AVRVDYFWGY GDEAGELAGK QKTTGYVWQL LPNGMKPEYR P*
```

ORF 919 shows 95.9% identity over a 441 aa overlap with a predicted ORF (ORF 919.ng) from *N. gonorrhoeae*:

m919/g919

```
                    10        20        30        40        50        60
m919.pep    MKKYLFRAALYGIAAAILAACQSKSIQTFPQPDTSVINGPDRPVGIPDPAGTTVGGGGAV
            |||:|:|:|||||||||||||||:||||||||||||||||||||:||||||||||:||||
g919        MKKHLLRSALYGIAAAILAACQSRSIQTFPQPDTSVINGPDRPAGIPDPAGTTVAGGGAV
                    10        20        30        40        50        60

                    70        80        90       100       110       120
m919.pep    YTVVPHLSLPHWAAQDFAKSLQSFRLGCANLKNRQGWQDVCAQAFQTPVHSFQAKQFFER
            ||||||||:||||||||||||||||||||||||||||||||||||||||||||||:||||
g919        YTVVPHLSMPHWAAQDFAKSLQSFRLGCANLKNRQGWQDVCAQAFQTPVHSFQAKRFFER
                    70        80        90       100       110       120

                   130       140       150       160       170       180
m919.pep    YFTPWQVAGNGSLAGTVTGYYEPVLKGDDRRTAQARFPIYGIPDDFISVPLPAGLRSGKA
            |||||||||||||||||||||||||||| ||| :||||||||||||||||||||||:||
g919        YFTPWQVAGNGSLAGTVTGYYEPVLKGDGRRTERARFPIYGIPDDFISVPLPAGLRGGKN
                   130       140       150       160       170       180

                   190       200       210       220       230       240
m919.pep    LVRIRQTGKNSGTIDNTGGTHTADLSRFPITARTTAIKGRFEGSRFLPYHTRNQINGGAL
            ||||||||||||||||:|||||||||||||||||||||||||||||||||||||||||||
g919        LVRIRQTGKNSGTIDNAGGTHTADLSRFPITARTTAIKGRFEGSRFLPYHTRNQINGGAL
                   190       200       210       220       230       240

                   250       260       270       280       290       300
m919.pep    DGKAPILGYAEDPVELFFMHIQGSGRLKTPSGKYIRIGYADKNEHPYVSIGRYMADKGYL
            ||||||||||||||||||||||||||||||||||||||||||||||||||||||||||||
g919        DGKAPILGYAEDPVELFFMHIQGSGRLKTPSGKYIRIGYADKNEHPYVSIGRYMADKGYL
                   250       260       270       280       290       300

                   310       320       330       340       350       360
m919.pep    KLGQTSMQGIKSYMRQNPQRLAEVLGQNPSYIFFRELAGSSNDGPVGALGTPLMGEYAGA
            |||||||||||:||||||||||||||||||||||||||||:|:|||||||||||||||||
g919        KLGQTSMQGIKAYMRQNPQRLAEVLGQNPSYIFFRELAGSGNEGPVGALGTPLMGEYAGA
                   310       320       330       340       350       360

                   370       380       390       400       410       420
m919.pep    VDRHYITLGAPLFVATAHPVTRKALNRLIMAQDTGSAIKGAVRVDYFWGYGDEAGELAGK
            :|||||||||||||||||||||||||||||||||||||||||||||||||||||||||||
g919        IDRHYITLGAPLFVATAHPVTRKALNRLIMAQDTGSAIKGAVRVDYFWGYGDEAGELAGK
                   370       380       390       400       410       420

                   430       440
m919.pep    QKTTGYVWQLLPNGMKPEYRPX
            |||||||||||||||||||||
g919        QKTTGYVWQLLPNGMKPEYRPX
                   430       440
```

The following partial DNA sequence was identified in *N.meningitidis* <SEQ ID 38>:

a919.seq

```
   1  ATGAAAAAAT ACCTATTCCG CGCCGCCCTG TGCGGCATCG CCGCCGCCAT
  51  CCTCGCCGCC TGCCAAAGCA AGAGCATCCA AACCTTTCCG CAACCCGACA
 101  CATCCGTCAT CAACGGCCCG GACCGGCCGG TCGGCATCCC CGACCCCGCC
 151  GGAACGACGG TCGGCGGCGG CGGGGCCGTT TATACCGTTG TGCCGCACCT
 201  GTCCCTGCCC CACTGGGCGG CGCAGGATTT CGCCAAAAGC CTGCAATCCT
 251  TCCGCCTCGG CTGCGCCAAT TTGAAAAACC GCCAAGGCTG GCAGGATGTG
 301  TGCGCCCAAG CCTTTCAAAC CCCCGTCCAT TCCGTTCAGG CAAAACAGTT
 351  TTTTGAACGC TATTTCACGC CGTGGCAGGT TGCAGGCAAC GGAAGCCTTG
 401  CCGGTACGGT TACCGGCTAT TACGAGCCGG TGCTGAAGGG CGACGACAGG
 451  CGGACGGCAC AAGCCCGCTT CCCGATTTAC GGTATTCCCG ACGATTTTAT
 501  CTCCGTCCCC CTGCCTGCCG GTTTGCGGAG CGGAAAAGCC CTTGTCCGCA
 551  TCAGGCAGAC GGGAAAAAAC AGCGGCACAA TCGACAATAC CGGCGGCACA
 601  CATACCGCCG ACCTCTCCCA ATTCCCCATC ACTGCGCGCA CAACGGCAAT
 651  CAAAGGCAGG TTTGAAGGAA GCCGCTTCCT CCCCTACCAC ACGCGCAACC
 701  AAATCAACGG CGGCGCGCTT GACGGCAAAG CCCCGATACT CGGTTACGCC
 751  GAAGACCCCG TCGAACTTTT TTTTATGCAC ATCCAAGGCT CGGGCCGTCT
 801  GAAAACCCCG TCCGGCAAAT ACATCCGCAT CGGCTATGCC GACAAAAACG
 851  AACATCCCTA CGTTTCCATC GGACGCTATA TGGCGGACAA AGGCTACCTC
 901  AAGCTCGGGC AGACCTCGAT GCAGGGCATC AAAGCCTATA TGCAGCAAAA
 951  CCCGCAACGC CTCGCCGAAG TTTTGGGGCA AAACCCCAGC TATATCTTTT
1001  TCCGAGAGCT TACCGGAAGC AGCAATGACG GCCCTGTCGG CGCACTGGGC
1051  ACGCCGCTGA TGGGCGAGTA CGCCGGCGCA GTCGACCGGC ACTACATTAC
1101  CTTGGGCGCG CCCTTATTTG TCGCCACCGC CCATCCGGTT ACCCGCAAAG
1151  CCCTCAACCG CCTGATTATG GCGCAGGATA CCGGCAGCGC GATTAAAGGC
1201  GCGGTGCGCG TGGATTATTT TTGGGGATAC GGCGACGAAG CCGGCGAACT
1251  TGCCGGCAAA CAGAAAACCA CGGGATATGT CTGGCAGCTT CTGCCCAACG
1301  GTATGAAGCC CGAATACCGC CCGTAA
```

This corresponds to the amino acid sequence <SEQ ID 39; ORF 919.a>:

```
a919.pep
     1  MKKYLFRAAL CGIAAAILAA CQSKSIQTFP QPDTSVINGP DRPVGIPDPA
    51  GTTVGGGGAV YTVVPHLSLP HWAAQDFAKS LQSFRLGCAN LKNRQGWQDV
   101  CAQAFQTPVH SVQAKQFFER YFTPWQVAGN GSLAGTVTGY YEPVLKGDDR
   151  RTAQARFPIY GIPDDFISVP LPAGLRSGKA LVRIRQTGKN SGTIDNTGGT
   201  HTADLSQFPI TARTTAIKGR FEGSRFLPYH TRNQINGGAL DGKAPILGYA
   251  EDPVELFFMH IQGSGRLKTP SGKYIRIGYA DKNEHPYVSI GRYMADKGYL
   301  KLGQTSMQGI KAYMQQNPQR LAEVLGQNPS YIFFRELTGS SNDGPVGALG
   351  TPLMGEYAGA VDRHYITLGA PLFVATAHPV TRKALNRLIM AQDTGSAIKG
   401  AVRVDYFWGY GDEAGELAGK QKTTGYVWQL LPNGMKPEYR P*
```

**m919/a919 ORFs 919 and 919.a showed a 98.6% identity in 441 aa overlap**

```
                     10        20        30        40        50        60
m919.pep    MKKYLFRAALYGIAAAILAACQSKSIQTFPQPDTSVINGPDRPVGIPDPAGTTVGGGGAV
            |||||||||| |||||||||||||||||||||||||||||||||||||||||||||||||
a919        MKKYLFRAALCGIAAAILAACQSKSIQTFPQPDTSVINGPDRPVGIPDPAGTTVGGGGAV
                     10        20        30        40        50        60

                     70        80        90       100       110       120
m919.pep    YTVVPHLSLPHWAAQDFAKSLQSFRLGCANLKNRQGWQDVCAQAFQTPVHSFQAKQFFER
            ||||||||||||||||||||||||||||||||||||||||||||||||||| ||||||||
a919        YTVVPHLSLPHWAAQDFAKSLQSFRLGCANLKNRQGWQDVCAQAFQTPVHSVQAKQFFER
                     70        80        90       100       110       120

                    130       140       150       160       170       180
m919.pep    YFTPWQVAGNGSLAGTVTGYYEPVLKGDDRRTAQARFPIYGIPDDFISVPLPAGLRSGKA
            ||||||||||||||||||||||||||||||||||||||||||||||||||||||||||||
a919        YFTPWQVAGNGSLAGTVTGYYEPVLKGDDRRTAQARFPIYGIPDDFISVPLPAGLRSGKA
                    130       140       150       160       170       180

                    190       200       210       220       230       240
m919.pep    LVRIRQTGKNSGTIDNTGGTHTADLSRFPITARTTAIKGRFEGSRFLPYHTRNQINGGAL
            ||||||||||||||||||||||||||:|||||||||||||||||||||||||||||||||
```

```
a919        LVRIRQTGKNSGTIDNTGGTHTADLSQFPITARTTAIKGRFEGSRFLPYHTRNQINGGAL
                       190       200       210       220       230       240

                       250       260       270       280       290       300
m919.pep    DGKAPILGYAEDPVELFFMHIQGSGRLKTPSGKYIRIGYADKNEHPYVSIGRYMADKGYL
            ||||||||||||||||||||||||||||||||||||||||||||||||||||||||||||
a919        DGKAPILGYAEDPVELFFMHIQGSGRLKTPSGKYIRIGYADKNEHPYVSIGRYMADKGYL
                       250       260       270       280       290       300

                       310       320       330       340       350       360
m919.pep    KLGQTSMQGIKSYMRQNPQRLAEVLGQNPSYIFFRELAGSSNDGPVGALGTPLMGEYAGA
            |||||||||||:||:||||||||||||||||||||||||:||||||||||||||||||||
a919        KLGQTSMQGIKAYMQQNPQRLAEVLGQNPSYIFFRELTGSSNDGPVGALGTPLMGEYAGA
                       310       320       330       340       350       360

                       370       380       390       400       410       420
m919.pep    VDRHYITLGAPLFVATAHPVTRKALNRLIMAQDTGSAIKGAVRVDYFWGYGDEAGELAGK
            ||||||||||||||||||||||||||||||||||||||||||||||||||||||||||||
a919        VDRHYITLGAPLFVATAHPVTRKALNRLIMAQDTGSAIKGAVRVDYFWGYGDEAGELAGK
                       370       380       390       400       410       420

                       430       440
m919.pep    QKTTGYVWQLLPNGMKPEYRPX
            ||||||||||||||||||||||
a919        QKTTGYVWQLLPNGMKPEYRPX
                       430       440
```

121 and 121-1

The following partial DNA sequence was identified in *N. meningitidis* <SEQ ID 40>:

**m121.seq**

```
   1  ATGGAAACAC AGCTTTACAT CGGCATCATG TCGGGAACCA GCATGGACGG
  51  GGCGGATGCC GTACTGATAC GGATGGACGG CGGCAAATGG CTGGGCGCGG
 101  AAGGGCACGC CTTTACCCCC TACCCCGGCA GGTTACGCCG CCAATTGCTG
 151  GATTTGCAGG ACACAGGCGC AGACGAACTG CACCGCAGCA GGATTTTGTC
 201  GCAAGAACTC AGCCGCCTAT ATGCGCAAAC CGCCGCCGAA CTGCTGTGCA
 251  GTCAAAACCT CGCACCGTCC GACATTACCG CCCTCGGCTG CCACGGGCAA
 301  ACCGTCCGAC ACGCGCCGGA ACACGGTTAC AGCATACAGC TTGCCGATTT
 351  GCCGCTGCTG GCGxxxxxxx xxxxxxxxxx xxxxxxxxxx xxxxxxxxxx
 401  xxxxxxxxxx xxxxxxxxxx xxxxxxxxxx xxxxxxxxxx xxxxxxxxxx
 451  xxxxxxxxxx xxxxxxxxxx xxxxxxxxxx xxxxxxxxxx xxxxxxxxxx
 501  xxxxxxxxxx xxxxxxxxxx xxxxxxxxxx xxxxxxxxxx xxxxxxxxxx
 551  xxxxxxxxxx xxxxxxxxxx xxxxxxxxxx xxxxxxxxxx xxxxxxxxxx
 601  xxxxxxCAGC TTCCTTACGA CAAAAACGGT GCAAAGTCGG CACAAGGCAA
 651  CATATTGCCG CAACTGCTCG ACAGGCTGCT CGCCCACCCG TATTTCGCAC
 701  AACGCCACCC TAAAAGCACG GGGCGCGAAC TGTTTGCCAT AAATTGGCTC
 751  GAAACCTACC TTGACGGCGG CGAAAACCGA TACGACGTAT TGCGGACGCT
 801  TTCCCGTTTT ACCGCGCAAA CCGTTTGCGA CGCCGTCTCA CACGCAGCGG
 851  CAGATGCCCG TCAAATGTAC ATTTGCGACG GCGGCATCCG CAATCCTGTT
 901  TTAATGGCGG ATTTGGCAGA ATGTTTCGGC ACACGCGTTT CCCTGCACAG
 951  CACCGCCGAC CTGAACCTCG ATCCGCAATG GGTGGAAGCC GCCGnATTTG
1001  CGTGGTTGGC GGCGTGTTGG ATTAATCGCA TTCCCGGTAG TCCGCACAAA
1051  GCAACCGGCG CATCCAAACC GTGTATTCTG AnCGCGGGAT ATTATTATTG
1101  A
```

This corresponds to the amino acid sequence <SEQ ID 41; ORF 121>:

**m121.pep**

```
   1  METQLYIGIM SGTSMDGADA VLIRMDGGKW LGAEGHAFTP YPGRLRRQLL
  51  DLQDTGADEL HRSRILSQEL SRLYAQTAAE LLCSQNLAPS DITALGCHGQ
```

```
101  TVRHAPEHGY SIQLADLPLL Axxxxxxxxx xxxxxxxxxx xxxxxxxxxx
151  xxxxxxxxxx xxxxxxxxxx xxxxxxxxxx xxxxxxxxxx xxxxxxxxxx
201  xxQLPYDKNG AKSAQGNILP QLLDRLLAHP YFAQRHPKST GRELFAINWL
251  ETYLDGGENR YDVLRTLSRF TAQTVCDAVS HAAADARQMY ICDGGIRNPV
301  LMADLAECFG TRVSLHSTAD LNLDPQWVEA AXFAWLAACW INRIPGSPHK
351  ATGASKPCIL XAGYYY*
```

The following partial DNA sequence was identified in *N. gonorrhoeae* <SEQ ID 42>:

**g121.seq**

```
   1  ATGGAAACAC AGCTTTACAT CGGCATTATG TCGGGAACCA GTATGGACGG
  51  GGCGGATGCC GTGCTGGTAC GGATGGACGG CGGCAAATGG CTGGGCGCGG
 101  AAGGGCACGC CTTTACCCCC TACCCTGACC GGTTGCGCCG CAAATTGCTG
 151  GATTTGCAGG ACACAGGCAC AGACGAACTG CACCGCAGCA GGATGTTGTC
 201  GCAAGAACTC AGCCGCCTGT ACGCGCAAAC CGCCGCCGAA CTGCTGTGCA
 251  GTCAAAACCT CGCTCCGTGC GACATTACCG CCCTCGGCTG CCACGGGCAA
 301  ACCGTCCGAC ACGCGCCGGA ACACGGTtac AGCATACAGC TTGCCGATTT
 351  GCCGCTGCTG GCGGAACTGa cgcggatttT TACCGTCggc gacttcCGCA
 401  GCCGCGACCT TGCTGCCGGC GGacaAGGTG CGCCGCTCGT CCCCGCCTTT
 451  CACGAAGCCC TGTTCCGCGA TGACAGGGAA ACACGCGTGG TACTGAACAT
 501  CGGCGGGATT GCCAACATCA GCGTACTCCC CCCCGGCGCA CCCGCCTTCG
 551  GCTTCGACAC AGGGCCGGGC AATATGCTGA TGGAcgcgtg gacgcaggca
 601  cacTGGcagc TGCCTTACGA CAAAAacggt gcAAAGgcgg cacAAGGCAA
 651  catatTGCcg cAACTGCTCG gcaggctGCT CGCCcaccCG TATTTCTCAC
 701  AACCCcaccc aaAAAGCACG GGgcGCGaac TgtttgcccT AAattggctc
 751  gaaacctAcc ttgacggcgg cgaaaaccga tacgacgtat tgcggacgct
 801  ttcccgattc accgcgcaaA ccgTttggga cgccgtctca CACGCAGCGG
 851  CAGATGCCCG TCAAATGTAC ATTTGCGGCG GCGGCATCCG CAATCCTGTT
 901  TTAATGGCGG ATTTGGCAGA ATGTTTCGGC ACACGCGTTT CCCTGCACAG
 951  CACCGCCGAA CTGAACCTCG ATCCTCAATG GGTGGAGGCG gccgCATTtg
1001  cgtggttggC GGCGTGTTGG ATTAACCGCA TTCCCGGTAG TCCGCACAAA
1051  GCGACCGGCG CATCCAAACC GTGTATTCTG GGCGCGGGAT ATTATTATTG
1101  A
```

This corresponds to the amino acid sequence <SEQ ID 43; ORF 121.ng>:

**g121.pep**

```
  1  METQLYIGIM SGTSMDGADA VLVRMDGGKW LGAEGHAFTP YPDRLRRKLL
 51  DLQDTGTDEL HRSRMLSQEL SRLYAQTAAE LLCSQNLAPC DITALGCHGQ
101  TVRHAPEHGY SIQLADLPLL AELTRIFTVG DFRSRDLAAG GQGAPLVPAF
151  HEALFRDDRE TRVVLNIGGI ANISVLPPGA PAFGFDTGPG NMLMDAWTQA
201  HWQLPYDKNG AKAAQGNILP QLLGRLLAHP YFSQPHPKST GRELFALNWL
251  ETYLDGGENR YDVLRTLSRF TAQTVWDAVS HAAADARQMY ICGGGIRNPV
301  LMADLAECFG TRVSLHSTAE LNLDPQWVEA AAFAWLAACW INRIPGSPHK
351  ATGASKPCIL GAGYYY*
```

ORF 121 shows 73.5% identity over a 366 aa overlap with a predicted ORF (ORF121.ng) from *N. gonorrhoeae:*

**m121/g121**

```
                     10        20        30        40        50        60
m121.pep    METQLYIGIMSGTSMDGADAVLIRMDGGKWLGAEGHAFTPYPGRLRRQLLDLQDTGADEL
            ||||||||||||||||||||||:||||||||||||||||||| ||||:||||||||:|||
g121        METQLYIGIMSGTSMDGADAVLVRMDGGKWLGAEGHAFTPYPDRLRRKLLDLQDTGTDEL
                     10        20        30        40        50        60
                     70        80        90       100       110       120
m121.pep    HRSRILSQELSRLYAQTAAELLCSQNLAPSDITALGCHGQTVRHAPEHGYSIQLADLPLL
            ||||:|||||||||||||||||||||||| ||||||||||||||||||||||||||||||
g121        HRSRMLSQELSRLYAQTAAELLCSQNLAPCDITALGCHGQTVRHAPEHGYSIQLADLPLL
                     70        80        90       100       110       120
                    130       140       150       160       170       180

m121.pep    AXXXXXXXXXXXXXXXXXXXXXXXXXXXXXXXXXXXXXXXXXXXXXXXXXXXXXXXXXXX
            |  :   :                                :
g121        AELTRIFTVGDFRSRDLAAGGQGAPLVPAFHEALFRDDRETRVVLNIGGIANISVLPPGA
                    130       140       150       160       170       180
                    190       200       210       220       230       240
m121.pep    XXXXXXXXXXXXXXXXXXXXXXQLPYDKNGAKSAQGNILPQLLDRLLAHPYFAQRHPKST
                  :           :   ||||||||||:|||||||||| ||||||||:| |||||
g121        PAFGFDTGPGNMLMDAWTQAHWQLPYDKNGAKAAQGNILPQLLGRLLAHPYFSQPHPKST
                    190       200       210       220       230       240
                    250       260       270       280       290       300
m121.pep    GRELFAINWLETYLDGGENRYDVLRTLSRFTAQTVCDAVSHAAADARQMYICDGGIRNPV
            ||||||:|||||||||||||||||||||||||||| |||||||||||||||| |||||||
g121        GRELFALNWLETYLDGGENRYDVLRTLSRFTAQTVWDAVSHAAADARQMYICGGGIRNPV
                    250       260       270       280       290       300
                    310       320       330       340       350       360
m121.pep    LMADLAECFGTRVSLHSTADLNLDPQWVEAAXFAWLAACWINRIPGSPHKATGASKPCIL
            |||||||||||||||||||:||||||||||| ||||||||||||||||||||||||||||
g121        LMADLAECFGTRVSLHSTAELNLDPQWVEAAAFAWLAACWINRIPGSPHKATGASKPCIL
                    310       320       330       340       350       360

m121.pep    XAGYYYX
             ||||||
g121        GAGYYYX
```

The following partial DNA sequence was identified in *N. meningitidis* <SEQ ID 44>:

```
a121.seq
     1  ATGGAAACAC AGCTTTACAT CGGCATCATG TCGGGAACCA GCATGGACGG
    51  GGCGGATGCC GTACTGATAC GGATGGACGG CGGCAAATGG CTGGGCGCGG
   101  AAGGGCACGC CTTTACCCCC TACCCCGGCA GGTTACGCCG CAAATTGCTG
   151  GATTTGCAGG ACACAGGCGC GGACGAACTG CACCGCAGCA GGATGTTGTC
   201  GCAAGAACTC AGCCGCCTGT ACGCGCAAAC CGCCGCCGAA CTGCTGTGCA
   251  GTCAAAACCT CGCGCCGTCC GACATTACCG CCCTCGGCTG CCACGGGCAA
   301  ACCGTCAGAC ACGCGCCGGA ACACAGTTAC AGCGTACAGC TTGCCGATTT
   351  GCCGCTGCTG GCGGAACGGA CTCAGATTTT TACCGTCGGC GACTTCCGCA
   401  GCCGCGACCT TGCGGCCGGC GGACAAGGCG CGCCGCTCGT CCCCGCCTTT
   451  CACGAAGCCC TGTTCCGCGA CGACAGGGAA ACACGCGCGG TACTGAACAT
   501  CGGCGGGATT GCCAACATCA GCGTACTCCC CCCCGACGCA CCCGCCTTCG
   551  GCTTCGACAC AGGACCGGGC AATATGCTGA TGGACGCGTG GATGCAGGCA
   601  CACTGGCAGC TTCCTTACGA CAAAAACGGT GCAAAGGCGG CACAAGGCAA
   651  CATATTGCCG CAACTGCTCG ACAGGCTGCT CGCCCACCCG TATTTCGCAC
   701  AACCCCACCC TAAAAGCACG GGGCGCGAAC TGTTTGCCCT AAATTGGCTC
   751  GAAACCTACC TTGACGGCGG CGAAAACCGA TACGACGTAT TGCGGACGCT
   801  TTCCCGATTC ACCGCGCAAA CCGTTTTCGA CGCCGTCTCA CACGCAGCGG
   851  CAGATGCCCG TCAAATGTAC ATTTGCGGCG GCGGCATCCG CAATCCTGTT
   901  TTAATGGCGG ATTTGGCAGA ATGTTTCGGC ACACGCGTTT CCCTGCACAG
   951  CACCGCCGAA CTGAACCTCG ATCCGCAATG GGTAGAAGCC GCCGCGTTCG
  1001  CATGGATGGC GGCGTGTTGG GTCAACCGCA TTCCCGGTAG TCCGCACAAA
  1051  GCAACCGGCG CATCCAAACC GTGTATTCTG GGCGCGGGAT ATTATTATTG
  1101  A
```

This corresponds to the amino acid sequence <SEQ ID 45; ORF 121.a>:

```
a121.pep
     1  METQLYIGIM SGTSMDGADA VLIRMDGGKW LGAEGHAFTP YPGRLRRKLL
    51  DLQDTGADEL HRSRMLSQEL SRLYAQTAAE LLCSQNLAPS DITALGCHGQ
   101  TVRHAPEHSY SVQLADLPLL AERTQIFTVG DFRSRDLAAG GQGAPLVPAF
   151  HEALFRDDRE TRAVLNIGGI ANISVLPPDA PAFGFDTGPG NMLMDAWMQA
   201  HWQLPYDKNG AKAAQGNILP QLLDRLLAHP YFAQPHPKST GRELFALNWL
   251  ETYLDGGENR YDVLRTLSRF TAQTVFDAVS HAAADARQMY ICGGGIRNPV
   301  LMADLAECFG TRVSLHSTAE LNLDPQWVEA AAFAWMAACW VNRIPGSPHK
```

```
351  ATGASKPCIL GAGYYY*
```

**m121/a121** **ORFs 121 and 121.a** 74.0% identity in 366 aa overlap

```
                    10        20        30        40        50        60
m121.pep    METQLYIGIMSGTSMDGADAVLIRMDGGKWLGAEGHAFTPYPGRLRRQLLDLQDTGADEL
            ||||||||||||||||||||||||||||||||||||||||||||||||:|||||||||||
a121        METQLYIGIMSGTSMDGADAVLIRMDGGKWLGAEGHAFTPYPGRLRRKLLDLQDTGADEL
                    10        20        30        40        50        60

                    70        80        90       100       110       120
m121.pep    HRSRILSQELSRLYAQTAAELLCSQNLAPSDITALGCHGQTVRHAPEHGYSIQLADLPLL
            ||||:||||||||||||||||||||||||||||||||||||||||||||:||:|||||||
a121        HRSRMLSQELSRLYAQTAAELLCSQNLAPSDITALGCHGQTVRHAPEHSYSVQLADLPLL
                    70        80        90       100       110       120

                   130       140       150       160       170       180
m121.pep    AXXXXXXXXXXXXXXXXXXXXXXXXXXXXXXXXXXXXXXXXXXXXXXXXXXXXXXXXXXX
            | :  :                                      :
a121        AERTQIFTVGDFRSRDLAAGGQGAPLVPAFHEALFRDDRETRAVLNIGGIANISVLPPDA
                   130       140       150       160       170       180

                   190       200       210       220       230       240
m121.pep    XXXXXXXXXXXXXXXXXXXXXXQLPYDKNGAKSAQGNILPQLLDRLLAHPYFAQRHPKST
               :                  ||||||||||:||||||||||||||||||| |||||
a121        PAFGFDTGPGNMLMDAWMQAHWQLPYDKNGAKAAQGNILPQLLDRLLAHPYFAQPHPKST
                   190       200       210       220       230       240

                   250       260       270       280       290       300
m121.pep    GRELFAINWLETYLDGGENRYDVLRTLSRFTAQTVCDAVSHAAADARQMYICDGGIRNPV
            ||||||:|||||||||||||||||||||||||||| ||||||||||||||| |||||||
a121        GRELFALNWLETYLDGGENRYDVLRTLSRFTAQTVFDAVSHAAADARQMYICGGGIRNPV
                   250       260       270       280       290       300

                   310       320       330       340       350       360
m121.pep    LMADLAECFGTRVSLHSTADLNLDPQWVEAAXFAWLAACWINRIPGSPHKATGASKPCIL
            |||||||||||||||||||:||||||||||| |||:||||:|||||||||||||||||||
a121        LMADLAECFGTRVSLHSTAELNLDPQWVEAAAFAWMAACWVNRIPGSPHKATGASKPCIL
                   310       320       330       340       350       360

m121.pep    XAGYYYX
             ||||||
a121        GAGYYYX
```

Further work revealed the DNA sequence identified in *N. meningitidis* <SEQ ID 46>:

```
m121-1.seq
      1  ATGGAAACAC AGCTTTACAT CGGCATCATG TCGGGAACCA GCATGGACGG
     51  GGCGGATGCC GTACTGATAC GGATGGACGG CGGCAAATGG CTGGGCGCGG
    101  AAGGGCACGC CTTTACCCCC TACCCCGGCA GGTTACGCCG CCAATTGCTG
    151  GATTTGCAGG ACACAGGCGC AGACGAACTG CACCGCAGCA GGATTTTGTC
    201  GCAAGAACTC AGCCGCCTAT ATGCGCAAAC CGCCGCCGAA CTGCTGTGCA
    251  GTCAAAACCT CGCACCGTCC GACATTACCG CCCTCGGCTG CCACGGGCAA
    301  ACCGTCCGAC ACGCGCCGGA ACACGGTTAC AGCATACAGC TTGCCGATTT
    351  GCCGCTGCTG GCGGAACGGA CGCGGATTTT TACCGTCGGC GACTTCCGCA
    401  GCCGCGACCT TGCGGCCGGC GGACAAGGCG CGCCACTCGT CCCCGCCTTT
    451  CACGAAGCCC TGTTCCGCGA CAACAGGGAA ACACGCGCGG TACTGAACAT
    501  CGGCGGGATT GCCAACATCA GCGTACTCCC CCCCGACGCA CCCGCCTTCG
    551  GCTTCGACAC AGGGCCGGGC AATATGCTGA TGGACGCGTG GACGCAGGCA
    601  CACTGGCAGC TTCCTTACGA CAAAAACGGT GCAAAGGCGG CACAAGGCAA
    651  CATATTGCCG CAACTGCTCG ACAGGCTGCT CGCCCACCCG TATTTCGCAC
    701  AACCCCACCC TAAAAGCACG GGGCGCGAAC TGTTTGCCCT AAATTGGCTC
    751  GAAACCTACC TTGACGGCGG CGAAAACCGA TACGACGTAT TGCGGACGCT
```

```
 801  TTCCCGTTTT ACCGCGCAAA CCGTTTGCGA CGCCGTCTCA CACGCAGCGG
 851  CAGATGCCCG TCAAATGTAC ATTTGCGGCG GCGGCATCCG CAATCCTGTT
 901  TTAATGGCGG ATTTGGCAGA ATGTTTCGGC ACACGCGTTT CCCTGCACAG
 951  CACCGCCGAC CTGAACCTCG ATCCGCAATG GGTGGAAGCC GCCGNATTTG
1001  CGTGGTTGGC GGCGTGTTGG ATTAATCGCA TTCCCGGTAG TCCGCACAAA
1051  GCAACCGGCG CATCCAAACC GTGTATTCTG ANCGCGGGAT ATTATTATTG
1101  A
```

This corresponds to the amino acid sequence <SEQ ID 47; ORF 121-1>:

```
m121-1.pep
  1  METQLYIGIM SGTSMDGADA VLIRMDGGKW LGAEGHAFTP YPGRLRRQLL
 51  DLQDTGADEL HRSRILSQEL SRLYAQTAAE LLCSQNLAPS DITALGCHGQ
101  TVRHAPEHGY SIQLADLPLL AERTRIFTVG DFRSRDLAAG GQGAPLVPAF
151  HEALFRDNRE TRAVLNIGGI ANISVLPPDA PAFGFDTGPG NMLMDAWTQA
201  HWQLPYDKNG AKAAQGNILP QLLDRLLAHP YFAQPHPKST GRELFALNWL
251  ETYLDGGENR YDVLRTLSRF TAQTVCDAVS HAAADARQMY ICGGGIRNPV
301  LMADLAECFG TRVSLHSTAD LNLDPQWVEA AXFAWLAACW INRIPGSPHK
351  ATGASKPCIL XAGYYY*
```

**m121-1/g121** **ORFs 121-1 and 121-1.ng showed a** 95.6% identity in 366 aa overlap

```
                    10        20        30        40        50        60
m121-1.pep   METQLYIGIMSGTSMDGADAVLIRMDGGKWLGAEGHAFTPYPGRLRRQLLDLQDTGADEL
             ||||||||||||||||||||||:||||||||||||||||||| ||||:||||||||:|||
g121         METQLYIGIMSGTSMDGADAVLVRMDGGKWLGAEGHAFTPYPDRLRRKLLDLQDTGTDEL
                    10        20        30        40        50        60

                    70        80        90       100       110       120
m121-1.pep   HRSRILSQELSRLYAQTAAELLCSQNLAPSDITALGCHGQTVRHAPEHGYSIQLADLPLL
             ||||:||||||||||||||||||||||||| ||||||||||||||||||||||||||||||
g121         HRSRMLSQELSRLYAQTAAELLCSQNLAPCDITALGCHGQTVRHAPEHGYSIQLADLPLL
                    70        80        90       100       110       120

                   130       140       150       160       170       180
m121-1.pep   AERTRIFTVGDFRSRDLAAGGQGAPLVPAFHEALFRDNRETRAVLNIGGIANISVLPPDA
             || ||||||||||||||||||||||||||||||||||:||||:||||||||||||||| |
g121         AELTRIFTVGDFRSRDLAAGGQGAPLVPAFHEALFRDDRETRVVLNIGGIANISVLPPGA
                   130       140       150       160       170       180

                   190       200       210       220       230       240
m121-1.pep   PAFGFDTGPGNMLMDAWTQAHWQLPYDKNGAKAAQGNILPQLLDRLLAHPYFAQPHPKST
             |||||||||||||||||||||||||||||||||||||||||||| ||||||||:||||||
g121         PAFGFDTGPGNMLMDAWTQAHWQLPYDKNGAKAAQGNILPQLLGRLLAHPYFSQPHPKST
                   190       200       210       220       230       240

                   250       260       270       280       290       300
m121-1.pep   GRELFALNWLETYLDGGENRYDVLRTLSRFTAQTVCDAVSHAAADARQMYICGGGIRNPV
             |||||||||||||||||||||||||||||||||||| |||||||||||||||||||||||
g121         GRELFALNWLETYLDGGENRYDVLRTLSRFTAQTVWDAVSHAAADARQMYICGGGIRNPV
                   250       260       270       280       290       300

                   310       320       330       340       350       360
m121-1.pep   LMADLAECFGTRVSLHSTADLNLDPQWVEAAXFAWLAACWINRIPGSPHKATGASKPCIL
             |||||||||||||||||||:|||||||||||| |||||||||||||||||||||||||||
g121         LMADLAECFGTRVSLHSTAELNLDPQWVEAAAFAWLAACWINRIPGSPHKATGASKPCIL
                   310       320       330       340       350       360

m121-1.pep   XAGYYYX
              ||||||
g121         GAGYYYX
```

The following partial DNA sequence was identified in *N. meningitidis* <SEQ ID 48>:

```
a121-1.seq
     1  ATGGAAACAC AGCTTTACAT CGGCATCATG TCGGGAACCA GCATGGACGG
    51  GGCGGATGCC GTACTGATAC GGATGGACGG CGGCAAATGG CTGGGCGCGG
   101  AAGGGCACGC CTTTACCCCC TACCCCGGCA GGTTACGCCG CAAATTGCTG
   151  GATTTGCAGG ACACAGGCGC GGACGAACTG CACCGCAGCA GGATGTTGTC
   201  GCAAGAACTC AGCCGCCTGT ACGCGCAAAC CGCCGCCGAA CTGCTGTGCA
   251  GTCAAAACCT CGCGCCGTCC GACATTACCG CCCTCGGCTG CCACGGGCAA
   301  ACCGTCAGAC ACGCGCCGGA ACACAGTTAC AGCGTACAGC TTGCCGATTT
   351  GCCGCTGCTG GCGGAACGGA CTCAGATTTT TACCGTCGGC GACTTCCGCA
   401  GCCGCGACCT TGCGGCCGGC GGACAAGGCG CGCCGCTCGT CCCCGCCTTT
   451  CACGAAGCCC TGTTCCGCGA CGACAGGGAA ACACGCGCGG TACTGAACAT
   501  CGGCGGGATT GCCAACATCA GCGTACTCCC CCCCGACGCA CCCGCCTTCG
   551  GCTTCGACAC AGGACCGGGC AATATGCTGA TGGACGCGTG GATGCAGGCA
   601  CACTGGCAGC TTCCTTACGA CAAAAACGGT GCAAAGGCGG CACAAGGCAA
   651  CATATTGCCG CAACTGCTCG ACAGGCTGCT CGCCCACCCG TATTTCGCAC
   701  AACCCCACCC TAAAAGCACG GGGCGCGAAC TGTTTGCCCT AAATTGGCTC
   751  GAAACCTACC TTGACGGCGG CGAAAACCGA TACGACGTAT TGCGGACGCT
   801  TTCCCGATTC ACCGCGCAAA CCGTTTTCGA CGCCGTCTCA CACGCAGCGG
   851  CAGATGCCCG TCAAATGTAC ATTTGCGGCG GCGGCATCCG CAATCCTGTT
   901  TTAATGGCGG ATTTGGCAGA ATGTTTCGGC ACACGCGTTT CCCTGCACAG
   951  CACCGCCGAA CTGAACCTCG ATCCGCAATG GGTAGAAGCC GCCGCGTTCG
  1001  CATGGATGGC GGCGTGTTGG GTCAACCGCA TTCCCGGTAG TCCGCACAAA
  1051  GCAACCGGCG CATCCAAACC GTGTATTCTG GGCGCGGGAT ATTATTATTG
  1101  A
```

This corresponds to the amino acid sequence <SEQ ID 49; ORF 121-1.a>:

```
a121-1.pep
     1  METQLYIGIM SGTSMDGADA VLIRMDGGKW LGAEGHAFTP YPGRLRRKLL
    51  DLQDTGADEL HRSRMLSQEL SRLYAQTAAE LLCSQNLAPS DITALGCHGQ
   101  TVRHAPEHSY SVQLADLPLL AERTQIFTVG DFRSRDLAAG GQGAPLVPAF
   151  HEALFRDDRE TRAVLNIGGI ANISVLPPDA PAFGFDTGPG NMLMDAWMQA
   201  HWQLPYDKNG AKAAQGNILP QLLDRLLAHP YFAQPHPKST GRELFALNWL
   251  ETYLDGGENR YDVLRTLSRF TAQTVFDAVS HAAADARQMY ICGGGIRNPV
   301  LMADLAECFG TRVSLHSTAE LNLDPQWVEA AAFAWMAACW VNRIPGSPHK
   351  ATGASKPCIL GAGYYY*
```

**m121-1/a121-1 ORFs 121-1 and 121-1.a showed a** 96.4% identity in 366 aa overlap

```
                    10        20        30        40        50        60
m121-1.pep   METQLYIGIMSGTSMDGADAVLIRMDGGKWLGAEGHAFTPYPGRLRRQLLDLQDTGADEL
             ||||||||||||||||||||||||||||||||||||||||||||||||:|||||||||||
a121-1       METQLYIGIMSGTSMDGADAVLIRMDGGKWLGAEGHAFTPYPGRLRRKLLDLQDTGADEL
                    10        20        30        40        50        60

                    70        80        90       100       110       120
m121-1.pep   HRSRILSQELSRLYAQTAAELLCSQNLAPSDITALGCHGQTVRHAPEHGYSIQLADLPLL
             ||||:|||||||||||||||||||||||||||||||||||||||||||:||:||||||||
a121-1       HRSRMLSQELSRLYAQTAAELLCSQNLAPSDITALGCHGQTVRHAPEHSYSVQLADLPLL
                    70        80        90       100       110       120

                   130       140       150       160       170       180
m121-1.pep   AERTRIFTVGDFRSRDLAAGGQGAPLVPAFHEALFRDNRETRAVLNIGGIANISVLPPDA
             ||||:||||||||||||||||||||||||||||||||:||||||||||||||||||||||
a121-1       AERTQIFTVGDFRSRDLAAGGQGAPLVPAFHEALFRDDRETRAVLNIGGIANISVLPPDA
                   130       140       150       160       170       180

                   190       200       210       220       230       240
m121-1.pep   PAFGFDTGPGNMLMDAWTQAHWQLPYDKNGAKAAQGNILPQLLDRLLAHPYFAQPHPKST
             |||||||||||||||| |||||||||||||||||||||||||||||||||||||||||||
a121-1       PAFGFDTGPGNMLMDAWMQAHWQLPYDKNGAKAAQGNILPQLLDRLLAHPYFAQPHPKST
                   190       200       210       220       230       240
```

```
                   250       260       270       280       290       300
m121-1.pep  GRELFALNWLETYLDGGENRYDVLRTLSRFTAQTVCDAVSHAAADARQMYICGGGIRNPV
            |||||||||||||||||||||||||||||||||||| |||||||||||||||||||||||
a121-1      GRELFALNWLETYLDGGENRYDVLRTLSRFTAQTVFDAVSHAAADARQMYICGGGIRNPV
                   250       260       270       280       290       300

                   310       320       330       340       350       360
m121-1.pep  LMADLAECFGTRVSLHSTADLNLDPQWVEAAXFAWLAACWINRIPGSPHKATGASKPCIL
            ||||||||||||||||||:|||||||||||| |||:||||:|||||||||||||||||||
a121        LMADLAECFGTRVSLHSTAELNLDPQWVEAAAFAWMAACWVNRIPGSPHKATGASKPCIL
                   310       320       330       340       350       360

m121-1.pep  XAGYYYX
             ||||||
a121        GAGYYYX
```

## 128 and 128-1

The following partial DNA sequence was identified in *N. meningitidis* <SEQ ID 50>:

```
m128.seq  (partial)
       1  ATGACTGACA ACGCACTGCT CCATTTGGGC GAAGAACCCC GTTTTGATCA
      51  AATCAAAACC GAAGACATCA AACCCGCCCT GCAAACCGCC ATCGCCGAAG
     101  CGCGCGAACA AATCGCCGCC ATCAAAGCCC AAACGCACAC CGGCTGGGCA
     151  AACACTGTCG AACCCCTGAC CGGCATCACC GAACGCGTCG GCAGGATTTG
     201  GGGCGTGGTG TCGCACCTCA ACTGCGTCGC CGACACGCCC GAACTGCGCG
     251  CCGTCTATAA CGAACTGATG CCCGAAATCA CCGTCTTCTT CACCGAAATC
     301  GGACAAGACA TCGAGCTGTA CAACCGCTTC AAAACCATCA AAAATTCCCC
     351  CGAATTCGAC ACCCTCTCCC CCGCACAAAA AACCAAACTC AACCAC
       1  TACGCCAGCG AAAAACTGCG CGAAGCCAAA TACGCGTTCA GCGAAACCGA
      51  wGTCAAAAAA TAyTTCCCyG TCGGCAAwGT ATTAAACGGA CTGTTCGCCC
     101  AAmTCAAAAA ACTmTACGGC ATCGGATTTA CCGAAAAAAC yGTCCCCGTC
     151  TGGCACAAAG ACGTGCGCTA TTkTGAATTG CAACAAAACG GCGAAmCCAT
     201  AGGCGGCGTT TATATGGATT TGTACGCACG CGAAGGCAAA CGCGGCGGCG
     251  CGTGGATGAA CGACTACAAA GGCCGCCGCC GTTTTTCAGA CGGCACGCTG
     301  CAAyTGCCCA CCGCCTACCT CGTCTGCAAC TTCGCCCCAC CCGTCGGCGG
     351  CAGGGAAGCC CGCyTGAGCC ACGACGAAAT CCTCATCCTC TTCCACGAAA
     401  CCGGACACGG GCTGCACCAC CTGCTTACCC AAGTGGACGA ACTGGGCGTA
     451  TCCGGCATCA ACGGCGTAkA ATGGGACGCG GTCGAACTGC CCAGCCAGTT
     501  TATGGAAAAT TTCGTTTGGG AATACAATGT CTTGGCACAA mTGTCAGCCC
     551  ACGAAGAAAC CGGcgTTCCC yTGCCGAAAG AACTCTTsGA CAAAwTGCTC
     601  GCCGCCAAAA ACTTCCAAsG CGGCATGTTC yTsGTCCGGC AAwTGGAGTT
     651  CGCCCTCTTT GATATGATGA TTTACAGCGA AGACGACGAA GGCCGTCTGA
     701  AAAACTGGCA ACAGGTTTTA GACAGCGTGC GCAAAAAAGT CGCCGTCATC
     751  CAGCCGCCCG AATACAACCG CTTCGCCTTG AGCTTCGGCC ACATCTTCGC
     801  AGGCGGCTAT TCCGCAGCTn ATTACAGCTA CGCGTGGGCG GAAGTATTGA
     851  GCGCGGACGC ATACGCCGCC TTTGAAGAAA GCGACGATGT CGCCGCCACA
     901  GGCAAACGCT TTTGGCAGGA AATCCTCGCC GTCGGGGnAT CGCGCAGCGG
     951  nGCAGAATCC TTCAAAGCCT TCCGCGGCCG CGAACCGAGC ATAGACGCAC
    1001  TCTTGCGCCA CAGCGGTTTC GACAACGCGG TCTGA
```

This corresponds to the amino acid sequence <SEQ ID 51; ORF 128>:

```
m128.pep   (partial)
      1  MTDNALLHLG EEPRFDQIKT EDIKPALQTA IAEAREQIAA IKAQTHTGWA
     51  NTVEPLTGIT ERVGRIWGVV SHLNCVADTP ELRAVYNELM PEITVFFTEI
    101  GQDIELYNRF KTIKNSPEFD TLSPAQKTKL NH
//
      1  YASEKLREAK YAFSETXVKK YFPVGXVLNG LFAQXKKLYG IGFTEKTVPV

     51  WHKDVRYXEL QQNGEXIGGV YMDLYAREGK RGGAWMNDYK GRRRFSDGTL
    101  QLPTAYLVCN FAPPVGGREA RLSHDEILIL FHETGHGLHH LLTQVDELGV
    151  SGINGVXWDA VELPSQFMEN FVWEYNVLAQ XSAHEETGVP LPKELXDKXL
    201  AAKNFQXGMF XVRQXEFALF DMMIYSEDDE GRLKNWQQVL DSVRKKVAVI
    251  QPPEYNRFAL SFGHIFAGGY SAAXYSYAWA EVLSADAYAA FEESDDVAAT
    301  GKRFWQEILA VGXSRSGAES FKAFRGREPS IDALLRHSGF DNAV*
```

The following partial DNA sequence was identified in *N. gonorrhoeae* <SEQ ID 52>:

```
g128.seq
      1  atgattgaca acgCActgct ccacttgggc gaagaaccCC GTTTTaatca
     51  aatccaaacc gaagACAtca AACCCGCCGT CCAAACCGCC ATCGCCGAAG
    101  CGCGCGGACA AATCGCCGCC GTCAAAGCGC AAACGCACAC CGGCTGGGCG
    151  AACACCGTCG AGCGTCTGAC CGGCATCACC GAACGCGTCG GCAGGATTTG
    201  GGGCGTCGTG TCCCATCTCA ACTCCGTCGT CGACACGCCC GAACTGCGCG
    251  CCGTCTATAA CGAACTGATG CCTGAAATCA CCGTCTTCTT CACCGAAATC
    301  GGACAAGACA TCGAACTGTA CAACCGCTTC AAAACCATCA AAAATTCCCC
    351  CGAATTTGCA ACGCTTTCCC CCGCACAAAA AACCAAGCTC GATCACGACC
    401  TGCGCGATTT CGTATTGAGC GGCGCGGAAC TGCCGCCCGA ACGGCAGGCA
    451  GAACTGGCAA AACTGCAAAC CGAAGGCGCG CAACTTTCCG CCAAATTCTC
    501  CCAAAACGTC CTAGACGCGA CCGACGCGTT CGGCATTTAC TTTGACGATG
    551  CCGCACCGCT TGCCGGCATT CCCGAAGACG CGCTCGCCAT GTTTGCCGCC
    601  GCCGCGCAAA GCGAAGGCAA AACAGGTTAC AAAATCGGCT TGCAGATTCC
    651  GCACTACCTT GCCGTTATCC AATACGCCGG CAACCGCGAA CTGCGCGAAC
    701  AAATCTACCG CGCCTACGTT ACCCGTGCCA GCGAACTTTC AAACGACGGC
    751  AAATTCGACA ACACCGCCAA CATCGACCGC ACGCTCGAAA ACGCATTGAA
    801  AACCGccaaa cTGCTCGGCT TTAAAAATTA CGCCGAATTG TCGCTGGCAA
    851  CCAAAATGGC GGACACGCCC GAACAGGTTT TAAACTTCCT GCACGACCTC
    901  GCCCGCCGCG CCAAACCCTA CGCCGAAAAA GACCTCGCCG AAGTCAAAGC
    951  CTTCGCCCGC GAACACCTCG GTCTCGCCGA CCCGCAGCCG TGGGACTTGA
   1001  GCTACGCCGG CGAAAAACTG CGCGAAGCCA AATACGCATT CAGCGAAACC
   1051  GAAGTCAAAA AATACTTCCC CGTCGGCAAA GTTCTGGCAG GCCTGTTCGC
   1101  CCAAATCAAA AAACTCTACG GCATCGGATT CGCCGAAAAA ACCGTTCCCG
   1151  TCTGGCACAA AGACGTGCGC TATTTTGAAT TGCAACAAAA CGGCAAAACC
   1201  ATCGGCGGCG TTTATATGGA TTTGTACGCA CGCGAAGGCA AACGCGGCGG
   1251  CGCGTGGATG AACGACtaca AAGGCCGCCG CCGCTTTGCC GACGgcacGC
   1301  TGCAACTGCC CACCGCCTAC CTCGTCTGCA ACTTCGCCCC GCCCGTCGGC
   1351  GGCAAAGAAG CGCGTTTAAG CCACGACGAA ATCCTCACCC TCTTCCACGA
   1401  AacCGGCCAC GGACTGCACC ACCTGCTTAC CCAAGTGGAC GAACTGGGCG
   1451  TGTCCGGCAT CAAcggcgtA GAATGGGACG CGGTCGAACT GCCCAGCCAG
   1501  TTTATGGAAA ACTTCGTTTG GGAATACAAT GTATTGGCAC AAATGTCCGC
   1551  CCACGAAGAA AccgGCGAGC CCCTGCCGAA AGAACTCTTC GACAAAATGC
   1601  TcgcCGCCAA AAACTTCCAG CGCGGTATGT TCCTCGTCCG GCAAATGGAG
   1651  TTCGCCCTCT TCGATATGAT GATTTACAGT GAAAGCGACG AATGCCGTCT
   1701  GAAAAACTGG CAGCAGGTTT TAGACAGCGT GCGCAAAGAA GTcGCCGTCA
   1751  TCCAACCGCC CGAATACAAC CGCTTCGCCA ACAGCTTCGG CCacatctTC
   1801  GCcggcGGCT ATTCCGCAGG CTATTACAGC TACGCATGGG CCGAAGTCCt
   1851  cAGCACCGAT GCCTACGCCG CCTTTGAAGA AAGcGACGac gtcGCCGCCA
   1901  CAGGCAAACG CTTCTGGCAA GAAAtccttg ccgtcggcgg ctCCCGCAGC
   1951  gcgGCGGAAT CCTTCAAAGC CTTCCGCGGA CGCGAACCGA GCATAGACGC
   2001  ACTGCTGCGC CAaagcggtT TCGACAACGC gGCttgA
```

This corresponds to the amino acid sequence <SEQ ID 53; ORF 128.ng>:

```
g128.pep
      1  MIDNALLHLG EEPRFNQIQT EDIKPAVQTA IAEARGQIAA VKAQTHTGWA
     51  NTVERLTGIT ERVGRIWGVV SHLNSVVDTP ELRAVYNELM PEITVFFTEI
    101  GQDIELYNRF KTIKNSPEFA TLSPAQKTKL DHDLRDFVLS GAELPPERQA
    151  ELAKLQTEGA QLSAKFSQNV LDATDAFGIY FDDAAPLAGI PEDALAMFAA
    201  AAQSEGKTGY KIGLQIPHYL AVIQYAGNRE LREQIYRAYV TRASELSNDG

    251  KFDNTANIDR TLENALKTAK LLGFKNYAEL SLATKMADTP EQVLNFLHDL
    301  ARRAKPYAEK DLAEVKAFAR EHLGLADPQP WDLSYAGEKL REAKYAFSET
    351  EVKKYFPVGK VLAGLFAQIK KLYGIGFAEK TVPVWHKDVR YFELQQNGKT
    401  IGGVYMDLYA REGKRGGAWM NDYKGRRRFA DGTLQLPTAY LVCNFAPPVG
    451  GKEARLSHDE ILTLFHETGH GLHHLLTQVD ELGVSGINGV EWDAVELPSQ
    501  FMENFVWEYN VLAQMSAHEE TGEPLPKELF DKMLAAKNFQ RGMFLVRQME
    551  FALFDMMIYS ESDECRLKNW QQVLDSVRKE VAVIQPPEYN RFANSFGHIF
    601  AGGYSAGYYS YAWAEVLSTD AYAAFEESDD VAATGKRFWQ EILAVGGSRS
    651  AAESFKAFRG REPSIDALLR QSGFDNAA*
```

ORF 128 shows 91.7% identity over a 475 aa overlap with a predicted ORF (ORF 128.ng) from *N.gonorrhoeae*:

m128/g128

```
                       10        20        30        40        50        60
g128.pep       MIDNALLHLGEEPRFNQIQTEDIKPAVQTAIAEARGQIAAVKAQTHTGWANTVERLTGIT
               | |||||||||||||:||:||||||:|||||||| ||||:||||||||||||| |||||
m128           MTDNALLHLGEEPRFDQIKTEDIKPALQTAIAEAREQIAAIKAQTHTGWANTVEPLTGIT
                       10        20        30        40        50        60

                       70        80        90       100       110       120
g128.pep       ERVGRIWGVVSHLNSVVDTPELRAVYNELMPEITVFFTEIGQDIELYNRFKTIKNSPEFA
               ||||||||||||||| |:||||||||||||||||||||||||||||||||||||||||||
m128           ERVGRIWGVVSHLNCVADTPELRAVYNELMPEITVFFTEIGQDIELYNRFKTIKNSPEFD
                       70        80        90       100       110       120

                      130       140       150       160       170       180
g128.pep       TLSPAQKTKLDHDLRDFVLSGAELPPERQAELAKLQTEGAQLSAKFSQNVLDATDAFGIY
               ||||||||||:|
m128           TLSPAQKTKLNH
                      130
                              //
                                                    340       350       360
g128.pep                                      YAGEKLREAKYAFSETEVKKYFPVGKVLAG
                                              ||:||||||||||||| |||||||| || |
m128                                          YASEKLREAKYAFSETXVKKYFPVGXVLNG
                                                      10        20        30

                      370       380       390       400       410       420
g128.pep       LFAQIKKLYGIGFAEKTVPVWHKDVRYFELQQNGKTIGGVYMDLYAREGKRGGAWMNDYK
               |||| |||||||:||||||||||||| ||||||::|||||||||||||||||||||||||
m128           LFAQXKKLYGIGFTEKTVPVWHKDVRYXELQQNGEXIGGVYMDLYAREGKRGGAWMNDYK
                       40        50        60        70        80        90

                      430       440       450       460       470       480
g128.pep       GRRRFADGTLQLPTAYLVCNFAPPVGGKEARLSHDEILTLFHETGHGLHHLLTQVDELGV
               |||||:||||||||||||||||||||||:||||||||||| ||||||||||||||||||||
m128           GRRRFSDGTLQLPTAYLVCNFAPPVGGREARLSHDEILILFHETGHGLHHLLTQVDELGV
                      100       110       120       130       140       150

                      490       500       510       520       530       540
g128.pep       SGINGVEWDAVELPSQFMENFVWEYNVLAQMSAHEETGEPLPKELFDKMLAAKNFQRGMF
               |||||| ||||||||||||||||||||||| |||||||| |||||| || ||||||| |||
m128           SGINGVXWDAVELPSQFMENFVWEYNVLAQXSAHEETGVPLPKELXDKXLAAKNFQXGMF
                      160       170       180       190       200       210

                      550       560       570       580       590       600
```

```
g128.pep    LVRQMEFALFDMMIYSESDECRLKNWQQVLDSVRKEVAVIQPPEYNRFANSFGHIFAGGY
            ||| ||||||||||||:|| |||||||||||||:|||||||||||| |||||||||
m128        XVRQXEFALFDMMIYSEDDEGRLKNWQQVLDSVRKKVAVIQPPEYNRFALSFGHIFAGGY
                    220       230       240       250       260       270

                610       620       630       640       650       660
g128.pep    SAGYYSYAWAEVLSTDAYAAFEESDDVAATGKRFWQEILAVGGSRSAAESFKAFRGREPS
            ||: |||||||||:|||||||||||||||||||||||||| |||:|||||||||||||
m128        SAAXYSYAWAEVLSADAYAAFEESDDVAATGKRFWQEILAVGXSRSGAESFKAFRGREPS
                    280       290       300       310       320       330

                670       679
g128.pep    IDALLRQSGFDNAAX
            ||||||:||||||:
m128        IDALLRHSGFDNAVX
                    340
```

The following partial DNA sequence was identified in *N. meningitidis* <SEQ ID 54>:

```
a128.seq
   1  ATGACTGACA ACGCACTGCT CCATTTGGGC GAAGAACCCC GTTTTGATCA
  51  AATCAAAACC GAAGACATCA AACCCGCCCT GCAAACCGCC ATTGCCGAAG
 101  CGCGCGAACA AATCGCCGCC ATCAAAGCCC AAACGCACAC CGGCTGGGCA
 151  AACACTGTCG AACCCCTGAC CGGCATCACC GAACGCGTCG GCAGGATTTG
 201  GGGCGTGGTG TCGCACCTCA ACTCCGTCAC CGACACGCCC GAACTGCGCG
 251  CCGCCTACAA TGAATTAATG CCCGAAATTA CCGTCTTCTT CACCGAAATC
 301  GGACAAGACA TCGAGCTGTA CAACCGCTTC AAAACCATCA AAAACTCCCC
 351  CGAGTTCGAC ACCCTCTCCC ACGCGCAAAA AACCAAACTC AACCACGATC
 401  TGCGCGATTT CGTCCTCAGC GGCGCGGAAC TGCCGCCCGA ACAGCAGGCA
 451  GAATTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCT
 601  GCCGCGCAAA GCGAAGGCAA AACAGGCTAC AAAATCGGTT TGCAGATTCC
 651  GCACTACCTC GCCGTCATCC AATACGCCGA CAACCGCAAA CTGCGCGAAC
 701  AAATCTACCG CGCCTACGTT ACCCGCGCCA GCGAGCTTTC AGACGACGGC
 751  AAATTCGACA ACACCGCCAA CATCGACCGC ACGCTCGAAA ACGCCCTGCA
 801  AACCGCCAAA CTGCTCGGCT TCAAAAACTA CGCCGAATTG TCGCTGGCAA
 851  CCAAAATGGC GGACACCCCC GAACAAGTTT TAAACTTCCT GCACGACCTC
 901  GCCCGCCGCG CCAAACCCTA CGCCGAAAAA GACCTCGCCG AAGTCAAAGC
 951  CTTCGCCCGC GAAAGCCTCG GCCTCGCCGA TTTGCAACCG TGGGACTTGG
1001  GCTACGCCGG CGAAAAACTG CGCGAAGCCA AATACGCATT CAGCGAAACC
1051  GAAGTCAAAA AATACTTCCC CGTCGGCAAA GTATTAAACG GACTGTTCGC
1101  CCAAATCAAA AAACTCTACG GCATCGGATT TACCGAAAAA ACCGTCCCCG
1151  TCTGGCACAA AGACGTGCGC TATTTTGAAT TGCAACAAAA CGGCGAAACC
1201  ATAGGCGGCG TTTATATGGA TTTGTACGCA CGCGAAGGCA AACGCGGCGG
1251  CGCGTGGATG AACGACTACA AAGGCCGCCG CCGTTTTTCA GACGGCACGC
1301  TGCAACTGCC CACCGCCTAC CTCGTCTGCA ACTTCACCCC GCCCGTCGGC
1351  GGCAAAGAAG CCCGCTTGAG CCATGACGAA ATCCTCACCC TCTTCCACGA
1401  AACCGGACAC GGCCTGCACC ACCTGCTTAC CCAAGTCGAC GAACTGGGCG
1451  TATCCGGCAT CAACGGCGTA GAATGGGACG CAGTCGAACT GCCCAGTCAG
1501  TTTATGGAAA ATTTCGTTTG GGAATACAAT GTCTTGGCGC AAATGTCCGC
1551  CCACGAAGAA ACCGGCGTTC CCCTGCCGAA AGAACTCTTC GACAAAATGC
1601  TCGCCGCCAA AAACTTCCAA CGCGGAATGT TCCTCGTCCG CCAAATGGAG
1651  TTCGCCCTCT TTGATATGAT GATTTACAGC GAAGACGACG AAGGCCGTCT
1701  GAAAAACTGG CAACAGGTTT TAGACAGCGT GCGCAAAGAA GTCGCCGTCG
1751  TCCGACCGCC CGAATACAAC CGCTTCGCCA ACAGCTTCGG CCACATCTTC
1801  GCAGGCGGCT ATTCCGCAGG CTATTACAGC TACGCGTGGG CGGAAGTATT
1851  GAGCGCGGAC GCATACGCCG CCTTTGAAGA AAGCGACGAT GTCGCCGCCA
1901  CAGGCAAACG CTTTTGGCAG GAAATCCTCG CCGTCGGCGG ATCGCGCAGC
1951  GCGGCAGAAT CCTTCAAAGC CTTCCGCGGA CGCGAACCGA GCATAGACGC
2001  ACTCTTGCGC CACAGCGGCT TCGACAACGC GGCTTGA
```

This corresponds to the amino acid sequence <SEQ ID 55; ORF 128.a>:

```
a128.pep
       1  MTDNALLHLG EEPRFDQIKT EDIKPALQTA IAEAREQIAA IKAQTHTGWA
      51  NTVEPLTGIT ERVGRIWGVV SHLNSVTDTP ELRAAYNELM PEITVFFTEI
     101  GQDIELYNRF KTIKNSPEFD TLSHAQKTKL NHDLRDFVLS GAELPPEQQA
     151  ELAKLQTEGA QLSAKFSQNV LDATDAFGIY FDDAAPLAGI PEDALAMFAA
     201  AAQSEGKTGY KIGLQIPHYL AVIQYADNRK LREQIYRAYV TRASELSDDG
     251  KFDNTANIDR TLENALQTAK LLGFKNYAEL SLATKMADTP EQVLNFLHDL
     301  ARRAKPYAEK DLAEVKAFAR ESLGLADLQP WDLGYAGEKL REAKYAFSET
     351  EVKKYFPVGK VLNGLFAQIK KLYGIGFTEK TVPVWHKDVR YFELQQNGET
     401  IGGVYMDLYA REGKRGGAWM NDYKGRRRFS DGTLQLPTAY LVCNFTPPVG
     451  GKEARLSHDE ILTLFHETGH GLHHLLTQVD ELGVSGINGV EWDAVELPSQ
     501  FMENFVWEYN VLAQMSAHEE TGVPLPKELF DKMLAAKNFQ RGMFLVRQME
     551  FALFDMMIYS EDDEGRLKNW QQVLDSVRKE VAVVRPPEYN RFANSFGHIF
     601  AGGYSAGYYS YAWAEVLSAD AYAAFEESDD VAATGKRFWQ EILAVGGSRS
     651  AAESFKAFRG REPSIDALLR HSGFDNAA*
```

m128/a128 ORFs 128 and 128.a showed a 66.0% identity in 677 aa overlap

```
                          10        20        30        40        50        60
m128.pep         MTDNALLHLGEEPRFDQIKTEDIKPALQTAIAEAREQIAAIKAQTHTGWANTVEPLTGIT
                 ||||||||||||||||||||||||||||||||||||||||||||||||||||||||||||
a128             MTDNALLHLGEEPRFDQIKTEDIKPALQTAIAEAREQIAAIKAQTHTGWANTVEPLTGIT
                          10        20        30        40        50        60

                          70        80        90       100       110       120
m128.pep         ERVGRIWGVVSHLNCVADTPELRAVYNELMPEITVFFTEIGQDIELYNRFKTIKNSPEFD
                 |||||||||||||| |:|||||||:|||||||||||||||||||||||||||||||||||
a128             ERVGRIWGVVSHLNSVTDTPELRAAYNELMPEITVFFTEIGQDIELYNRFKTIKNSPEFD
                          70        80        90       100       110       120

                         130
m128.pep         TLSPAQKTKLNH------------------------------------------------
                 ||| ||||||||
a128             TLSHAQKTKLNHDLRDFVLSGAELPPEQQAELAKLQTEGAQLSAKFSQNVLDATDAFGIY
                         130       140       150       160       170       180

m128.pep         ------------------------------------------------------------

a128             FDDAAPLAGIPEDALAMFAAAAQSEGKTGYKIGLQIPHYLAVIQYADNRKLREQIYRAYV
                         190       200       210       220       230       240

m128.pep         ------------------------------------------------------------

a128             TRASELSDDGKFDNTANIDRTLENALQTAKLLGFKNYAELSLATKMADTPEQVLNFLHDL
                         250       260       270       280       290       300

                                                         140       150
m128.pep         ----------------------------------YASEKLREAKYAFSETXVKKYFPVGX
                                                   ||:||||||||||||| |||||||| 
a128             ARRAKPYAEKDLAEVKAFARESLGLADLQPWDLGYAGEKLREAKYAFSETEVKKYFPVGK
                         310       320       330       340       350       360

                160       170       180       190       200       210
m128.pep         VLNGLFAQXKKLYGIGFTEKTVPVWHKDVRYXELQQNGEXIGGVYMDLYAREGKRGGAWM
                 |||||||| |||||||||||||||||||||| |||||||:||||||||||||||||||||
a128             VLNGLFAQIKKLYGIGFTEKTVPVWHKDVRYFELQQNGETIGGVYMDLYAREGKRGGAWM
                         370       380       390       400       410       420

                220       230       240       250       260       270
m128.pep         NDYKGRRRFSDGTLQLPTAYLVCNFAPPVGGREARLSHDEILILFHETGHGLHHLLTQVD
```

```
               ||||||||||||||||||||||||||:|||||:|||||||||| ||||||||||||||||
a128           NDYKGRRRFSDGTLQLPTAYLVCNFTPPVGGKEARLSHDEILTLFHETGHGLHHLLTQVD
                       430       440       450       460       470       480

              280       290       300       310       320       330
m128.pep       ELGVSGINGVXWDAVELPSQFMENFVWEYNVLAQXSAHEETGVPLPKELXDKXLAAKNFQ
               |||||||||| |||||||||||||||||||||||| |||||||||||||| || |||||||
a128           ELGVSGINGVEWDAVELPSQFMENFVWEYNVLAQMSAHEETGVPLPKELFDKMLAAKNFQ
                       490       500       510       520       530       540

              340       350       360       370       380       390
m128.pep       XGMFXVRQXEFALFDMMIYSEDDEGRLKNWQQVLDSVRKKVAVIQPPEYNRFALSFGHIF
                ||| ||| ||||||||||||||||||||||||||||:|||::|||||||| ||||||
a128           RGMFLVRQMEFALFDMMIYSEDDEGRLKNWQQVLDSVRKEVAVVRPPEYNRFANSFGHIF
                       550       560       570       580       590       600

              400       410       420       430       440       450
m128.pep       AGGYSAAXYSYAWAEVLSADAYAAFEESDDVAATGKRFWQEILAVGXSRSGAESFKAFRG
               ||||||: |||||||||||||||||||||||||||||||||||||| |||:|||||||||
a128           AGGYSAGYYSYAWAEVLSADAYAAFEESDDVAATGKRFWQEILAVGGSRSAAESFKAFRG
                       610       620       630       640       650       660

              460       470
m128.pep       REPSIDALLRHSGFDNAVX
               |||||||||||||||||:
a128           REPSIDALLRHSGFDNAAX
                       670
```

Further work revealed the DNA sequence identified in *N. meningitidis* <SEQ ID 56>:

```
m128-1.seq
      1  ATGACTGACA ACGCACTGCT CCATTTGGGC GAAGAACCCC GTTTTGATCA
     51  AATCAAAACC GAAGACATCA AACCCGCCCT GCAAACCGCC ATCGCCGAAG
    101  CGCGCGAACA AATCGCCGCC ATCAAAGCCC AAACGCACAC CGGCTGGGCA
    151  AACACTGTCG AACCCCTGAC CGGCATCACC GAACGCGTCG GCAGGATTTG
    201  GGGCGTGGTG TCGCACCTCA ACTCCGTCGC CGACACGCCC GAACTGCGCG
    251  CCGTCTATAA CGAACTGATG CCCGAAATCA CCGTCTTCTT CACCGAAATC
    301  GGACAAGACA TCGAGCTGTA CAACCGCTTC AAAACCATCA AAAATTCCCC
    351  CGAATTCGAC ACCCTCTCCC CCGCACAAAA AACCAAACTC AACCACGATC
    401  TGCGCGATTT CGTCCTCAGC GGCGCGGAAC TGCCGCCCGA ACAGCAGGCA
    451  GAACTGGCAA AACTGCAAAC CGAAGGCGCG CAACTTTCCG CCAAATTCTC
    501  CCAAAACGTC CTAGACGCGA CCGACGCGTT CGGCATTTAC TTTGACGATG
    551  CCGCACCGCT TGCCGGCATT CCCGAAGACG CGCTCGCCAT GTTTGCCGCC
    601  GCCGCGCAAA GCGAAAGCAA AACAGGCTAC AAAATCGGCT TGCAGATTCC
    651  ACACTACCTC GCCGTCATCC AATACGCCGA CAACCGCGAA CTGCGCGAAC
    701  AAATCTACCG CGCCTACGTT ACCCGCGCCA GCGAACTTTC AGACGACGGC
    751  AAATTCGACA ACACCGCCAA CATCGACCGC ACGCTCGCAA ACGCCCTGCA
    801  AACCGCCAAA CTGCTCGGCT TCAAAAACTA CGCCGAATTG TCGCTGGCAA
    851  CCAAAATGGC GGACACGCCC GAACAAGTTT TAAACTTCCT GCACGACCTC
    901  GCCCGCCGCG CCAAACCCTA CGCCGAAAAA GACCTCGCCG AAGTCAAAGC
    951  CTTCGCCCGC GAAAGCCTGA ACCTCGCCGA TTTGCAACCG TGGGACTTGG
   1001  GCTACGCCAG CGAAAAACTG CGCGAAGCCA AATACGCGTT CAGCGAAACC
   1051  GAAGTCAAAA AATACTTCCC CGTCGGCAAA GTATTAAACG GACTGTTCGC
   1101  CCAAATCAAA AAACTCTACG GCATCGGATT TACCGAAAAA ACCGTCCCCG
   1151  TCTGGCACAA AGACGTGCGC TATTTTGAAT TGCAACAAAA CGGCGAAACC
   1201  ATAGGCGGCG TTTATATGGA TTTGTACGCA CGCGAAGGCA AACGCGGCGG
   1251  CGCGTGGATG AACGACTACA AAGGCCGCCG CCGTTTTTCA GACGGCACGC
   1301  TGCAACTGCC CACCGCCTAC CTCGTCTGCA ACTTCGCCCC ACCCGTCGGC
   1351  GGCAGGGAAG CCCGCCTGAG CCACGACGAA ATCCTCATCC TCTTCCACGA
   1401  AACCGGACAC GGGCTGCACC ACCTGCTTAC CCAAGTGGAC GAACTGGGCG
```

```
1451  TATCCGGCAT CAACGGCGTA GAATGGGACG CGGTCGAACT GCCCAGCCAG
1501  TTTATGGAAA ATTTCGTTTG GGAATACAAT GTCTTGGCAC AAATGTCAGC
1551  CCACGAAGAA ACCGGCGTTC CCCTGCCGAA AGAACTCTTC GACAAAATGC
1601  TCGCCGCCAA AAACTTCCAA CGCGGCATGT TCCTCGTCCG GCAAATGGAG
1651  TTCGCCCTCT TTGATATGAT GATTTACAGC GAAGACGACG AAGGCCGTCT
1701  GAAAAACTGG CAACAGGTTT TAGACAGCGT GCGCAAAAAA GTCGCCGTCA
1751  TCCAGCCGCC CGAATACAAC CGCTTCGCCT TGAGCTTCGG CCACATCTTC
1801  GCAGGCGGCT ATTCCGCAGG CTATTACAGC TACGCGTGGG CGGAAGTATT
1851  GAGCGCGGAC GCATACGCCG CCTTTGAAGA AAGCGACGAT GTCGCCGCCA
1901  CAGGCAAACG CTTTTGGCAG GAAATCCTCG CCGTCGGCGG ATCGCGCAGC
1951  GCGGCAGAAT CCTTCAAAGC CTTCCGCGGC CGCGAACCGA GCATAGACGC
2001  ACTCTTGCGC CACAGCGGTT TCGACAACGC GGTCTGA
```

This corresponds to the amino acid sequence <SEQ ID 57; ORF 128-1>:

```
m128-1.pep.
   1  MTDNALLHLG EEPRFDQIKT EDIKPALQTA IAEAREQIAA IKAQTHTGWA
  51  NTVEPLTGIT ERVGRIWGVV SHLNSVADTP ELRAVYNELM PEITVFFTEI
 101  GQDIELYNRF KTIKNSPEFD TLSPAQKTKL NHDLRDFVLS GAELPPEQQA
 151  ELAKLQTEGA QLSAKFSQNV LDATDAFGIY FDDAAPLAGI PEDALAMFAA
 201  AAQSESKTGY KIGLQIPHYL AVIQYADNRE LREQIYRAYV TRASELSDDG
 251  KFDNTANIDR TLANALQTAK LLGFKNYAEL SLATKMADTP EQVLNFLHDL
 301  ARRAKPYAEK DLAEVKAFAR ESLNLADLQP WDLGYASEKL REAKYAFSET
 351  EVKKYFPVGK VLNGLFAQIK KLYGIGFTEK TVPVWHKDVR YFELQQNGET
 401  IGGVYMDLYA REGKRGGAWM NDYKGRRRFS DGTLQLPTAY LVCNFAPPVG
 451  GREARLSHDE ILILFHETGH GLHHLLTQVD ELGVSGINGV EWDAVELPSQ
 501  FMENFVWEYN VLAQMSAHEE TGVPLPKELF DKMLAAKNFQ RGMFLVRQME
 551  FALFDMMIYS EDDEGRLKNW QQVLDSVRKK VAVIQPPEYN RFALSFGHIF
 601  AGGYSAGYYS YAWAEVLSAD AYAAFEESDD VAATGKRFWQ EILAVGGSRS
 651  AAESFKAFRG REPSIDALLR HSGFDNAV*
```

The following partial DNA sequence was identified in *N. gonorrhoeae* <SEQ ID 58>:

```
g128-1.seq  (partial)
   1  ATGATTGACA ACGCACTGCT CCACTTGGGC GAAGAACCCC GTTTTAATCA
  51  AATCAAAACC GAAGACATCA AACCCGCCGT CCAAACCGCC ATCGCCGAAG
 101  CGCGCGGACA AATCGCCGCC GTCAAAGCGC AAACGCACAC CGGCTGGGCG
 151  AACACCGTCG AGCGTCTGAC CGGCATCACC GAACGCGTCG GCAGGATTTG
 201  GGGCGTCGTG TCCCATCTCA ACTCCGTCGT CGACACGCCC GAACTGCGCG
 251  CCGTCTATAA CGAACTGATG CCTGAAATCA CCGTCTTCTT CACCGAAATC
 301  GGACAAGACA TCGAACTGTA CAACCGCTTC AAAACCATCA AAAATTCCCC
 351  CGAATTTGCA ACGCTTTCCC CCGCACAAAA AACCAAGCTC GATCACGACC
 401  TGCGCGATTT CGTATTGAGC GGCGCGGAAC TGCCGCCCGA ACGGCAGGCA
 451  GAACTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCC
 601  GCCGCGCAAA GCGAAGGCAA AACAGGTTAC AAAATCGGCT TGCAGATTCC
 651  GCACTACCTT GCCGTTATCC AATACGCCGG CAACCGCGAA CTGCGCGAAC
 701  AAATCTACCG CGCCTACGTT ACCCGTGCCA GCGAACTTTC AAACGACGGC
 751  AAATTCGACA ACACCGCCAA CATCGACCGC ACGCTCGAAA ACGCATTGAA
 801  AACCGCCAAA CTGCTCGGCT TTAAAAATTA CGCCGAATTG TCGCTGGCAA
 851  CCAAAATGGC GGACACGCCC GAACAGGTTT TAAACTTCCT GCACGACCTC
 901  GCCCGCCGCG CCAAACCCTA CGCCGAAAAA GACCTCGCCG AAGTCAAAGC
 951  CTTCGCCCGC GAACACCTCG GTCTCGCCGA CCCGCAGCCG TGGGACTTGA
1001  GCTACGCCGG CGAAAAACTG CGCGAAGCCA AATACGCATT CAGCGAAACC
1051  GAAGTCAAAA AATACTTCCC CGTCGGCAAA GTTCTGGCAG GCCTGTTCGC
1101  CCAAATCAAA AAACTCTACG GCATCGGATT CGCCGAAAAA ACCGTTCCCG
1151  TCTGGCACAA AGACGTGCGC TATTTTGAAT TGCAACAAAA CGGCAAAACC
1201  ATCGGCGGCG TTTATATGGA TTTGTACGCA CGCGAAGGCA AACGCGGCGG
1251  CGCGTGGATG AACGACTACA AAGGCCGCCG CCGCTTTGCC GACGGCACGC
1301  TGCAACTGCC CACCGCCTAC CTCGTCTGCA ACTTCGCCCC GCCCGTCGGC
1351  GGCAAAGAAG CGCGTTTAAG CCACGACGAA ATCCTCACCC TCTTCCACGA
1401  AACCGGCCAC GGACTGCACC ACCTGCTTAC CCAAGTGGAC GAACTGGGCG
1451  TGTCCGGCAT CAACGGCGTA AAA
```

This corresponds to the amino acid sequence <SEQ ID 59; ORF 128-1.ng>:

```
g128-1.pep  (partial)
      1  MIDNALLHLG EEPRFNQIKT EDIKPAVQTA IAEARGQIAA VKAQTHTGWA
     51  NTVERLTGIT ERVGRIWGVV SHLNSVVDTP ELRAVYNELM PEITVFFTEI
    101  GQDIELYNRF KTIKNSPEFA TLSPAQKTKL DHDLRDFVLS GAELPPERQA
    151  ELAKLQTEGA QLSAKFSQNV LDATDAFGIY FDDAAPLAGI PEDALAMFAA
    201  AAQSEGKTGY KIGLQIPHYL AVIQYAGNRE LREQIYRAYV TRASELSNDG
    251  KFDNTANIDR TLENALKTAK LLGFKNYAEL SLATKMADTP EQVLNFLHDL
    301  ARRAKPYAEK DLAEVKAFAR EHLGLADPQP WDLSYAGEKL REAKYAFSET
    351  EVKKYFPVGK VLAGLFAQIK KLYGIGFAEK TVPVWHKDVR YFELQQNGKT
    401  IGGVYMDLYA REGKRGGAWM NDYKGRRRFA DGTLQLPTAY LVCNFAPPVG
    451  GKEARLSHDE ILTLFHETGH GLHHLLTQVD ELGVSGINGV K
```

**m128-1/g128-1** ORFs 128-1 and 128-1.ng showed a 94.5% identity in 491 aa overlap

```
                      10        20        30        40        50        60
g128-1.pep  MIDNALLHLGEEPRFNQIKTEDIKPAVQTAIAEARGQIAAVKAQTHTGWANTVERLTGIT
            | |||||||||||||:||||||||||:|||||||| ||||:|||||||||||||| |||||
m128-1      MTDNALLHLGEEPRFDQIKTEDIKPALQTAIAEAREQIAAIKAQTHTGWANTVEPLTGIT
                      10        20        30        40        50        60

                      70        80        90       100       110       120
g128-1.pep  ERVGRIWGVVSHLNSVVDTPELRAVYNELMPEITVFFTEIGQDIELYNRFKTIKNSPEFA
            ||||||||||||||||:|||||||||||||||||||||||||||||||||||||||||| 
m128-1      ERVGRIWGVVSHLNSVADTPELRAVYNELMPEITVFFTEIGQDIELYNRFKTIKNSPEFD
                      70        80        90       100       110       120

                     130       140       150       160       170       180
g128-1.pep  TLSPAQKTKLDHDLRDFVLSGAELPPERQAELAKLQTEGAQLSAKFSQNVLDATDAFGIY
            ||||||||||:||||||||||||||||:||||||||||||||||||||||||||||||||
m128-1      TLSPAQKTKLNHDLRDFVLSGAELPPEQQAELAKLQTEGAQLSAKFSQNVLDATDAFGIY
                     130       140       150       160       170       180

                     190       200       210       220       230       240
g128-1.pep  FDDAAPLAGIPEDALAMFAAAAQSEGKTGYKIGLQIPHYLAVIQYAGNRELREQIYRAYV
            |||||||||||||||||||||||||:|||||||||||||||||||| |||||||||||||
m128-1      FDDAAPLAGIPEDALAMFAAAAQSESKTGYKIGLQIPHYLAVIQYADNRELREQIYRAYV
                     190       200       210       220       230       240

                     250       260       270       280       290       300
g128-1.pep  TRASELSNDGKFDNTANIDRTLENALKTAKLLGFKNYAELSLATKMADTPEQVLNFLHDL
            |||||||:|||||||||||||| |||:|||||||||||||||||||||||||||||||||
m128-1      TRASELSDDGKFDNTANIDRTLANALQTAKLLGFKNYAELSLATKMADTPEQVLNFLHDL
                     250       260       270       280       290       300

                     310       320       330       340       350       360
g128-1.pep  ARRAKPYAEKDLAEVKAFAREHLGLADPQPWDLSYAGEKLREAKYAFSETEVKKYFPVGK
            ||||||||||||||||||||| |:||| |||||:||:|||||||||||||||||||||||
m128-1      ARRAKPYAEKDLAEVKAFARESLNLADLQPWDLGYASEKLREAKYAFSETEVKKYFPVGK
                     310       320       330       340       350       360

                     370       380       390       400       410       420
g128-1.pep  VLAGLFAQIKKLYGIGFAEKTVPVWHKDVRYFELQQNGKTIGGVYMDLYAREGKRGGAWM
            || ||||||||||||||:|||||||||||||||||||||:||||||||||||||||||||
m128-1      VLNGLFAQIKKLYGIGFTEKTVPVWHKDVRYFELQQNGETIGGVYMDLYAREGKRGGAWM
                     370       380       390       400       410       420

                     430       440       450       460       470       480
g128-1.pep  NDYKGRRRFADGTLQLPTAYLVCNFAPPVGGKEARLSHDEILTLFHETGHGLHHLLTQVD
```

```
            ||||||||:|||||||||||||||||||||:|||||||||| ||||||||||||||||
m128-1      NDYKGRRRFSDGTLQLPTAYLVCNFAPPVGGREARLSHDEILILFHETGHGLHHLLTQVD
                    430       440       450       460       470       480

                    490
g128-1.pep  ELGVSGINGVK
            ||||||||||:
m128-1      ELGVSGINGVEWDAVELPSQFMENFVWEYNVLAQMSAHEETGVPLPKELFDKMLAAKNFQ
                    490       500       510       520       530       540
```

The following DNA sequence was identified in *N. meningitidis* <SEQ ID 60>:

```
a128-1.seq
   1 ATGACTGACA ACGCACTGCT CCATTTGGGC GAAGAACCCC GTTTTGATCA
  51 AATCAAAACC GAAGACATCA AACCCGCCCT GCAAACCGCC ATTGCCGAAG
 101 CGCGCGAACA AATCGCCGCC ATCAAAGCCC AAACGCACAC CGGCTGGGCA
 151 AACACTGTCG AACCCCTGAC CGGCATCACC GAACGCGTCG GCAGGATTTG
 201 GGGCGTGGTG TCGCACCTCA ACTCCGTCAC CGACACGCCC GAACTGCGCG
 251 CCGCCTACAA TGAATTAATG CCCGAAATTA CCGTCTTCTT CACCGAAATC
 301 GGACAAGACA TCGAGCTGTA CAACCGCTTC AAAACCATCA AAAACTCCCC
 351 CGAGTTCGAC ACCCTCTCCC ACGCGCAAAA AACCAAACTC AACCACGATC
 401 TGCGCGATTT CGTCCTCAGC GGCGCGGAAC TGCCGCCCGA ACAGCAGGCA
 451 GAATTGGCAA AACTGCAAAC CGAAGGCGCG CAACTTTCCG CCAAATTCTC
 501 CCAAAACGTC CTAGACGCGA CCGACGCGTT CGGCATTTAC TTTGACGATG
 551 CCGCACCGCT TGCCGGCATT CCCGAAGACG CGCTCGCCAT GTTTGCCGCT
 601 GCCGCGCAAA GCGAAGGCAA AACAGGCTAC AAAATCGGTT TGCAGATTCC
 651 GCACTACCTC GCCGTCATCC AATACGCCGA CAACCGCAAA CTGCGCGAAC
 701 AAATCTACCG CGCCTACGTT ACCCGCGCCA GCGAGCTTTC AGACGACGGC
 751 AAATTCGACA ACACCGCCAA CATCGACCGC ACGCTCGAAA ACGCCCTGCA
 801 AACCGCCAAA CTGCTCGGCT TCAAAAACTA CGCCGAATTG TCGCTGGCAA
 851 CCAAAATGGC GGACACCCCC GAACAAGTTT TAAACTTCCT GCACGACCTC
 901 GCCCGCCGCG CCAAACCCTA CGCCGAAAAA GACCTCGCCG AAGTCAAAGC
 951 CTTCGCCCGC GAAAGCCTCG GCCTCGCCGA TTTGCAACCG TGGGACTTGG
1001 GCTACGCCGG CGAAAAACTG CGCGAAGCCA AATACGCATT CAGCGAAACC
1051 GAAGTCAAAA AATACTTCCC CGTCGGCAAA GTATTAAACG GACTGTTCGC
1101 CCAAATCAAA AAACTCTACG GCATCGGATT TACCGAAAAA ACCGTCCCCG
1151 TCTGGCACAA AGACGTGCGC TATTTTGAAT TGCAACAAAA CGGCGAAACC
1201 ATAGGCGGCG TTTATATGGA TTTGTACGCA CGCGAAGGCA AACGCGGCGG
1251 CGCGTGGATG AACGACTACA AAGGCCGCCG CCGTTTTTCA GACGGCACGC
1301 TGCAACTGCC CACCGCCTAC CTCGTCTGCA ACTTCACCCC GCCCGTCGGC
1351 GGCAAAGAAG CCCGCTTGAG CCATGACGAA ATCCTCACCC TCTTCCACGA
1401 AACCGGACAC GGCCTGCACC ACCTGCTTAC CCAAGTCGAC GAACTGGGCG
1451 TATCCGGCAT CAACGGCGTA GAATGGGACG CAGTCGAACT GCCCAGTCAG
1501 TTTATGGAAA ATTTCGTTTG GGAATACAAT GTCTTGGCGC AAATGTCCGC
1551 CCACGAAGAA ACCGGCGTTC CCCTGCCGAA AGAACTCTTC GACAAAATGC
1601 TCGCCGCCAA AAACTTCCAA CGCGGAATGT TCCTCGTCCG CCAAATGGAG
1651 TTCGCCCTCT TTGATATGAT GATTTACAGC GAAGACGACG AAGGCCGTCT
1701 GAAAAACTGG CAACAGGTTT TAGACAGCGT GCGCAAAGAA GTCGCCGTCG
1751 TCCGACCGCC CGAATACAAC CGCTTCGCCA ACAGCTTCGG CCACATCTTC
1801 GCAGGCGGCT ATTCCGCAGG CTATTACAGC TACGCGTGGG CGGAAGTATT
1851 GAGCGCGGAC GCATACGCCG CCTTTGAAGA AAGCGACGAT GTCGCCGCCA
1901 CAGGCAAACG CTTTTGGCAG GAAATCCTCG CCGTCGGCGG ATCGCGCAGC
1951 GCGGCAGAAT CCTTCAAAGC CTTCCGCGGA CGCGAACCGA GCATAGACGC
2001 ACTCTTGCGC CACAGCGGCT TCGACAACGC GGCTTGA
```

This corresponds to the amino acid sequence <SEQ ID 61; ORF 128-1.a>:

a128-1.pep

```
  1  MTDNALLHLG EEPRFDQIKT EDIKPALQTA IAEAREQIAA IKAQTHTGWA
 51  NTVEPLTGIT ERVGRIWGVV SHLNSVTDTP ELRAAYNELM PEITVFFTEI
101  GQDIELYNRF KTIKNSPEFD TLSHAQKTKL NHDLRDFVLS GAELPPEQQA
151  ELAKLQTEGA QLSAKFSQNV LDATDAFGIY FDDAAPLAGI PEDALAMFAA
201  AAQSEGKTGY KIGLQIPHYL AVIQYADNRK LREQIYRAYV TRASELSDDG
251  KFDNTANIDR TLENALQTAK LLGFKNYAEL SLATKMADTP EQVLNFLHDL
```

```
301  ARRAKPYAEK DLAEVKAFAR ESLGLADLQP WDLGYAGEKL REAKYAFSET
351  EVKKYFPVGK VLNGLFAQIK KLYGIGFTEK TVPVWHKDVR YFELQQNGET
401  IGGVYMDLYA REGKRGGAWM NDYKGRRRFS DGTLQLPTAY LVCNFTPPVG
451  GKEARLSHDE ILTLFHETGH GLHHLLTQVD ELGVSGINGV EWDAVELPSQ
501  FMENFVWEYN VLAQMSAHEE TGVPLPKELF DKMLAAKNFQ RGMFLVRQME
551  FALFDMMIYS EDDEGRLKNW QQVLDSVRKE VAVVRPPEYN RFANSFGHIF
601  AGGYSAGYYS YAWAEVLSAD AYAAFEESDD VAATGKRFWQ EILAVGGSRS
651  AAESFKAFRG REPSIDALLR HSGFDNAA*
```

**m128-1/a128-1** ORFs 128-1 and 128-1.a showed a 97.8% identity in 677 aa overlap

```
                    10        20        30        40        50        60
a128-1.pep  MTDNALLHLGEEPRFDQIKTEDIKPALQTAIAEAREQIAAIKAQTHTGWANTVEPLTGIT
            ||||||||||||||||||||||||||||||||||||||||||||||||||||||||||||
m128-1      MTDNALLHLGEEPRFDQIKTEDIKPALQTAIAEAREQIAAIKAQTHTGWANTVEPLTGIT
                    10        20        30        40        50        60

                    70        80        90       100       110       120
a128-1.pep  ERVGRIWGVVSHLNSVTDTPELRAAYNELMPEITVFFTEIGQDIELYNRFKTIKNSPEFD
            ||||||||||||||||:||||||:||||||||||||||||||||||||||||||||||||
m128-1      ERVGRIWGVVSHLNSVADTPELRAVYNELMPEITVFFTEIGQDIELYNRFKTIKNSPEFD
                    70        80        90       100       110       120

                   130       140       150       160       170       180
a128-1.pep  TLSHAQKTKLNHDLRDFVLSGAELPPEQQAELAKLQTEGAQLSAKFSQNVLDATDAFGIY
            ||| ||||||||||||||||||||||||||||||||||||||||||||||||||||||||
m128-1      TLSPAQKTKLNHDLRDFVLSGAELPPEQQAELAKLQTEGAQLSAKFSQNVLDATDAFGIY
                   130       140       150       160       170       180

                   190       200       210       220       230       240
a128-1.pep  FDDAAPLAGIPEDALAMFAAAAQSEGKTGYKIGLQIPHYLAVIQYADNRKLREQIYRAYV
            ||||||||||||||||||||||||||:|||||||||||||||||||||||:|||||||||
m128-1      FDDAAPLAGIPEDALAMFAAAAQSESKTGYKIGLQIPHYLAVIQYADNRELREQIYRAYV
                   190       200       210       220       230       240

                   250       260       270       280       290       300
a128-1.pep  TRASELSDDGKFDNTANIDRTLENALQTAKLLGFKNYAELSLATKMADTPEQVLNFLHDL
            |||||||||||||||||||||| |||||||||||||||||||||||||||||||||||||
m128-1      TRASELSDDGKFDNTANIDRTLANALQTAKLLGFKNYAELSLATKMADTPEQVLNFLHDL
                   250       260       270       280       290       300

                   310       320       330       340       350       360
a128-1.pep  ARRAKPYAEKDLAEVKAFARESLGLADLQPWDLGYAGEKLREAKYAFSETEVKKYFPVGK
            ||||||||||||||||||||||:|||||||||||||:|||||||||||||||||||||||
m128-1      ARRAKPYAEKDLAEVKAFARESLNLADLQPWDLGYASEKLREAKYAFSETEVKKYFPVGK
                   310       320       330       340       350       360

                   370       380       390       400       410       420
a128-1.pep  VLNGLFAQIKKLYGIGFTEKTVPVWHKDVRYFELQQNGETIGGVYMDLYAREGKRGGAWM
            ||||||||||||||||||||||||||||||||||||||||||||||||||||||||||||
m128-1      VLNGLFAQIKKLYGIGFTEKTVPVWHKDVRYFELQQNGETIGGVYMDLYAREGKRGGAWM
                   370       380       390       400       410       420

                   430       440       450       460       470       480
a128-1.pep  NDYKGRRRFSDGTLQLPTAYLVCNFTPPVGGKEARLSHDEILTLFHETGHGLHHLLTQVD
            |||||||||||||||||||||||||:|||||:|||||||||| |||||||||||||||||
m128-1      NDYKGRRRFSDGTLQLPTAYLVCNFAPPVGGREARLSHDEILILFHETGHGLHHLLTQVD
                   430       440       450       460       470       480

                   490       500       510       520       530       540
a128-1.pep  ELGVSGINGVEWDAVELPSQFMENFVWEYNVLAQMSAHEETGVPLPKELFDKMLAAKNFQ
            ||||||||||||||||||||||||||||||||||||||||||||||||||||||||||||
```

```
m128-1       ELGVSGINGVEWDAVELPSQFMENFVWEYNVLAQMSAHEETGVPLPKELFDKMLAAKNFQ
                     490       500       510       520       530       540

                     550       560       570       580       590       600
a128-1.pep   RGMFLVRQMEFALFDMMIYSEDDEGRLKNWQQVLDSVRKEVAVVRPPEYNRFANSFGHIF
             ||||||||||||||||||||||||||||||||||||||||:|||::|||||||| ||||||
m128-1       RGMFLVRQMEFALFDMMIYSEDDEGRLKNWQQVLDSVRKKVAVIQPPEYNRFALSFGHIF
                     550       560       570       580       590       600

                     610       620       630       640       650       660
a128-1.pep   AGGYSAGYYSYAWAEVLSADAYAAFEESDDVAATGKRFWQEILAVGGSRSAAESFKAFRG
             ||||||||||||||||||||||||||||||||||||||||||||||||||||||||||||
m128-1       AGGYSAGYYSYAWAEVLSADAYAAFEESDDVAATGKRFWQEILAVGGSRSAAESFKAFRG
                     610       620       630       640       650       660

                     670      679
a128-1.pep   REPSIDALLRHSGFDNAAX
             |||||||||||||||||:
m128-1       REPSIDALLRHSGFDNAVX
                     670
```

## 206

The following partial DNA sequence was identified in *N. meningitidis* <SEQ ID 62>:

```
m206.seq
     1  ATGTTTCCCC CCGACAAAAC CCTTTTCCTC TGTCTCAGCG CACTGCTCCT
    51  CGCCTCATGC GGCACGACCT CCGGCAAACA CCGCCAACCG AAACCCAAAC
   101  AGACAGTCCG GCAAATCCAA GCCGTCCGCA TCAGCCACAT CGACCGCACA
   151  CAAGGCTCGC AGGAACTCAT GCTCCACAGC CTCGGACTCA TCGGCACGCC
   201  CTACAAATGG GGCGGCAGCA GCACCGCAAC CGGCTTCGAT TGCAGCGGCA
   251  TGATTCAATT CGTTTACAAr AACGCCCTCA ACGTCAAGCT GCCGCGCACC
   301  GCCCGCGACA TGGCGGCGGC AAGCCGsAAA ATCCCCGAcA GCCGCyTCAA
   351  GGCCGGCGAC CTCGTATTCT TCAACACCGG CGGCGCACAC CGCTACTCAC
   401  ACGTCGGACT CTACATCGGC AACGGCGAAT TCATCCATGC CCCCAGCAGC
   451  GGCAAAACCA TCAAAACCGA AAAACTCTCC ACACCGTTTT ACGCCAAAAA
   501  CTACCTCGGC GCACATACTT TTTTTACAGA ATGA
```

This corresponds to the amino acid sequence <SEQ ID 63; ORF 206>:

```
m206.pep..
     1  MFPPDKTLFL CLSALLLASC GTTSGKHRQP KPKQTVRQIQ AVRISHIDRT
    51  QGSQELMLHS LGLIGTPYKW GGSSTATGFD CSGMIQFVYK NALNVKLPRT
   101  ARDMAAASRK IPDSRXKAGD LVFFNTGGAH RYSHVGLYIG NGEFIHAPSS
   151  GKTIKTEKLS TPFYAKNYLG AHTFFTE*
```

The following partial DNA sequence was identified in *N. gonorrhoeae* <SEQ ID 64>:

```
g206.seq
      1  atgttttccc ccgacaaaac ccttttcctc tgtctcggcg cactgctcct
     51  cgcctcatgc ggcacgacct ccggcaaaca ccgccaaccg aaacccaaac
    101  agacagtccg gcaaatccaa gccgtccgca tcagccacat cggccgcaca
    151  caaggctcgc aggaactcat gctccacagc ctcggactca tcggcacgcc
    201  ctacaaatgg ggcggcagca gcaccgcaac cggcttcgac tgcagcggca
    251  tgattcaatt ggtttacaaa aacgccctca acgtcaagct gccgcgcacc
    301  gcccgcgaca tggcggcggc aagccgcaaa atccccgaca gccgcctcaa
    351  ggccggcgac atcgtattct tcaacaccgg cggcgcacac cgctactcac
    401  acgtcggact ctacatcggc aacggcgaat tcatccatgc ccccggcagc
    451  ggcaaaacca tcaaaaccga aaaactctcc acaccgtttt acgccaaaaa
    501  ctaccttgga gcgcatacgt tttttacaga atga
```

This corresponds to the amino acid sequence <SEQ ID 65; ORF 206.ng>:

```
g206.pep
      1  MFSPDKTLFL CLGALLLASC GTTSGKHRQP KPKQTVRQIQ AVRISHIGRT
     51  QGSQELMLHS LGLIGTPYKW GGSSTATGFD CSGMIQLVYK NALNVKLPRT
    101  ARDMAAASRK IPDSRLKAGD IVFFNTGGAH RYSHVGLYIG NGEFIHAPGS
    151  GKTIKTEKLS TPFYAKNYLG AHTFFTE*
```

ORF 206 shows 96.0% identity over a 177 aa overlap with a predicted ORF (ORF 206.ng) from *N. gonorrhoeae*:

```
m206/g206

                      10        20        30        40        50        60
m206.pep    MFPPDKTLFLCLSALLLASCGTTSGKHRQPKPKQTVRQIQAVRISHIDRTQGSQELMLHS
            || |||||||||:||||||||||||||||||||||||||||||||||| ||||||||||||
g206        MFSPDKTLFLCLGALLLASCGTTSGKHRQPKPKQTVRQIQAVRISHIGRTQGSQELMLHS
                      10        20        30        40        50        60

                      70        80        90       100       110       120
m206.pep    LGLIGTPYKWGGSSTATGFDCSGMIQFVYKNALNVKLPRTARDMAAASRKIPDSRXKAGD
            ||||||||||||||||||||||||||:||||||||||||||||||||||||||||| ||||
g206        LGLIGTPYKWGGSSTATGFDCSGMIQLVYKNALNVKLPRTARDMAAASRKIPDSRLKAGD
                      70        80        90       100       110       120

                     130       140       150       160       170
m206.pep    LVFFNTGGAHRYSHVGLYIGNGEFIHAPSSGKTIKTEKLSTPFYAKNYLGAHTFFTEX
            :|||||||||||||||||||||||||||:|||||||||||||||||||||||||||||
g206        IVFFNTGGAHRYSHVGLYIGNGEFIHAPGSGKTIKTEKLSTPFYAKNYLGAHTFFTE
                     130       140       150       160       170
```

The following partial DNA sequence was identified in *N. meningitidis* <SEQ ID 66>:

```
a206.seq
      1  ATGTTTCCCC CCGACAAAAC CCTTTTCCTC TGTCTCAGCG CACTGCTCCT
     51  CGCCTCATGC GGCACGACCT CCGGCAAACA CCGCCAACCG AAACCCAAAC
    101  AGACAGTCCG GCAAATCCAA GCCGTCCGCA TCAGCCACAT CGACCGCACA
    151  CAAGGCTCGC AGGAACTCAT GCTCCACAGC CTCGGACTCA TCGGCACGCC
    201  CTACAAATGG GGCGGCAGCA GCACCGCAAC CGGCTTCGAT TGCAGCGGCA
    251  TGATTCAATT CGTTTACAAA AACGCCCTCA ACGTCAAGCT GCCGCGCACC
    301  GCCCGCGACA TGGCGGCGGC AAGCCGCAAA ATCCCCGACA GCCGCCTTAA
    351  GGCCGGCGAC CTCGTATTCT TCAACACCGG CGGCGCACAC CGCTACTCAC
    401  ACGTCGGACT CTATATCGGC AACGGCGAAT TCATCCATGC CCCCAGCAGC
    451  GGCAAAACCA TCAAAACCGA AAAACTCTCC ACACCGTTTT ACGCCAAAAA
    501  CTACCTCGGC GCACATACTT TCTTTACAGA ATGA
```

This corresponds to the amino acid sequence <SEQ ID 67; ORF 206.a>:

```
a206.pep
      1  MFPPDKTLFL CLSALLLASC GTTSGKHRQP KPKQTVRQIQ AVRISHIDRT
     51  QGSQELMLHS LGLIGTPYKW GGSSTATGFD CSGMIQFVYK NALNVKLPRT
    101  ARDMAAASRK IPDSRLKAGD LVFFNTGGAH RYSHVGLYIG NGEFIHAPSS
    151  GKTIKTEKLS TPFYAKNYLG AHTFFTE*
```

m206/a206 ORFs 206 and 206.a showed a 99.4% identity in 177 aa overlap

```
                      10        20        30        40        50        60
m206.pep      MFPPDKTLFLCLSALLLASCGTTSGKHRQPKPKQTVRQIQAVRISHIDRTQGSQELMLHS
              ||||||||||||||||||||||||||||||||||||||||||||||||||||||||||||
a206          MFPPDKTLFLCLSALLLASCGTTSGKHRQPKPKQTVRQIQAVRISHIDRTQGSQELMLHS
                      10        20        30        40        50        60

                      70        80        90       100       110       120
m206.pep      LGLIGTPYKWGGSSTATGFDCSGMIQFVYKNALNVKLPRTARDMAAASRKIPDSRXKAGD
              ||||||||||||||||||||||||||||||||||||||||||||||||||||||| ||||
a206          LGLIGTPYKWGGSSTATGFDCSGMIQFVYKNALNVKLPRTARDMAAASRKIPDSRLKAGD
                      70        80        90       100       110       120

                     130       140       150       160       170
m206.pep      LVFFNTGGAHRYSHVGLYIGNGEFIHAPSSGKTIKTEKLSTPFYAKNYLGAHTFFTEX
              ||||||||||||||||||||||||||||||||||||||||||||||||||||||||||
a206          LVFFNTGGAHRYSHVGLYIGNGEFIHAPSSGKTIKTEKLSTPFYAKNYLGAHTFFTEX
                     130       140       150       160       170
```

## 287

The following partial DNA sequence was identified in *N. meningitidis* <SEQ ID 68>:

m287.seq
```
   1 ATGTTTAAAC GCAGCGTAAT CGCAATGGCT TGTATTTTTG CCCTTTCAGC
  51 CTGCGGGGGC GGCGGTGGCG GATCGCCCGA TGTCAAGTCG GCGGACACGC
 101 TGTCAAAACC TGCCGCCCCT GTTGTTTCTG AAAAAGAGAC AGAGGCAAAG
 151 GAAGATGCGC CACAGGCAGG TTCTCAAGGA CAGGGCGCGC CATCCGCACA
 201 AGGCAGTCAA GATATGGCGG CGGTTTCGGA AGAAAATACA GGCAATGGCG
 251 GTGCGGTAAC AGCGGATAAT CCCAAAAATG AAGACGAGGT GGCACAAAAT
 301 GATATGCCGC AAAATGCCGC CGGTACAGAT AGTTCGACAC CGAATCACAC
 351 CCCGGATCCG AATATGCTTG CCGGAAATAT GGAAAATCAA GCAACGGATG
 401 CCGGGGAATC GTCTCAGCCG GCAAACCAAC CGGATATGGC AAATGCGGCG
 451 GACGGAATGC AGGGGGACGA TCCGTCGGCA GGCGGGCAAA ATGCCGGCAA
 501 TACGGCTGCC CAAGGTGCAA ATCAAGCCGG AAACAATCAA GCCGCCGGTT
 551 CTTCAGATCC CATCCCCGCG TCAAACCCTG CACCTGCGAA TGGCGGTAGC
 601 AATTTTGGAA GGGTTGATTT GGCTAATGGC GTTTTGATTG ACGGGCCGTC
 651 GCAAAATATA ACGTTGACCC ACTGTAAAGG CGATTCTTGT AGTGGCAATA
 701 ATTTCTTGGA TGAAGAAGTA CAGCTAAAAT CAGAATTTGA AAAATTAAGT
 751 GATGCAGACA AAATAAGTAA TTACAAGAAA GATGGGAAGA ATGATAAATT
 801 TGTCGGTTTG GTTGCCGATA GTGTGCAGAT GAAGGGAATC AATCAATATA
 851 TTATCTTTTA TAAACCTAAA CCCACTTCAT TTGCGCGATT TAGGCGTTCT
 901 GCACGGTCGA GGCGGTCGCT TCCGGCCGAG ATGCCGCTGA TTCCCGTCAA
 951 TCAGGCGGAT ACGCTGATTG TCGATGGGGA AGCGGTCAGC CTGACGGGGC
1001 ATTCCGGCAA TATCTTCGCG CCCGAAGGGA ATTACCGGTA TCTGACTTAC
1051 GGGGCGGAAA AATTGCCCGG CGGATCGTAT GCCCTTCGTG TTCAAGGCGA
1101 ACCGGCAAAA GGCGAAATGC TTGCGGGCGC GGCCGTGTAC AACGGCGAAG
1151 TACTGCATTT CCATACGGAA AACGGCCGTC CGTACCCGAC CAGGGGCAGG
1201 TTTGCCGCAA AAGTCGATTT CGGCAGCAAA TCTGTGGACG GCATTATCGA
1251 CAGCGGCGAT GATTTGCATA TGGGTACGCA AAAATTCAAA GCCGCCATCG
1301 ATGGAAACGG CTTTAAGGGG ACTTGGACGG AAAATGGCAG CGGGGATGTT
1351 TCCGGAAAGT TTTACGGCCC GGCCGGCGAG GAAGTGGCGG GAAAATACAG
1401 CTATCGCCCG ACAGATGCGG AAAAGGGCGG ATTCGGCGTG TTTGCCGGCA
1451 AAAAAGAGCA GGATTGA
```

This corresponds to the amino acid sequence <SEQ ID 69; ORF 287>:

m287.pep
```
   1 MFKRSVIAMA CIFALSACGG GGGGSPDVKS ADTLSKPAAP VVSEKETEAK
  51 EDAPQAGSQG QGAPSAQGSQ DMAAVSEENT GNGGAVTADN PKNEDEVAQN
 101 DMPQNAAGTD SSTPNHTPDP NMLAGNMENQ ATDAGESSQP ANQPDMANAA
 151 DGMQGDDPSA GGQNAGNTAA QGANQAGNNQ AAGSSDPIPA SNPAPANGGS
 201 NFGRVDLANG VLIDGPSQNI TLTHCKGDSC SGNNFLDEEV QLKSEFEKLS
 251 DADKISNYKK DGKNDKFVGL VADSVQMKGI NQYIIFYKPK PTSFARFRRS
 301 ARSRRSLPAE MPLIPVNQAD TLIVDGEAVS LTGHSGNIFA PEGNYRYLTY

 351 GAEKLPGGSY ALRVQGEPAK GEMLAGAAVY NGEVLHFHTE NGRPYPTRGR
 401 FAAKVDFGSK SVDGIIDSGD DLHMGTQKFK AAIDGNGFKG TWTENGSGDV
 451 SGKFYGPAGE EVAGKYSYRP TDAEKGGFGV FAGKKEQD*
```

The following partial DNA sequence was identified in *N. gonorrhoeae* <SEQ ID 70>:

```
g287.seq
       1  atgtttaaac gcagtgtgat tgcaatggct tgtatttttc ccctttcagc
      51  ctgtgggggc ggcggtggcg gatcgcccga tgtcaagtcg gcggacacgc
     101  cgtcaaaacc ggccgccccc gttgttgctg aaaatgccgg ggaaggggtg
     151  ctgccgaaag aaaagaaaga tgaggaggca gcgggcggtg cgccgcaagc
     201  cgatacgcag gacgcaaccg ccggagaagg cagccaagat atggcggcag
     251  tttcggcaga aaatacaggc aatggcggtg cggcaacaac ggacaacccc
     301  aaaaatgaag acgcgggggc gcaaaatgat atgccgcaaa atgccgccga
     351  atccgcaaat caaacaggga acaaccaacc cgccggttct tcagattccg
     401  cccccgcgtc aaaccctgcc cctgcgaatg gcggtagcga ttttggaagg
     451  acgaacgtgg gcaattctgt tgtgattgac ggaccgtcgc aaaatataac
     501  gttgacccac tgtaaaggcg attcttgtaa tggtgataat ttattggatg
     551  aagaagcacc gtcaaaatca gaatttgaaa aattaagtga tgaagaaaaa
     601  attaagcgat ataaaaaaga cgagcaacgg gagaattttg tcggtttggt
     651  tgctgacagg gtaaaaaagg atggaactaa caaatatatc atcttctata
     701  cggacaaacc acctactcgt tctgcacggt cgaggaggtc gcttccggcc
     751  gagattccgc tgattcccgt caatcaggcc gatacgctga ttgtggatgg
     801  ggaagcggtc agcctgacgg ggcattccgg caatatcttc gcgcccgaag
     851  ggaattaccg gtatctgact tacggggcgg aaaaattgcc cggcggatcg
     901  tatgccctcc gtgtgcaagg cgaaccggca aaaggcgaaa tgcttgttgg
     951  cacggccgtg tacaacggcg aagtgctgca tttccatatg gaaaacggcc
    1001  gtccgtaccc gtccggaggc aggtttgccg caaaagtcga tttcggcagc
    1051  aaatctgtgg acggcattat cgacagcggc gatgatttgc atatgggtac
    1101  gcaaaaattc aaagccgcca tcgatggaaa cggctttaag ggacttggga
    1151  cggaaaatgg cggcggggat gtttccggaa ggttttacgg cccggccggc
    1201  gaggaagtgg cgggaaaata cagctatcgc ccgacagatg ctgaaaaggg
    1251  cggattcggc gtgtttgccg gcaaaaaaga tcgggattga
```

This corresponds to the amino acid sequence <SEQ ID 71; ORF 287.ng>:

```
g287.pep
       1  MFKRSVIAMA CIFPLSACGG GGGGSPDVKS ADTPSKPAAP VVAENAGEGV
      51  LPKEKKDEEA AGGAPQADTQ DATAGEGSQD MAAVSAENTG NGGAATTDNP
     101  KNEDAGAQND MPQNAAESAN QTGNNQPAGS SDSAPASNPA PANGGSDFGR
     151  TNVGNSVVID GPSQNITLTH CKGDSCNGDN LLDEEAPSKS EFEKLSDEEK
     201  IKRYKKDEQR ENFVGLVADR VKKDGTNKYI IFYTDKPPTR SARSRRSLPA
     251  EIPLIPVNQA DTLIVDGEAV SLTGHSGNIF APEGNYRYLT YGAEKLPGGS
     301  YALRVQGEPA KGEMLVGTAV YNGEVLHFHM ENGRPYPSGG RFAAKVDFGS
     351  KSVDGIIDSG DDLHMGTQKF KAAIDGNGFK GTWTENGGGD VSGRFYGPAG
     401  EEVAGKYSYR PTDAEKGGFG VFAGKKDRD*
```

**m287/g287** ORFs 287 and 287.ng showed a 70.1% identity in 499 aa overlap

```
                        10        20        30        40                  49
m287.pep        MFKRSVIAMACIFALSACGGGGGGSPDVKSADTLSKPAAPVVSE-----------KETEA
                ||||||||||||| ||||||||||||||||||| ||||||||:|           |: ||
g287            MFKRSVIAMACIFPLSACGGGGGGSPDVKSADTPSKPAAPVVAENAGEGVLPKEKKDEEA
                        10        20        30        40        50        60

               50        60        70        80        90       100      109
m287.pep        KEDAPQAGSQGQGAPSAQGSQDMAAVSEENTGNGGAVTADNPKNEDEVAQNDMPQNAAGT
                   |||| :|   | :::||||||||| ||||||||:|:|||||||  ||||||||||  
g287            AGGAPQADTQD--ATAGEGSQDMAAVSAENTGNGGAATTDNPKNEDAGAQNDMPQNAA--
                        70          80        90       100       110
```

```
               110       120       130       140       150       160      169
m287.pep        DSSTPNHTPDPNMLAGNMENQATDAGESSQPANQPDMANAADGMQGDDPSAGGQNAGNTA

g287            ------------------------------------------------------------

               170       180       190       200       210       220      229
m287.pep        AQGANQAGNNQAAGSSDPIPASNPAPANGGSNFGRVDLANGVLIDGPSQNITLTHCKGDS
                 ::|||:|||| |||||  ||||||||||||:|||::::|:|:|||||||||||||||||
g287            -ESANQTGNNQPAGSSDSAPASNPAPANGGSDFGRTNVGNSVVIDGPSQNITLTHCKGDS
                   120       130       140       150       160       170

               230       240       250       260       270       280      289
m287.pep        CSGNNFLDEEVQLKSEFEKLSDADKISNYKKDGKNDKFVGLVADSVQMKGINQYIIFYKP
                |:|:|:||||:  ||||||||| :||: |||| : ::||||||| |:  | |:|||||
g287            CNGDNLLDEEAPSKSEFEKLSDEEKIKRYKKDEQRENFVGLVADRVKKDGTNKYIIFYTD
                   180       190       200       210       220       230

               290       300       310       320       330       340      349
m287.pep        KPTSFARFRRSARSRRSLPAEMPLIPVNQADTLIVDGEAVSLTGHSGNIFAPEGNYRYLT
                || :     ||||||||||||:||||||||||||||||||||||||||||||||||||||
g287            KPPT-----RSARSRRSLPAEIPLIPVNQADTLIVDGEAVSLTGHSGNIFAPEGNYRYLT
                          240       250       260       270       280       290

               350       360       370       380       390       400      409
m287.pep        YGAEKLPGGSYALRVQGEPAKGEMLAGAAVYNGEVLHFHTENGRPYPTRGRFAAKVDFGS
                |||||||||||||||||||||||||:|:||||||||||| |||||||: |||||||||||
g287            YGAEKLPGGSYALRVQGEPAKGEMLVGTAVYNGEVLHFHMENGRPYPSGGRFAAKVDFGS
                          300       310       320       330       340       350

               410       420       430       440       450       460      469
m287.pep        KSVDGIIDSGDDLHMGTQKFKAAIDGNGFKGTWTENGSGDVSGKFYGPAGEEVAGKYSYR
                |||||||||||||||||||||||||||||||||||||:|||||:||||||||||||||||
g287            KSVDGIIDSGDDLHMGTQKFKAAIDGNGFKGTWTENGGGDVSGRFYGPAGEEVAGKYSYR
                          360       370       380       390       400       410

               470       480      489
m287.pep        PTDAEKGGFGVFAGKKEQDX
                ||||||||||||||||::||
g287            PTDAEKGGFGVFAGKKDRDX
                          420       430
```

The following partial DNA sequence was identified in *N. meningitidis* <SEQ ID 72>:

```
a287.seq
      1  ATGTTTAAAC GCAGTGTGAT TGCAATGGCT TGTATTGTTG CCCTTTCAGC
     51  CTGTGGGGGC GGCGGTGGCG GATCGCCCGA TGTTAAGTCG GCGGACACGC
    101  TGTCAAAACC TGCCGCCCCT GTTGTTACTG AAGATGTCGG GGAAGAGGTG
    151  CTGCCGAAAG AAAAGAAAGA TGAGGAGGCG GTGAGTGGTG CGCCGCAAGC
    201  CGATACGCAG GACGCAACCG CCGGAAAAGG CGGTCAAGAT ATGGCGGCAG
    251  TTTCGGCAGA AAATACAGGC AATGGCGGTG CGGCAACAAC GGATAATCCC
    301  GAAAATAAAG ACGAGGGACC GCAAAATGAT ATGCCGCAAA ATGCCGCCGA
    351  TACAGATAGT TCGACACCGA ATCACACCCC TGCACCGAAT ATGCCAACCA
    401  GAGATATGGG AAACCAAGCA CCGGATGCCG GGGAATCGGC ACAACCGGCA
    451  AACCAACCGG ATATGGCAAA TGCGGCGGAC GGAATGCAGG GGGACGATCC
    501  GTCGGCAGGG GAAAATGCCG GCAATACGGC AGATCAAGCT GCAAATCAAG
    551  CTGAAAACAA TCAAGTCGGC GGCTCTCAAA ATCCTGCCTC TTCAACCAAT
    601  CCTAACGCCA CGAATGGCGG CAGCGATTTT GGAAGGATAA ATGTAGCTAA
    651  TGGCATCAAG CTTGACAGCG GTTCGGAAAA TGTAACGTTG ACACATTGTA
    701  AAGACAAAGT ATGCGATAGA GATTTCTTAG ATGAAGAAGC ACCACCAAAA
    751  TCAGAATTTG AAAAATTAAG TGATGAAGAA AAAATTAATA AATATAAAAA
    801  AGACGAGCAA CGAGAGAATT TTGTCGGTTT GGTTGCTGAC AGGGTAGAAA
```

```
 851  AGAATGGAAC TAACAAATAT GTCATCATTT ATAAAGACAA GTCCGCTTCA
 901  TCTTCATCTG CGCGATTCAG GCGTTCTGCA CGGTCGAGGC GGTCGCTTCC
 951  GGCCGAGATG CCGCTGATTC CCGTCAATCA GGCGGATACG CTGATTGTCG
1001  ATGGGGAAGC GGTCAGCCTG ACGGGGCATT CCGGCAATAT CTTCGCGCCC
1051  GAAGGGAATT ACCGGTATCT GACTTACGGG GCGGAAAAAT TGTCCGGCGG
1101  ATCGTATGCC CTCAGTGTGC AAGGCGAACC GGCAAAAGGC GAAATGCTTG
1151  CGGGCACGGC CGTGTACAAC GGCGAAGTGC TGCATTTCCA TATGGAAAAC
1201  GGCCGTCCGT CCCCGTCCGG AGGCAGGTTT GCCGCAAAAG TCGATTTCGG
1251  CAGCAAATCT GTGGACGGCA TTATCGACAG CGGCGATGAT TTGCATATGG
1301  GTACGCAAAA ATTCAAAGCC GTTATCGATG GAAACGGCTT TAAGGGGACT
1351  TGGACGGAAA ATGGCGGCGG GGATGTTTCC GGAAGGTTTT ACGGCCCGGC
1401  CGGCGAAGAA GTGGCGGGAA AATACAGCTA TCGCCCGACA GATGCGGAAA
1451  AGGGCGGATT CGGCGTGTTT GCCGGCAAAA AAGAGCAGGA TTGA
```

This corresponds to the amino acid sequence <SEQ ID 73; ORF 287.a>:

```
a287.pep
       1  MFKRSVIAMA CIVALSACGG GGGGSPDVKS ADTLSKPAAP VVTEDVGEEV
      51  LPKEKKDEEA VSGAPQADTQ DATAGKGGQD MAAVSAENTG NGGAATTDNP
     101  ENKDEGPQND MPQNAADTDS STPNHTPAPN MPTRDMGNQA PDAGESAQPA
     151  NQPDMANAAD GMQGDDPSAG ENAGNTADQA ANQAENNQVG GSQNPASSTN
     201  PNATNGGSDF GRINVANGIK LDSGSENVTL THCKDKVCDR DFLDEEAPPK
     251  SEFEKLSDEE KINKYKKDEQ RENFVGLVAD RVEKNGTNKY VIIYKDKSAS
     301  SSSARFRRSA RSRRSLPAEM PLIPVNQADT LIVDGEAVSL TGHSGNIFAP
     351  EGNYRYLTYG AEKLSGGSYA LSVQGEPAKG EMLAGTAVYN GEVLHFHMEN
     401  GRPSPSGGRF AAKVDFGSKS VDGIIDSGDD LHMGTQKFKA VIDGNGFKGT
     451  WTENGGGDVS GRFYGPAGEE VAGKYSYRPT DAEKGGFGVF AGKKEQD*
```

```
m287/a287      ORFs 287 and 287.a showed a 77.2% identity in 501 aa overlap

                       10        20        30        40                  49
m287.pep       MFKRSVIAMACIFALSACGGGGGGSPDVKSADTLSKPAAPVVSE-----------KETEA
               |||||||||||| |||||||||||||||||||||||||||||:|           |: ||
a287           MFKRSVIAMACIVALSACGGGGGGSPDVKSADTLSKPAAPVVTEDVGEEVLPKEKKDEEA
                       10        20        30        40        50        60

              50        60        70        80        90       100      109
m287.pep       KEDAPQAGSQGQGAPSAQGSQDMAAVSEENTGNGGAVTADNPKNEDEVAQNDMPQNAAGT
                  |||| :|   | :::|:||||||| ||||||||:|:|||:|:||  |||||||||| |
a287           VSGAPQADTQ--DATAGKGGQDMAAVSAENTGNGGAATTDNPENKDEGPQNDMPQNAADT
                       70          80        90       100       110

             110       120       130       140       150       160       169
m287.pep       DSSTPNHTPDPNMLAGNMENQATDAGESSQPANQPDMANAADGMQGDDPSAGGQNAGNTA
               ||||||||| ||| : :| ||| |||||:||||||||||||||||||||||| :||||||
a287           DSSTPNHTPAPNMPTRDMGNQAPDAGESAQPANQPDMANAADGMQGDDPSAG-ENAGNTA
              120       130       140       150       160       170

             170       180       190       200       210       220       229
m287.pep       AQGANQAGNNQAAGSSDPIPASNPAPANGGSNFGRVDLANGVLIDGPSQNITLTHCKGDS
                |:|||| |||::||::|  ::||  :||||:|||:::|||: :|: |:|:||||||
a287           DQAANQAENNQVGGSQNPASSTNPNATNGGSDFGRINVANGIKLDSGSENVTLTHCKDKV
                180       190       200       210       220       230

             230       240       250       260       270       280       289
m287.pep       CSGNNFLDEEVQLKSEFEKLSDADKISNYKKDGKNDKFVGLVADSVQMKGINQYIIFYKP
               |:  :|||||:  ||||||||| :||::|||| : ::||||||| |: :| |:|:|:||
a287           CD-RDFLDEEAPPKSEFEKLSDEEKINKYKKDEQRENFVGLVADRVEKNGTNKYVIIYKD
                 240       250       260       270       280       290

             290         300       310       320       330       340
m287.pep       KP--TSFARFRRSARSRRSLPAEMPLIPVNQADTLIVDGEAVSLTGHSGNIFAPEGNYRY
               |   :| |||||||||||||||||||||||||||||||||||||||||||||||||||||
```

```
a287         KSASSSSARFRRSARSRRSLPAEMPLIPVNQADTLIVDGEAVSLTGHSGNIFAPEGNYRY
              300       310       320       330       340       350

             350       360       370       380       390       400
m287.pep     LTYGAEKLPGGSYALRVQGEPAKGEMLAGAAVYNGEVLHFHTENGRPYPTRGRFAAKVDF
             |||||||| |||||| |||||||||||||:||||||||||| ||||| |: |||||||||
a287         LTYGAEKLSGGSYALSVQGEPAKGEMLAGTAVYNGEVLHFHMENGRPSPSGGRFAAKVDF
              360       370       380       390       400       410

             410       420       430       440       450       460
m287.pep     GSKSVDGIIDSGDDLHMGTQKFKAAIDGNGFKGTWTENGSGDVSGKFYGPAGEEVAGKYS
             ||||||||||||||||||||||||:||||||||||||||:|||||:||||||||||||||
a287         GSKSVDGIIDSGDDLHMGTQKFKAVIDGNGFKGTWTENGGGDVSGRFYGPAGEEVAGKYS
              420       430       440       450       460       470

             470       480      489
m287.pep     YRPTDAEKGGFGVFAGKKEQDX
             ||||||||||||||||||||||
a287         YRPTDAEKGGFGVFAGKKEQDX
              480       490
```

## 406

The following partial DNA sequence was identified in *N. meningitidis* <SEQ ID 74>:

**m406.seq**

```
  1  ATGCAAGCAC GGCTGCTGAT ACCTATTCTT TTTTCAGTTT TTATTTTATC
 51  CGCCTGCGGG ACACTGACAG GTATTCCATC GCATGGCGGA GGTAAACGCT
101  TTGCGGTCGA ACAAGAACTT GTGGCCGCTT CTGCCAGAGC TGCCGTTAAA
151  GACATGGATT TACAGGCATT ACACGGACGA AAAGTTGCAT TGTACATTGC
201  CACTATGGGC GACCAAGGTT CAGGCAGTTT GACAGGGGGT CGCTACTCCA
251  TTGATGCACT GATTCGTGGC GAATACATAA ACAGCCCTGC CGTCCGTACC
301  GATTACACCT ATCCACGTTA CGAAACCACC GCTGAAACAA CATCAGGCGG
351  TTTGACAGGT TTAACCACTT CTTTATCTAC ACTTAATGCC CCTGCACTCT
401  CTCGCACCCA ATCAGACGGT AGCGGAAGTA AAAGCAGTCT GGGCTTAAAT
451  ATTGGCGGGA TGGGGGATTA TCGAAATGAA ACCTTGACGA CTAACCCGCG
501  CGACACTGCC TTTCTTTCCC ACTTGGTACA GACCGTATTT TTCCTGCGCG
551  GCATAGACGT TGTTTCTCCT GCCAATGCCG ATACAGATGT GTTTATTAAC
601  ATCGACGTAT TCGGAACGAT ACGCAACAGA ACCGAAATGC ACCTATACAA
651  TGCCGAAACA CTGAAAGCCC AAACAAAACT GGAATATTTC GCAGTAGACA
701  GAACCAATAA AAAATTGCTC ATCAAACCAA AAACCAATGC GTTTGAAGCT
751  GCCTATAAAG AAAATTACGC ATTGTGGATG GGGCCGTATA AAGTAAGCAA
801  AGGAATTAAA CCGACGGAAG GATTAATGGT CGATTTCTCC GATATCCGAC
851  CATACGGCAA TCATACGGGT AACTCCGCCC CATCCGTAGA GGCTGATAAC
901  AGTCATGAGG GGTATGGATA CAGCGATGAA GTAGTGCGAC AACATAGACA
951  AGGACAACCT TGA
```

This corresponds to the amino acid sequence <SEQ ID 75; ORF 406>:

**m406.pep**

```
  1  MQARLLIPIL FSVFILSACG TLTGIPSHGG GKRFAVEQEL VAASARAAVK
 51  DMDLQALHGR KVALYIATMG DQGSGSLTGG RYSIDALIRG EYINSPAVRT
101  DYTYPRYETT AETTSGGLTG LTTSLSTLNA PALSRTQSDG SGSKSSLGLN
151  IGGMGDYRNE TLTTNPRDTA FLSHLVQTVF FLRGIDVVSP ANADTDVFIN
201  IDVFGTIRNR TEMHLYNAET LKAQTKLEYF AVDRTNKKLL IKPKTNAFEA
251  AYKENYALWM GPYKVSKGIK PTEGLMVDFS DIRPYGNHTG NSAPSVEADN
301  SHEGYGYSDE VVRQHRQGQP *
```

The following partial DNA sequence was identified in *N. gonorrhoeae* <SEQ ID 76>:

```
g406.seq
      1  ATGCGGGCAC GGCTGCTGAT ACCTATTCTT TTTTCAGTTT TTATTTTATC
     51  CGCCTGCGGG ACACTGACAG GTATTCCATC GCATGGCGGA GGCAAACGCT
    101  TCGCGGTCGA ACAAGAACTT GTGGCCGCTT CTGCCAGAGC TGCCGTTAAA
    151  GACATGGATT TACAGGCATT ACACGGACGA AAAGTTGCAT TGTACATTGC
    201  AACTATGGGC GACCAAGGTT CAGGCAGTTT GACAGGGGGT CGCTACTCCA
    251  TTGATGCACT GATTCGCGGC GAATACATAA ACAGCCCTGC CGTCCGCACC
    301  GATTACACCT ATCCGCGTTA CGAAACCACC GCTGAAACAA CATCAGGCGG
    351  TTTGACGGGT TTAACCACTT CTTTATCTAC ACTTAATGCC CCTGCACTCT
    401  CGCGCACCCA ATCAGACGGT AGCGGAAGTA GGAGCAGTCT GGGCTTAAAT
    451  ATTGGCGGGA TGGGGGATTA TCGAAATGAA ACCTTGACGA CCAACCCGCG
    501  CGACACTGCC TTTCTTTCCC ACTTGGTGCA GACCGTATTT TTCCTGCGCG
    551  GCATAGACGT TGTTTCTCCT GCCAATGCCG ATACAGATGT GTTTATTAAC
    601  ATCGACGTAT TCGGAACGAT ACGCAACAGA ACCGAAATGC ACCTATACAA
    651  TGCCGAAACA CTGAAAGCCC AAACAAAACT GGAATATTTC GCAGTAGACA
    701  GAACCAATAA AAAATTGCTC ATCAAACCCA AAACCAATGC GTTTGAAGCT
    751  GCCTATAAAG AAAATTACGC ATTGTGGATG GGGCCGTATA AAGTAAGCAA
    801  AGGAATCAAA CCGACGGAAG GATTGATGGT CGATTTCTCC GATATCCAAC
    851  CATACGGCAA TCATACGGGT AACTCCGCCC CATCCGTAGA GGCTGATAAC
    901  AGTCATGAGG GGTATGGATA CAGCGATGAA GCAGTGCGAC AACATAGACA
    951  AGGGCAACCT TGA
```

This corresponds to the amino acid sequence <SEQ ID 77; ORF 406.ng>:

```
g406.pep
      1  MRARLLIPIL FSVFILSACG TLTGIPSHGG GKRFAVEQEL VAASARAAVK
     51  DMDLQALHGR KVALYIATMG DQGSGSLTGG RYSIDALIRG EYINSPAVRT
    101  DYTYPRYETT AETTSGGLTG LTTSLSTLNA PALSRTQSDG SGSRSSLGLN
    151  IGGMGDYRNE TLTTNPRDTA FLSHLVQTVF FLRGIDVVSP ANADTDVFIN
    201  IDVFGTIRNR TEMHLYNAET LKAQTKLEYF AVDRTNKKLL IKPKTNAFEA
    251  AYKENYALWM GPYKVSKGIK PTEGLMVDFS DIQPYGNHTG NSAPSVEADN
    301  SHEGYGYSDE AVRQHRQGQP *
```

ORF 406.ng shows 98.8% identity over a 320 aa overlap with a predicted ORF (ORF406.a) from *N. gonorrhoeae:*

```
g406/m406

                    10        20        30        40        50        60
g406.pep    MRARLLIPILFSVFILSACGTLTGIPSHGGGKRFAVEQELVAASARAAVKDMDLQALHGR
            |:||||||||||||||||||||||||||||||||||||||||||||||||||||||||||
m406        MQARLLIPILFSVFILSACGTLTGIPSHGGGKRFAVEQELVAASARAAVKDMDLQALHGR
                    10        20        30        40        50        60

                    70        80        90       100       110       120
g406.pep    KVALYIATMGDQGSGSLTGGRYSIDALIRGEYINSPAVRTDYTYPRYETTAETTSGGLTG
            ||||||||||||||||||||||||||||||||||||||||||||||||||||||||||||
m406        KVALYIATMGDQGSGSLTGGRYSIDALIRGEYINSPAVRTDYTYPRYETTAETTSGGLTG
                    70        80        90       100       110       120

                   130       140       150       160       170       180
g406.pep    LTTSLSTLNAPALSRTQSDGSGSRSSLGLNIGGMGDYRNETLTTNPRDTAFLSHLVQTVF
            |||||||||||||||||||||||:||||||||||||||||||||||||||||||||||||
m406        LTTSLSTLNAPALSRTQSDGSGSKSSLGLNIGGMGDYRNETLTTNPRDTAFLSHLVQTVF
                   130       140       150       160       170       180

                   190       200       210       220       230       240
```

```
g406.pep    FLRGIDVVSPANADTDVFINIDVFGTIRNRTEMHLYNAETLKAQTKLEYFAVDRTNKKLL
            ||||||||||||||||||||||||||||||||||||||||||||||||||||||||||||
m406        FLRGIDVVSPANADTDVFINIDVFGTIRNRTEMHLYNAETLKAQTKLEYFAVDRTNKKLL
                    190       200       210       220       230       240

                    250       260       270       280       290       300
g406.pep    IKPKTNAFEAAYKENYALWMGPYKVSKGIKPTEGLMVDFSDIQPYGNHTGNSAPSVEADN
            ||||||||||||||||||||||||||||||||||||||||||:|||||||||||||||||
m406        IKPKTNAFEAAYKENYALWMGPYKVSKGIKPTEGLMVDFSDIRPYGNHTGNSAPSVEADN
                    250       260       270       280       290       300

                    310       320
g406.pep    SHEGYGYSDEAVRQHRQGQPX
            ||||||||||:||||||||||
m406        SHEGYGYSDEVVRQHRQGQPX
                    310       320
```

The following partial DNA sequence was identified in *N. meningitidis* <SEQ ID 78>:

```
a406.seq
      1  ATGCAAGCAC GGCTGCTGAT ACCTATTCTT TTTTCAGTTT TTATTTTATC
     51  CGCCTGCGGG ACACTGACAG GTATTCCATC GCATGGCGGA GGTAAACGCT
    101  TCGCGGTCGA ACAAGAACTT GTGGCCGCTT CTGCCAGAGC TGCCGTTAAA
    151  GACATGGATT TACAGGCATT ACACGGACGA AAAGTTGCAT TGTACATTGC
    201  AACTATGGGC GACCAAGGTT CAGGCAGTTT GACAGGGGGT CGCTACTCCA
    251  TTGATGCACT GATTCGTGGC GAATACATAA ACAGCCCTGC CGTCCGTACC
    301  GATTACACCT ATCCACGTTA CGAAACCACC GCTGAAACAA CATCAGGCGG
    351  TTTGACAGGT TTAACCACTT CTTTATCTAC ACTTAATGCC CCTGCACTCT
    401  CGCGCACCCA ATCAGACGGT AGCGGAAGTA AAAGCAGTCT GGGCTTAAAT
    451  ATTGGCGGGA TGGGGGATTA TCGAAATGAA ACCTTGACGA CTAACCCGCG
    501  CGACACTGCC TTTCTTTCCC ACTTGGTACA GACCGTATTT TTCCTGCGCG
    551  GCATAGACGT TGTTTCTCCT GCCAATGCCG ATACGGATGT GTTTATTAAC
    601  ATCGACGTAT TCGGAACGAT ACGCAACAGA ACCGAAATGC ACCTATACAA
    651  TGCCGAAACA CTGAAAGCCC AAACAAAACT GGAATATTTC GCAGTAGACA
    701  GAACCAATAA AAAATTGCTC ATCAAACCAA AAACCAATGC GTTTGAAGCT
    751  GCCTATAAAG AAAATTACGC ATTGTGGATG GGACCGTATA AAGTAAGCAA
    801  AGGAATTAAA CCGACAGAAG GATTAATGGT CGATTTCTCC GATATCCAAC
    851  CATACGGCAA TCATATGGGT AACTCTGCCC CATCCGTAGA GGCTGATAAC
    901  AGTCATGAGG GGTATGGATA CAGCGATGAA GCAGTGCGAC GACATAGACA
    951  AGGGCAACCT TGA
```

This corresponds to the amino acid sequence <SEQ ID 79; ORF 406.a>:

```
a406.pep
      1  MQARLLIPIL FSVFILSACG TLTGIPSHGG GKRFAVEQEL VAASARAAVK
     51  DMDLQALHGR KVALYIATMG DQGSGSLTGG RYSIDALIRG EYINSPAVRT
    101  DYTYPRYETT AETTSGGLTG LTTSLSTLNA PALSRTQSDG SGSKSSLGLN
    151  IGGMGDYRNE TLTTNPRDTA FLSHLVQTVF FLRGIDVVSP ANADTDVFIN
    201  IDVFGTIRNR TEMHLYNAET LKAQTKLEYF AVDRTNKKLL IKPKTNAFEA
    251  AYKENYALWM GPYKVSKGIK PTEGLMVDFS DIQPYGNHMG NSAPSVEADN
    301  SHEGYGYSDE AVRRHRQGQP *
```

```
m406/a406     ORFs 406 and 406.a showed a 98.8% identity in 320 aa overlap

                      10        20        30        40        50        60
m406.pep      MQARLLIPILFSVFILSACGTLTGIPSHGGGKRFAVEQELVAASARAAVKDMDLQALHGR
              ||||||||||||||||||||||||||||||||||||||||||||||||||||||||||||
a406          MQARLLIPILFSVFILSACGTLTGIPSHGGGKRFAVEQELVAASARAAVKDMDLQALHGR
                      10        20        30        40        50        60

                      70        80        90       100       110       120
m406.pep      KVALYIATMGDQGSGSLTGGRYSIDALIRGEYINSPAVRTDYTYPRYETTAETTSGGLTG
              ||||||||||||||||||||||||||||||||||||||||||||||||||||||||||||
```

```
a406        KVALYIATMGDQGSGSLTGGRYSIDALIRGEYINSPAVRTDYTYPRYETTAETTSGGLTG
                    70        80        90       100       110       120

                   130       140       150       160       170       180
m406.pep    LTTSLSTLNAPALSRTQSDGSGSKSSLGLNIGGMGDYRNETLTTNPRDTAFLSHLVQTVF
            ||||||||||||||||||||||||||||||||||||||||||||||||||||||||||||
a406        LTTSLSTLNAPALSRTQSDGSGSKSSLGLNIGGMGDYRNETLTTNPRDTAFLSHLVQTVF
                   130       140       150       160       170       180

                   190       200       210       220       230       240
m406.pep    FLRGIDVVSPANADTDVFINIDVFGTIRNRTEMHLYNAETLKAQTKLEYFAVDRTNKKLL
            ||||||||||||||||||||||||||||||||||||||||||||||||||||||||||||
a406        FLRGIDVVSPANADTDVFINIDVFGTIRNRTEMHLYNAETLKAQTKLEYFAVDRTNKKLL
                   190       200       210       220       230       240

                   250       260       270       280       290       300
m406.pep    IKPKTNAFEAAYKENYALWMGPYKVSKGIKPTEGLMVDFSDIRPYGNHTGNSAPSVEADN
            |||||||||||||||||||||||||||||||||||||||||:||||| ||||||||||||
a406        IKPKTNAFEAAYKENYALWMGPYKVSKGIKPTEGLMVDFSDIQPYGNHMGNSAPSVEADN
                   250       260       270       280       290       300

                   310       320
m406.pep    SHEGYGYSDEVVRQHRQGQPX
            ||||||||||:||:|||||||
a406        SHEGYGYSDEAVRRHRQGQPX
                   310       320
```

The following partial DNA sequence was identified in *N. meningitidis* <SEQ ID 80>:

**m726.seq**

```
  1  ATGACCATCT ATTTCAAAAA CGGCTTTTAC GACGACACAT TGGGCGGCAT
 51  CCCCGAAGGC GCGGTTGCCG TCCGCGCCGA AGAATACGCC GCCCTTTTGG
101  CAGGACAGGC GCAGGGCGGG CAGATTGCCG CAGATTCCGA CGGCCGCCCC
151  GTTTTAACCC CGCCGCGCCC GTCCGATTAC CACGAATGGG ACGGCAAAAA
201  ATGGAAAATC AGCAAAGCCG CCGCCGCCGC CCGTTTCGCC AAACAAAAAA
251  CCGCCTTGGC ATTCCGCCTC GCGGAAAAGG CGGACGAACT CAAAAACAGC
301  CTCTTGGCGG GCTATCCCCA AGTGGAAATC GACAGCTTTT ACAGGCAGGA
351  AAAAGAAGCC CTCGCGCGGC AGGCGGACAA CAACGCCCCG ACCCCGATGC
401  TGGCGCAAAT CGCCGCCGCA AGGGGCGTGG AATTGGACGT TTTGATTGAA
451  AAAGTTATCG AAAAATCCGC CCGCCTGGCT GTTGCCGCCG GCGCGATTAT
501  CGGAAAGCGT CAGCAGCTCG AAGACAAATT GAACACCATC GAAACCGCGC
551  CCGGATTGGA CGCGCTGGAA AAGGAAATCG AAGAATGGAC GCTAAACATC
601  GGCTGA
```

This corresponds to the amino acid sequence <SEQ ID 81; ORF 726>:

**m726.pep**

```
  1  MTIYFKNGFY DDTLGGIPEG AVAVRAEEYA ALLAGQAQGG QIAADSDGRP
 51  VLTPPRPSDY HEWDGKKWKI SKAAAAARFA KQKTALAFRL AEKADELKNS
101  LLAGYPQVEI DSFYRQEKEA LARQADNNAP TPMLAQIAAA RGVELDVLIE
151  KVIEKSARLA VAAGAIIGKR QQLEDKLNTI ETAPGLDALE KEIEEWTLNI
201  G*
```

The following partial DNA sequence was identified in *N. meningitidis* <SEQ ID 82>:

**m907-2.seq**

```
  1  ATGAGAAAAC CGACCGATAC CCTACCCGTT AATCTGCAAC GCCGCCGCCT
 51  GTTGTGTGCC GCCGGTGCGT TGTTGCTCAG TCCTCTGGCG CACGCCGGCG
101  CGCAACGTGA GGAAACGCTT GCCGACGATG TGGCTTCCGT GATGAGGAGT
```

```
151  TCTGTCGGCA GCGTCAATCC GCCGAGGCTG GTGTTTGACA ATCCGAAAGA
201  GGGCGAGCGT TGGTTGTCTG CCATGTCGGC ACGTTTGGCA AGGTTCGTCC
251  CCGAGGAGGA GGAGCGGCGC AGGCTGCTGG TCAATATCCA GTACGAAAGC
301  AGCCGGGCCG GTTTGGATAC GCAGATTGTG TTGGGGCTGA TTGAGGTGGA
351  AAGCGCGTTC CGCCAGTATG CAATCAGCGG TGTCGGCGCG CGCGGCCTGA
401  TGCAGGTTAT GCCGTTTTGG AAAAACTACA TCGGCAAACC GGCGCACAAC
451  CTGTTCGACA TCCGCACCAA CCTGCGTTAC GGCTGTACCA TCCTGCGCCA
501  TTACCGGAAT CTTGAAAAAG GCAACATCGT CCGCGCGCTT GCCCGCTTTA
551  ACGGCAGCTT GGGCAGCAAT AAATATCCGA ACGCCGTTTT GGGCGCGTGG
601  CGCAACCGCT GGCAGTGGCG TTGA
```

This corresponds to the amino acid sequence <SEQ ID 83; ORF 907-2>:

**m907-2.pep**

```
  1  MRKPTDTLPV NLQRRRLLCA AGALLLSPLA HAGAQREETL ADDVASVMRS
 51  SVGSVNPPRL VFDNPKEGER WLSAMSARLA RFVPEEEERR RLLVNIQYES
101  SRAGLDTQIV LGLIEVESAF RQYAISGVGA RGLMQVMPFW KNYIGKPAHN
151  LFDIRTNLRY GCTILRHYRN LEKGNIVRAL ARFNGSLGSN KYPNAVLGAW
201  RNRWQWR*
```

The following partial DNA sequence was identified in *N. meningitidis* <SEQ ID 84>:

**m953.seq**

```
  1  ATGAAAAAAA TCATCTTCGC CGCACTCGCA GCCGCCGCCA TCAGTACTGC
 51  CTCCGCCGCC ACCTACAAAG TGGACGAATA TCACGCCAAC GCCCGTTTCG
101  CCATCGACCA TTTCAACACC AGCACCAACG TCGGCGGTTT TTACGGTCTG
151  ACCGGTTCCG TCGAGTTCGA CCAAGCAAAA CGCGACGGTA AAATCGACAT
201  CACCATCCCC ATTGCCAACC TGCAAAGCGG TTCGCAACAC TTTACCGACC
251  ACCTGAAATC AGCCGACATC TTCGATGCCG CCCAATATCC GGACATCCGC
301  TTTGTTTCCA CCAAATTCAA CTTCAACGGC AAAAAACTGG TTTCCGTTGA
351  CGGCAACCTG ACCATGCACG GCAAAACCGC CCCCGTCAAA CTCAAAGCCG
401  AAAAATTCAA CTGCTACCAA AGCCCGATGG AGAAAACCGA AGTTTGTGGC
451  GGCGACTTCA GCACCACCAT CGACCGCACC AAATGGGGCA TGGACTACCT
501  CGTTAACGTT GGTATGACCA AAAGCGTCCG CATCGACATC CAAATCGAGG
551  CAGCCAAACA ATAA
```

This corresponds to the amino acid sequence <SEQ ID 85; ORF 953>:

**m953.pep**

```
  1  MKKIIFAALA AAAISTASAA TYKVDEYHAN ARFAIDHFNT STNVGGFYGL
 51  TGSVEFDQAK RDGKIDITIP IANLQSGSQH FTDHLKSADI FDAAQYPDIR
101  FVSTKFNFNG KKLVSVDGNL TMHGKTAPVK LKAEKFNCYQ SPMEKTEVCG
151  GDFSTTIDRT KWGMDYLVNV GMTKSVRIDI QIEAAKQ*
```

The following partial DNA sequence was identified in *N. meningitidis* <SEQ ID 86>:

**orf1-1.seq**

```
  1  ATGAAAACAA CCGACAAACG GACAACCGAA ACACACCGCA AAGCCCCGAA
 51  AACCGGCCGC ATCCGCTTCT CGCCTGCTTA CTTAGCCATA TGCCTGTCGT
101  TCGGCATTCT TCCCCAAGCC TGGGCGGGAC ACACTTATTT CGGCATCAAC
151  TACCAATACT ATCGCGACTT TGCCGAAAAT AAAGGCAAGT TTGCAGTCGG
201  GGCGAAAGAT ATTGAGGTTT ACAACAAAAA AGGGGAGTTG GTCGGCAAAT
251  CAATGACAAA AGCCCCGATG ATTGATTTTT CTGTGGTGTC GCGTAACGGC
301  GTGGCGGCAT TGGTGGGCGA TCAATATATT GTGAGCGTGG CACATAACGG
351  CGGCTATAAC AACGTTGATT TTGGTGCGGA AGGAAGAAAT CCCGATCAAC
401  ATCGTTTTAC TTATAAAATT GTGAAACGGA ATAATTATAA AGCAGGGACT
451  AAAGGCCATC CTTATGGCGG CGATTATCAT ATGCCGCGTT TGCATAAATT
501  TGTCACAGAT GCAGAACCTG TTGAAATGAC CAGTTATATG GATGGGCGGA
```

```
551   AATATATCGA TCAAAATAAT TACCCTGACC GTGTTCGTAT TGGGGCAGGC
601   AGGCAATATT GGCGATCTGA TGAAGATGAG CCCAATAACC GCGAAAGTTC
651   ATATCATATT GCAAGTGCGT ATTCTTGGCT CGTTGGTGGC AATACCTTTG
701   CACAAAATGG ATCAGGTGGT GGCACAGTCA ACTTAGGTAG TGAAAAAATT
751   AAACATAGCC CATATGGTTT TTTACCAACA GGAGGCTCAT TTGGCGACAG
801   TGGCTCACCA ATGTTTATCT ATGATGCCCA AAAGCAAAAG TGGTTAATTA
851   ATGGGGTATT GCAAACGGGC AACCCCTATA TAGGAAAAAG CAATGGCTTC
901   CAGCTGGTTC GTAAAGATTG GTTCTATGAT GAAATCTTTG CTGGAGATAC
951   CCATTCAGTA TTCTACGAAC CACGTCAAAA TGGGAAATAC TCTTTTAACG
1001  ACGATAATAA TGGCACAGGA AAAATCAATG CCAAACATGA ACACAATTCT
1051  CTGCCTAATA GATTAAAAAC ACGAACCGTT CAATTGTTTA ATGTTTCTTT
1101  ATCCGAGACA GCAAGAGAAC CTGTTTATCA TGCTGCAGGT GGTGTCAACA
1151  GTTATCGACC CAGACTGAAT AATGGAGAAA ATATTTCCTT TATTGACGAA
1201  GGAAAAGGCG AATTGATACT TACCAGCAAC ATCAATCAAG GTGCTGGAGG
1251  ATTATATTTC CAAGGAGATT TTACGGTCTC GCCTGAAAAT AACGAAACTT
1301  GGCAAGGCGC GGGCGTTCAT ATCAGTGAAG ACAGTACCGT TACTTGGAAA
1351  GTAAACGGCG TGGCAAACGA CCGCCTGTCC AAAATCGGCA AAGGCACGCT
1401  GCACGTTCAA GCCAAAGGGG AAAACCAAGG CTCGATCAGC GTGGGCGACG
1451  GTACAGTCAT TTTGGATCAG CAGGCAGACG ATAAAGGCAA AAAACAAGCC
1501  TTTAGTGAAA TCGGCTTGGT CAGCGGCAGG GGTACGGTGC AACTGAATGC
1551  CGATAATCAG TTCAACCCCG ACAAACTCTA TTTCGGCTTT CGCGGCGGAC
1601  GTTTGGATTT AAACGGGCAT TCGCTTTCGT TCCACCGTAT TCAAAATACC
1651  GATGAAGGGG CGATGATTGT CAACCACAAT CAAGACAAAG AATCCACCGT
1701  TACCATTACA GGCAATAAAG ATATTGCTAC AACCGGCAAT AACAACAGCT
1751  TGGATAGCAA AAAAGAAATT GCCTACAACG GTTGGTTTGG CGAGAAAGAT
1801  ACGACCAAAA CGAACGGGCG GCTCAACCTT GTTTACCAGC CCGCCGCAGA
1851  AGACCGCACC CTGCTGCTTT CCGGCGGAAC AAATTTAAAC GGCAACATCA
1901  CGCAAACAAA CGGCAAACTG TTTTTCAGCG GCAGACCAAC ACCGCACGCC
1951  TACAATCATT TAAACGACCA TTGGTCGCAA AAAGAGGGCA TTCCTCGCGG
2001  GGAAATCGTG TGGGACAACG ACTGGATCAA CCGCACATTT AAAGCGGAAA
2051  ACTTCCAAAT TAAAGGCGGA CAGGCGGTGG TTTCCCGCAA TGTTGCCAAA
2101  GTGAAAGGCG ATTGGCATTT GAGCAATCAC GCCCAAGCAG TTTTTGGTGT
2151  CGCACCGCAT CAAAGCCACA CAATCTGTAC ACGTTCGGAC TGGACGGGTC
2201  TGACAAATTG TGTCGAAAAA ACCATTACCG ACGATAAAGT GATTGCTTCA
2251  TTGACTAAGA CCGACATCAG CGGCAATGTC GATCTTGCCG ATCACGCTCA
2301  TTTAAATCTC ACAGGGCTTG CCACACTCAA CGGCAATCTT AGTGCAAATG
2351  GCGATACACG TTATACAGTC AGCCACAACG CCACCCAAAA CGGCAACCTT
2401  AGCCTCGTGG GCAATGCCCA AGCAACATTT AATCAAGCCA CATTAAACGG
2451  CAACACATCG GCTTCGGGCA ATGCTTCATT TAATCTAAGC GACCACGCCG
2501  TACAAAACGG CAGTCTGACG CTTTCCGGCA ACGCTAAGGC AAACGTAAGC
2551  CATTCCGCAC TCAACGGTAA TGTCTCCCTA GCCGATAAGG CAGTATTCCA
2601  TTTTGAAAGC AGCCGCTTTA CCGGACAAAT CAGCGGCGGC AAGGATACGG
2651  CATTACACTT AAAAGACAGC GAATGGACGC TGCCGTCAGG CACGGAATTA
2701  GGCAATTTAA ACCTTGACAA CGCCACCATT ACACTCAATT CCGCCTATCG
2751  CCACGATGCG GCAGGGGCGC AAACCGGCAG TGCGACAGAT GCGCCGCGCC
2801  GCCGTTCGCG CCGTTCGCGC CGTTCCCTAT TATCCGTTAC ACCGCCAACT
2851  TCGGTAGAAT CCCGTTTCAA CACGCTGACG GTAAACGGCA AATTGAACGG
2901  TCAGGGAACA TTCCGCTTTA TGTCGGAACT CTTCGGCTAC CGCAGCGACA
2951  AATTGAAGCT GGCGGAAAGT TCCGAAGGCA CTTACACCTT GGCGGTCAAC
3001  AATACCGGCA ACGAACCTGC AAGCCTCGAA CAATTGACGG TAGTGGAAGG
3051  AAAAGACAAC AAACCGCTGT CCGAAAACCT TAATTTCACC CTGCAAAACG
3101  AACACGTCGA TGCCGGCGCG TGGCGTTACC AACTCATCCG CAAAGACGGC
3151  GAGTTCCGCC TGCATAATCC GGTCAAAGAA CAAGAGCTTT CCGACAAACT
3201  CGGCAAGGCA GAAGCCAAAA AACAGGCGGA AAAAGACAAC GCGCAAAGCC
3251  TTGACGCGCT GATTGCGGCC GGGCGCGATG CCGTCGAAAA GACAGAAAGC
3301  GTTGCCGAAC CGGCCCGGCA GGCAGGCGGG GAAAATGTCG GCATTATGCA
3351  GGCGGAGGAA GAGAAAAAAC GGGTGCAGGC GGATAAAGAC ACCGCCTTGG
3401  CGAAACAGCG CGAAGCGGAA ACCCGGCCGG CTACCACCGC CTTCCCCCGC
3451  GCCCGCCGCG CCCGCCGGGA TTTGCCGCAA CTGCAACCCC AACCGCAGCC
3501  CCAACCGCAG CGCGACCTGA TCAGCCGTTA TGCCAATAGC GGTTTGAGTG
3551  AATTTTCCGC CACGCTCAAC AGCGTTTTCG CCGTACAGGA CGAATTAGAC
3601  CGCGTATTTG CCGAAGACCG CCGCAACGCC GTTTGGACAA GCGGCATCCG
3651  GGACACCAAA CACTACCGTT CGCAAGATTT CCGCGCCTAC CGCCAACAAA
```

```
3701  CCGACCTGCG CCAAATCGGT ATGCAGAAAA ACCTCGGCAG CGGGCGCGTC
3751  GGCATCCTGT TTTCGCACAA CCGGACCGAA AACACCTTCG ACGACGGCAT
3801  CGGCAACTCG GCACGGCTTG CCCACGGCGC CGTTTTCGGG CAATACGGCA
3851  TCGACAGGTT CTACATCGGC ATCAGCGCGG GCGCGGGTTT TAGCAGCGGC
3901  AGCCTTTCAG ACGGCATCGG AGGCAAAATC CGCCGCCGCG TGCTGCATTA
3951  CGGCATTCAG GCACGATACC GCGCCGGTTT CGGCGGATTC GGCATCGAAC
4001  CGCACATCGG CGCAACGCGC TATTTCGTCC AAAAAGCGGA TTACCGCTAC
4051  GAAAACGTCA ATATCGCCAC CCCCGGCCTT GCATTCAACC GCTACCGCGC
4101  GGGCATTAAG GCAGATTATT CATTCAAACC GGCGCAACAC ATTTCCATCA
4151  CGCCTTATTT GAGCCTGTCC TATACCGATG CCGCTTCGGG CAAAGTCCGA
4201  ACACGCGTCA ATACCGCCGT ATTGGCTCAG GATTTCGGCA AAACCCGCAG
4251  TGCGGAATGG GGCGTAAACG CCGAAATCAA AGGTTTCACG CTGTCCCTCC
4301  ACGCTGCCGC CGCCAAAGGC CCGCAACTGG AAGCGCAACA CAGCGCGGGC
4351  ATCAAATTAG GCTACCGCTG GTAA
```

This corresponds to the amino acid sequence <SEQ ID 87; ORF oral-1>:

**orf1-1.pep**

```
   1  MKTTDKRTTE THRKAPKTGR IRFSPAYLAI CLSFGILPQA WAGHTYFGIN
  51  YQYYRDFAEN KGKFAVGAKD IEVYNKKGEL VGKSMTKAPM IDFSVVSRNG
 101  VAALVGDQYI VSVAHNGGYN NVDFGAEGRN PDQHRFTYKI VKRNNYKAGT
 151  KGHPYGGDYH MPRLHKFVTD AEPVEMTSYM DGRKYIDQNN YPDRVRIGAG
 201  RQYWRSDEDE PNNRESSYHI ASAYSWLVGG NTFAQNGSGG GTVNLGSEKI
 251  KHSPYGFLPT GGSFGDSGSP MFIYDAQKQK WLINGVLQTG NPYIGKSNGF
 301  QLVRKDWFYD EIFAGDTHSV FYEPRQNGKY SFNDDNNGTG KINAKHEHNS
 351  LPNRLKTRTV QLFNVSLSET AREPVYHAAG GVNSYRPRLN NGENISFIDE
 401  GKGELILTSN INQGAGGLYF QGDFTVSPEN NETWQGAGVH ISEDSTVTWK
 451  VNGVANDRLS KIGKGTLHVQ AKGENQGSIS VGDGTVILDQ QADDKGKKQA
 501  FSEIGLVSGR GTVQLNADNQ FNPDKLYFGF RGGRLDLNGH SLSFHRIQNT
 551  DEGAMIVNHN QDKESTVTIT GNKDIATTGN NNSLDSKKEI AYNGWFGEKD
 601  TTKTNGRLNL VYQPAAEDRT LLLSGGTNLN GNITQTNGKL FFSGRPTPHA
 651  YNHLNDHWSQ KEGIPRGEIV WDNDWINRTF KAENFQIKGG QAVVSRNVAK
 701  VKGDWHLSNH AQAVFGVAPH QSHTICTRSD WTGLTNCVEK TITDDKVIAS
 751  LTKTDISGNV DLADHAHLNL TGLATLNGNL SANGDTRYTV SHNATQNGNL
 801  SLVGNAQATF NQATLNGNTS ASGNASFNLS DHAVQNGSLT LSGNAKANVS
 851  HSALNGNVSL ADKAVFHFES SRFTGQISGG KDTALHLKDS EWTLPSGTEL
 901  GNLNLDNATI TLNSAYRHDA AGAQTGSATD APRRRSRRSR RSLLSVTPPT
 951  SVESRFNTLT VNGKLNGQGT FRFMSELFGY RSDKLKLAES SEGTYTLAVN
1001  NTGNEPASLE QLTVVEGKDN KPLSENLNFT LQNEHVDAGA WRYQLIRKDG
1051  EFRLHNPVKE QELSDKLGKA EAKKQAEKDN AQSLDALIAA GRDAVEKTES
1101  VAEPARQAGG ENVGIMQAEE EKKRVQADKD TALAKQREAE TRPATTAFPR
1151  ARRARRDLPQ LQPQPQPQPQ RDLISRYANS GLSEFSATLN SVFAVQDELD
1201  RVFAEDRRNA VWTSGIRDTK HYRSQDFRAY RQQTDLRQIG MQKNLGSGRV
1251  GILFSHNRTE NTFDDGIGNS ARLAHGAVFG QYGIDRFYIG ISAGAGFSSG
1301  SLSDGIGGKI RRRVLHYGIQ ARYRAGFGGF GIEPHIGATR YFVQKADYRY
1351  ENVNIATPGL AFNRYRAGIK ADYSFKPAQH ISITPYLSLS YTDAASGKVR
1401  TRVNTAVLAQ DFGKTRSAEW GVNAEIKGFT LSLHAAAAKG PQLEAQHSAG
1451  IKLGYRW*
```

The following partial DNA sequence was identified in *N. meningitidis* <SEQ ID 88>:

**orf46-2.seq**

```
   1  TTGGGCATTT CCCGCAAAAT ATCCCTTATT CTGTCCATAC TGGCAGTGTG
  51  CCTGCCGATG CATGCACACG CCTCAGATTT GGCAAACGAT TCTTTTATCC
 101  GGCAGGTTCT CGACCGTCAG CATTTCGAAC CCGACGGGAA ATACCACCTA
 151  TTCGGCAGCA GGGGGGAACT TGCCGAGCGC AGCGGCCATA TCGGATTGGG
 201  AAAAATACAA AGCCATCAGT TGGGCAACCT GATGATTCAA CAGGCGGCCA
 251  TTAAAGGAAA TATCGGCTAC ATTGTCCGCT TTTCCGATCA CGGGCACGAA
 301  GTCCATTCCC CCTTCGACAA CCATGCCTCA CATTCCGATT CTGATGAAGC
 351  CGGTAGTCCC GTTGACGGAT TTAGCCTTTA CCGCATCCAT TGGGACGGAT
```

```
 401  ACGAACACCA TCCCGCCGAC GGCTATGACG GGCCACAGGG CGGCGGCTAT
 451  CCCGCTCCCA AAGGCGCGAG GGATATATAC AGCTACGACA TAAAAGGCGT
 501  TGCCCAAAAT ATCCGCCTCA ACCTGACCGA CAACCGCAGC ACCGGACAAC
 551  GGCTTGCCGA CCGTTTCCAC AATGCCGGTA GTATGCTGAC GCAAGGAGTA
 601  GGCGACGGAT TCAAACGCGC CACCCGATAC AGCCCCGAGC TGGACAGATC
 651  GGGCAATGCC GCCGAAGCCT TCAACGGCAC TGCAGATATC GTTAAAAACA
 701  TCATCGGCGC GGCAGGAGAA ATTGTCGGCG CAGGCGATGC CGTGCAGGGC
 751  ATAAGCGAAG GCTCAAACAT TGCTGTCATG CACGGCTTGG GTCTGCTTTC
 801  CACCGAAAAC AAGATGGCGC GCATCAACGA TTTGGCAGAT ATGGCGCAAC
 851  TCAAAGACTA TGCCGCAGCA GCCATCCGCG ATTGGGCAGT CCAAAACCCC
 901  AATGCCGCAC AAGGCATAGA AGCCGTCAGC AATATCTTTA TGGCAGCCAT
 951  CCCCATCAAA GGGATTGGAG CTGTTCGGGG AAAATACGGC TTGGGCGGCA
1001  TCACGGCACA TCCTATCAAG CGGTCGCAGA TGGGCGCGAT CGCATTGCCG
1051  AAAGGGAAAT CCGCCGTCAG CGACAATTTT GCCGATGCGG CATACGCCAA
1101  ATACCCGTCC CCTTACCATT CCCGAAATAT CCGTTCAAAC TTGGAGCAGC
1151  GTTACGGCAA AGAAAACATC ACCTCCTCAA CCGTGCCGCC GTCAAACGGC
1201  AAAAATGTCA AACTGGCAGA CCAACGCCAC CCGAAGACAG GCGTACCGTT
1251  TGACGGTAAA GGGTTTCCGA ATTTTGAGAA GCACGTGAAA TATGATACGA
1301  AGCTCGATAT TCAAGAATTA TCGGGGGGCG GTATACCTAA GGCTAAGCCT
1351  GTGTTTGATG CGAAACCGAG ATGGGAGGTT GATAGGAAGC TTAATAAATT
1401  GACAACTCGT GAGCAGGTGG AGAAAAATGT TCAGGAAATA AGGAACGGTA
1451  ATATAAACAG TAACTTTAGC CAACATGCTC AACTAGAGAG GGAAATTAAT
1501  AAACTAAAAT CTGCCGATGA AATTAATTTT GCAGATGGAA TGGGAAAATT
1551  TACCGATAGC ATGAATGACA AGGCTTTTAG TAGGCTTGTG AAATCAGTTA
1601  AAGAGAATGG CTTCACAAAT CCAGTTGTGG AGTACGTTGA AATAAATGGA
1651  AAAGCATATA TCGTAAGAGG AAATAATRGG GTTTTTGCTG CAGAATACCT
1701  TGGCAGGATA CATGAATTAA AATTTAAAAA AGTTGACTTT CCTGTTCCTA
1751  ATACTAGTTG GAAAAATCCT ACTGATGTCT TGAATGAATC AGGTAATGTT
1801  AAGAGACCTC GTTATAGGAG TAAATAA
```

This corresponds to the amino acid sequence <SEQ ID 89; ORF orf46-2>:

```
orf46-2.pep
  1  LGISRKISLI LSILAVCLPM HAHASDLAND SFIRQVLDRQ HFEPDGKYHL
 51  FGSRGELAER SGHIGLGKIQ SHQLGNLMIQ QAAIKGNIGY IVRFSDHGHE
101  VHSPFDNHAS HSDSDEAGSP VDGFSLYRIH WDGYEHHPAD GYDGPQGGGY
151  PAPKGARDIY SYDIKGVAQN IRLNLTDNRS TGQRLADRFH NAGSMLTQGV
201  GDGFKRATRY SPELDRSGNA AEAFNGTADI VKNIIGAAGE IVGAGDAVQG
251  ISEGSNIAVM HGLGLLSTEN KMARINDLAD MAQLKDYAAA AIRDWAVQNP
301  NAAQGIEAVS NIFMAAIPIK GIGAVRGKYG LGGITAHPIK RSQMGAIALP
351  KGKSAVSDNF ADAAYAKYPS PYHSRNIRSN LEQRYGKENI TSSTVPPSNG
401  KNVKLADQRH PKTGVPFDGK GFPNFEKHVK YDTKLDIQEL SGGGIPKAKP
451  VFDAKPRWEV DRKLNKLTTR EQVEKNVQEI RNGNINSNFS QHAQLEREIN
501  KLKSADEINF ADGMGKFTDS MNDKAFSRLV KSVKENGFTN PVVEYVEING
551  KAYIVRGNNR VFAAEYLGRI HELKFKKVDF PVPNTSWKNP TDVLNESGNV
601  KRPRYRSK*
```

Using the above-described procedures, the following oligonucleotide primers were employed in the polymerase chain reaction (PCR) assay in order to clone the ORFs as indicated:

**Oligonucleotides used for PCR**

Table 1

| ORF | Primer | Sequence | Restriction sites |
|---|---|---|---|
| **279** | Forward | CGCGGATCCCATATG-TTGCCTGCAATCACGATT <SEQ ID 90> | BamHI-NdeI |
| | Reverse | CCCGCTCGAG-TTTAGAAGCGGGCGGCAA <SEQ ID 91 > | XhoI |
| **519** | Forward | CGCGGATCCCATATG-TTCAAATCCTTTGTCGTCA <SEQ ID 92> | BamHI-NdeI |
| | Reverse | CCCGCTCGAG-TTTGGCGGTTTTGCTGC <SEQ ID 93> | XhoI |

(continued)

| ORF | Primer | Sequence | Restriction sites |
|---|---|---|---|
| **576** | Forward | CGCGGATCCCATATG-GCCGCCCCGCATCT <SEQ ID 94> | BamHI-NdeI |
| | Reverse | CCCGCTCGAG-ATTTACTTTTTGATGTCGAC <SEQ ID 95> | XhoI |
| **919** | Forward | CGCGGATCCCATATG-TGCCAAAGCAAGAGCATC <SEQ ID 96> | BamHI-NdeI |
| | Reverse | CCCGCTCGAG-CGGGCGGTATTCGGG <SEQ ID 97> | XhoI |
| 121 | Forward | CGCGGATCCCATATG-GAAACACAGCTTTACAT <SEQ ID 98> | BamHI-NdeI |
| | Reverse | CCCGCTCGAG-ATAATAATATCCCGCGCCC <SEQ ID 99> | XhoI |
| 128 | Forward | CGCGGATCCCATATG-ACTGACAACGCACT <SEQ ID 100> | BamHI-NdeI |
| | Reverse | CCCGCTCGAG-GACCGCGTTGTCGAAA <SEQ ID 101> | XhoI |
| 206 | Forward | CGCGGATCCCATATG-AAACACCGCCAACCGA <SEQ ID 102> | BamHI-NdeI |
| | Reverse | CCCGCTCGAG-TTCTGTAAAAAAAGTATGTGC <SEQ ID 103> | XhoI |
| 287 | Forward | CCGGAATTCTAGCTAGC-CTTTCAGCCTGCGGG <SEQ ID 104> | EcoRI-NheI |
| | Reverse | CCCGCTCGAG-ATCCTGCTCTTTTTTGCC <SEQ ID 105> | XhoI |
| 406 | Forward | CGCGGATCCCATATG-TGCGGGACACTGACAG <SEQ ID 106> | BamHI-NdeI |
| | Reverse | CCCGCTCGAG-AGGTTGTCCTTGTCTATG <SEQ ID 107> | XhoI |

EXAMPLE 2

Expression of ORF 919

The primer described in Table 1 for ORF 919 was used to locate and clone ORF 919. The predicted gene 919 was cloned in pET vector and expressed in *E. coli.* The product of protein expression and purification was analyzed by SDS-PAGE. In panel A) is shown the analysis of 919-His fusion protein purification. Mice were immunized with the purified 919-His and sera were used for Western blot (panel B), FACS analysis (panel C), bactericidal assay (panel D), and ELISA assay (panel E). Symbols: M1, molecular weight marker; PP, purified protein, TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 919 is a surface-exposed protein and that it is a useful immunogen. The hydrophilicity plots, antigenic index, and amphipatic regions of ORF 919 are provided in Figure 10. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 919 and the amino acid sequence encoded thereby is provided in Example 1.

EXAMPLE 3

Expression of ORF 279

The primer described in Table 1 for ORF 279 was used to locate and clone ORF 279. The predicted gene 279 was cloned in pGex vector and expressed in *E. coli.* The product of protein expression and purification was analyzed by SDS-PAGE. In panel A) is shown the analysis of 279-GST purification. Mice were immunized with the purified 279-GST and sera were used for Western blot analysis (panel B), FACS analysis (panel C), bactericidal assay (panel D), and ELISA assay (panel E). Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 279 is a surface-exposed protein and that it is a useful immunogen. The hydrophilicity plots, antigenic index, and amphipatic regions of ORF 279 are provided in Figure 11. The AMPHI program is used to predict putative T-cell epitopes (Gao et al 1989, J. Immunol 143: 3007; Roberts et al. 1996, AIDS Res Human Retroviruses 12:593; Quakyi et al. 1992, Scand J Immunol Suppl 11:9). The nucleic acid sequence of ORF 279 and the amino acid sequence encoded thereby is provided in Example 1.

EXAMPLE 4

Expression of ORF 576

The primer described in Table 1 for ORF 576 was used to locate and clone ORF 576. The predicted gene *576* was cloned in pGex vector and expressed in *E. coli.* The product of protein purification was analyzed by SDS-PAGE. In panel A) is shown the analysis of 576-GST fusion protein purification. Mice were immunized with the purified 576-GST and sera were used for Western blot (panel B), FACS analysis (panel C), bactericidal assay (panel D), and ELISA assay (panel E). Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B).. These experiments confirm that ORF 576 is a surface-exposed protein and that it is a useful immunogen. The hydrophilicity plots, antigenic index, and amphipatic regions of ORF 576 are provided in Figure 12. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand JImmunol Suppl 11:9). The nucleic acid sequence of ORF 576 and the amino acid sequence encoded thereby is provided in Example 1.

EXAMPLE 5

<u>Expression of ORF 519</u>

The primer described in Table 1 for ORF 519 was used to locate and clone ORF 519. The predicted gene *519* was cloned in pET vector and expressed in *E. coli.* The product of protein purification was analyzed by SDS-PAGE. In panel A) is shown the analysis of 519-His fusion protein purification. Mice were immunized with the purified 519-His and sera were used for Western blot (panel B), FACS analysis (panel C), bactericidal assay (panel D), and ELISA assay (panel E). Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 519 is a surface-exposed protein and that it is a useful immunogen. The hydrophilicity plots, antigenic index, and amphipatic regions of ORF 519 are provided in Figure 13. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 519 and the amino acid sequence encoded thereby is provided in Example 1.

EXAMPLE 6

<u>Expression of ORF 121</u>

The primer described in Table 1 for ORF 121 was used to locate and clone ORF 121. The predicted gene *121* was cloned in pET vector and expressed in *E. coli.* The product of protein purification was analyzed by SDS-PAGE. In panel A) is shown the analysis of 121-His fusion protein purification. Mice were immunized with the purified 121-His and sera were used for Western blot analysis (panel B), FACS analysis (panel C), bactericidal assay (panel D), and ELISA assay (panel E). Results show that 121 is a surface-exposed protein. Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 121 is a surface-exposed protein and that it is a useful immunogen. The hydrophilicity plots, antigenic index, and amphipatic regions of ORF 121 are provided in Figure 14. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 121 and the amino acid sequence encoded thereby is provided in Example 1.

EXAMPLE 7

<u>Expression of ORF 128</u>

The primer described in Table 1 for ORF 128 was used to locate and clone ORF 128. The predicted gene *128* was cloned in pET vector and expressed in *E. coli.* The product of protein purification was analyzed by SDS-PAGE. In panel A) is shown the analysis of 128-His purification. Mice were immunized with the purified 128-His and sera were used for Western blot analysis (panel B), FACS analysis (panel C), bactericidal assay (panel D) and ELISA assay (panel E). Results show that 128 is a surface-exposed protein. Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 128 is a surface-exposed protein and that it is a useful immunogen. The hydrophilicity plots, antigenic index, and amphipatic regions of ORF 128 are provided in Figure 15. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 128 and the amino acid sequence encoded thereby is provided in Example 1.

EXAMPLE 8

Expression of ORF 206

The primer described in Table 1 for ORF 206 was used to locate and clone ORF 206. The predicted gene *206* was cloned in pET vector and expressed in *E. coli.* The product of protein purification was analyzed by SDS-PAGE. In panel A) is shown the analysis of 206-His purification. Mice were immunized with the purified 206-His and sera were used for Western blot analysis (panel B). It is worthnoting that the immunoreactive band in protein extracts from meningococcus is 38 kDa instead of 17 kDa (panel A). To gain information on the nature of this antibody staining we expressed ORF 206 in *E. coli* without the His-tag and including the predicted leader peptide. Western blot analysis on total protein extracts from *E. coli* expressing this native form of the 206 protein showed a recative band at a position of 38 kDa, as observed in meningococcus. We conclude that the 38 kDa band in panel B) is specific and that anti-206 antibodies, likely recognize a multimeric protein complex. In panel C is shown the FACS analysis, in panel D the bactericidal assay, and in panel E) the ELISA assay. Results show that 206 is a surface-exposed protein. Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 206 is a surface-exposed protein and that it is a useful immunogen. The hydrophilicity plots, antigenic index, and amphipatic regions of ORF 519 are provided in Figure 16. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 206 and the amino acid sequence encoded thereby is provided in Example 1.

EXAMPLE 9

Expression of ORF 287

The primer described in Table 1 for ORF 287 was used to locate and clone ORF 287. The predicted gene 287 was cloned in pGex vector and expressed in *E. coli.* The product of protein purification was analyzed by SDS-PAGE. In panel A) is shown the analysis of 287-GST fusion protein purification. Mice were immunized with the purified 287-GST and sera were used for FACS analysis (panel B), bactericidal assay (panel C), and ELISA assay (panel D). Results show that 287 is a surface-exposed protein. Symbols: M1, molecular weight marker. Arrow indicates the position of the main recombinant protein product (A). These experiments confirm that 287 is a surface-exposed protein and that it is a useful immunogen. The hydrophilicity plots, antigenic index, and amphipatic regions of ORF 287 are provided in Figure 17. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 287 and the amino acid sequence encoded thereby is provided in Example 1.

EXAMPLE 10

Expression of ORF 406

The primer described in Table 1 for ORF 406 was used to locate and clone ORF 406. The predicted gene *406* was cloned in pET vector and expressed in *E. coli.* The product of protein purification was analyzed by SDS-PAGE. In panel A) is shown the analysis of 406-His fusion protein purification. Mice were immunized with the purified 406-His and sera were used for Western blot analysis (panel B), FACS analysis (panel C), bactericidal assay (panel D), and ELISA assay (panel E). Results show that 406 is a surface-exposed protein. Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 406 is a surface-exposed protein and that it is a useful immunogen. The hydrophilicity plots, antigenic index, and amphipatic regions of ORF 406 are provided in Figure 18. The AMPHI program is used to predict putative T-cell epitopes (Gao et al 1989, J. Immunol 143:3007; Roberts et al. 1996, AIDS Res Human Retroviruses 12:593; Quakyi et al. 1992, Scand J Immunol Suppl 11:9). The nucleic acid sequence of ORF 406 and the amino acid sequence encoded thereby is provided in Example 1.

The foregoing examples are intended to illustrate but not to limit the invention.

## Claims

1. A protein consisting of the amino acid sequence encoded by nucleotides 1363680 to 1364114 of SEQ ID NO:1.

2. A protein consisting of an amino acid sequence differing from the amino acid sequence encoded by nucleotides 1363680 to 1364114 of SEQ ID NO:1, which sequence has 95% or greater identity to the amino acid sequence encoded by nucleotides 1363680 to 1364114 of SEQ ID NO: 1.

3. An antibody which binds to a protein according to claim 1 or 2.

4. The antibody of claim 3, wherein the antibody is a monoclonal antibody.

5. Nucleic acid which encodes a protein according to claim 1, consisting of nucleotides 1363680 to 1364114 of SEQ ID NO: 1.

6. An expression vector comprising the nucleic acid of claim 5.

7. Nucleic acid consisting of a nucleotide sequence complementary to a nucleic acid sequence as defined in claim 5.

8. A composition comprising a protein, a nucleic acid, or an antibody according to any preceding claim.

## Patentansprüche

1. Protein, das aus der Aminosäuresequenz besteht, welche von den Nukleotiden 1363680 bis 1364114 von SEQ ID NO: 1 kodiert wird.

2. Protein, das aus einer Aminosäuresequenz besteht, die sich von der Aminosäuresequenz unterscheidet, welche durch die Nukleotide 1363680 bis 1364114 von SEQ ID NO: 1 kodiert wird, wobei die Sequenz 95 % oder mehr Identität zu der Aminosäuresequenz aufweist, die von den Nukleotiden 1363680 bis 1364114 von SEQ ID NO: 1 kodiert wird.

3. Antikörper, der an ein Protein nach Anspruch 1 oder 2 bindet.

4. Antikörper nach Anspruch 3, wobei der Antikörper ein monoklonaler Antikörper ist.

5. Nukleinsäure, die für ein Protein nach Anspruch 1 kodiert.

6. Expressionsvektor, der die Nukleinsäure nach Anspruch 5 umfasst.

7. Nukleinsäure, die aus einer Nukleotidsequenz besteht, welche komplementär zu einer Nukleinsäuresequenz ist, wie sie in Anspruch 5 definiert wird.

8. Zusammensetzung, die ein Protein, eine Nukleinsäure oder einen Antikörper nach einem der vorhergehenden Ansprüche umfasst.

## Revendications

1. Protéine consistant en la séquence d’acides aminés codée par les nucléotides 1363680 à 1364114 de la SÉQ ID NO : 1.

2. Protéine consistant en une séquence d’acides aminés qui diffère de la séquence d’acides aminés codée par les nucléotides 1363680 à 1364114 de la SÉQ ID NO : 1, laquelle séquence possède 95 % d’identité ou davantage avec la séquence d’acides aminés codée par les nucléotides 1363680 à 1364114 de la SÉQ ID NO : 1.

3. Anticorps qui se lie à une protéine selon la revendication 1 ou 2.

4. Anticorps selon la revendication 3, où l'anticorps est un anticorps monoclonal.

5. Acide nucléique qui code pour une protéine selon la revendication 1, consistant en les nucléotides 1363680 à 1364114 de la SÉQ ID NO : 1.

6. Vecteur d'expression comprenant l'acide nucléique selon la revendication 5.

7. Acide nucléique consistant en une séquence nucléotidique complémentaire à une séquence d'acide nucléique telle que définie dans la revendication 5.

8. Composition comprenant une protéine, un acide nucléique, ou un anticorps selon l'une quelconque des revendications précédentes.

FIG. 1A

919 (46 kDa)

PURIFICATION

M1 919

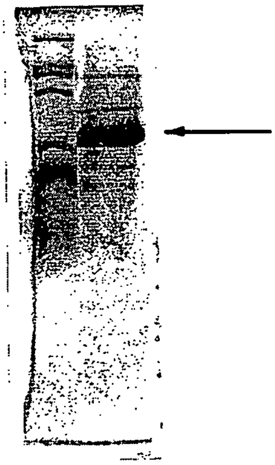

FIG. 1B

919 (46 kDa)

WESTERN BLOT

OMV TP PP

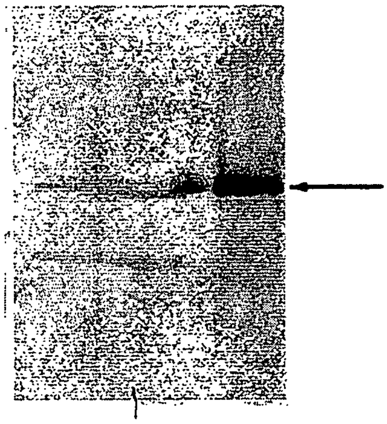

FIG. 1C

919 (46 kDa)

FACS

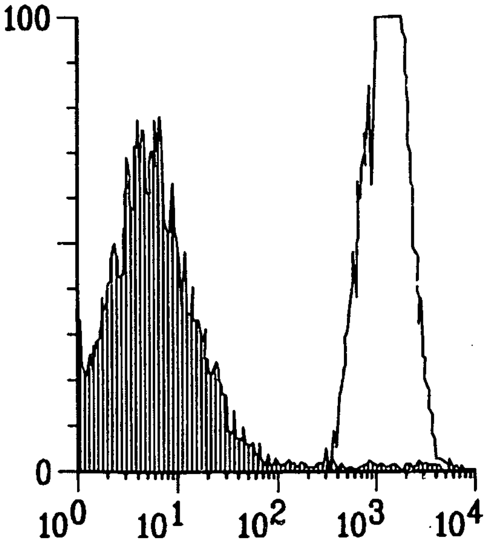


FIG. 1D

919 (46 kDa)

BACTERICIDAL ASSAY

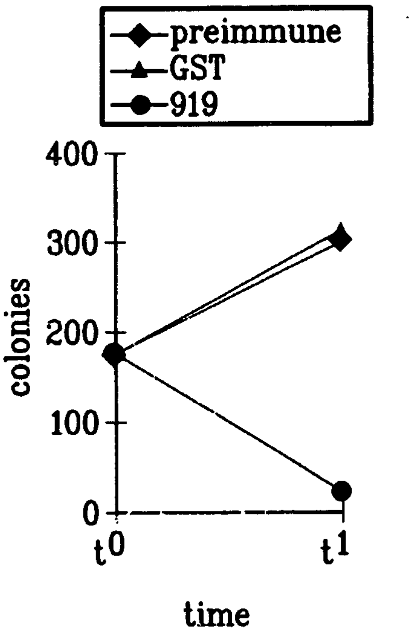


FIG. 1E

919 (46 kDa)

ELISA assay: positive

FIG. 2A

279 (10.5 kDa)
PURIFICATION
M1 279

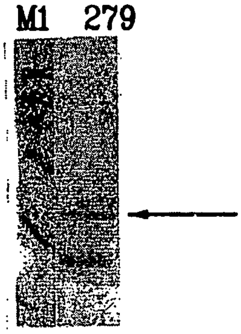

FIG. 2B

279 (10.5 kDa)
WESTERN BLOT
TP OMV

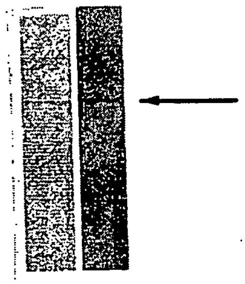

FIG. 2C

279 (10.5 kDa)
FACS

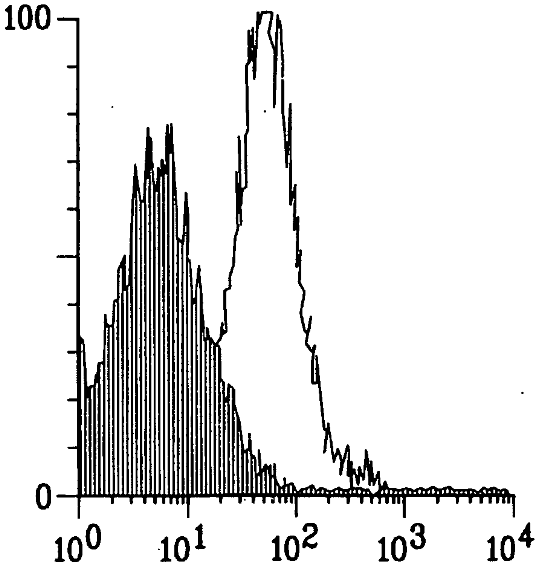


FIG. 2D

279 (10.5 kDa)
BACTERICIDAL ASSAY

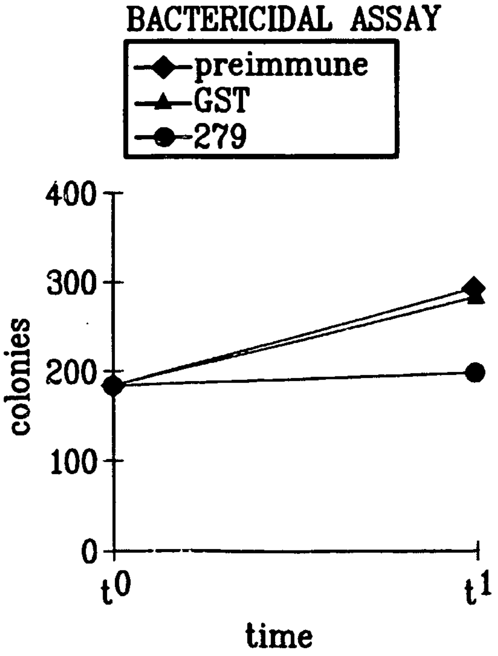


FIG. 2E

279 (10.5 kDa)

ELISA assay: <u>positive</u>

FIG. 3A

576 (27.8 kDa)
PURIFICATION
M1 576

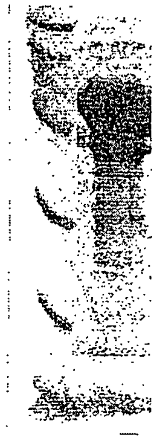

FIG. 3B

576 (27.8 kDa)
WESTERN BLOT
TP OMV

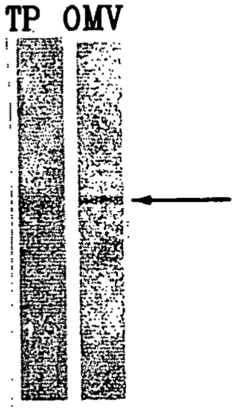

FIG. 3C

576 (27.8 kDa)
FACS

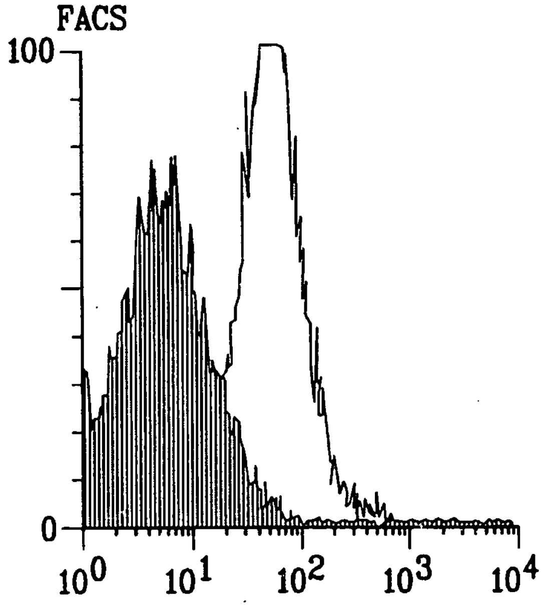


FIG. 3D

576 (27.8 kDa)
BACTERICIDAL ASSAY

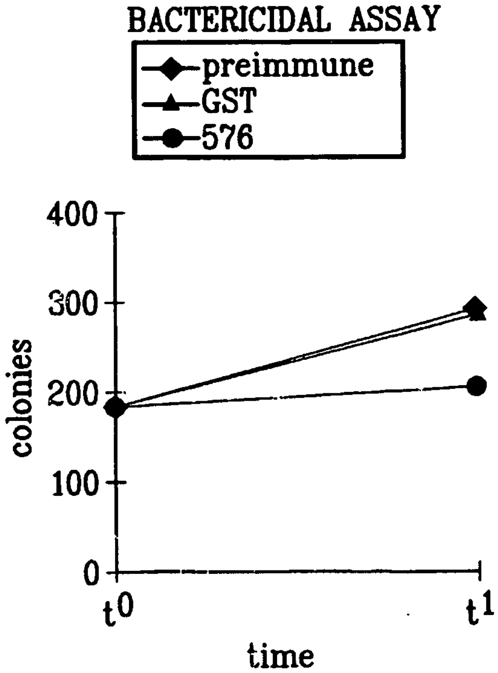


FIG. 3E

576 (27.8 kDa)
ELISA assay: positive

FIG. 4A

519 (33 kDa)
PURIFICATION

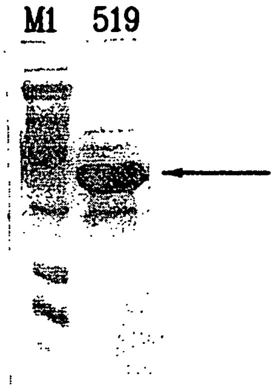


FIG. 4B

519 (33 kDa)
WESTERN BLOT

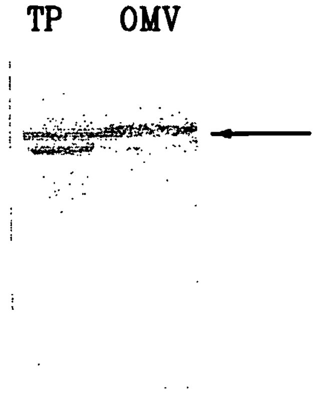


FIG. 4C

519 (33 kDa)
FACS

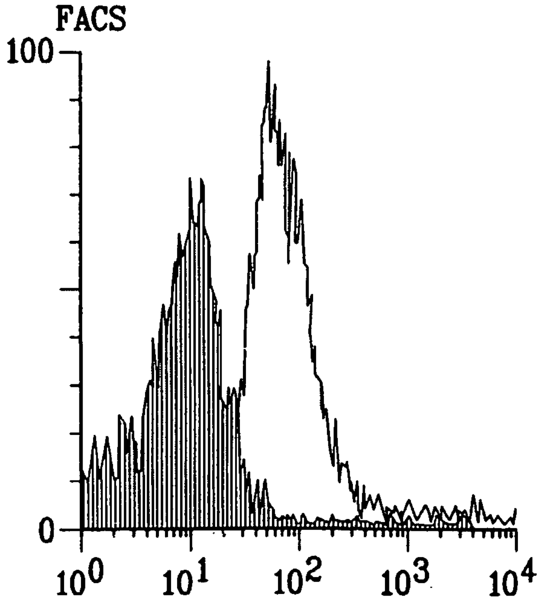


FIG. 4D

519 (33 kDa)
BACTERICIDAL ASSAY

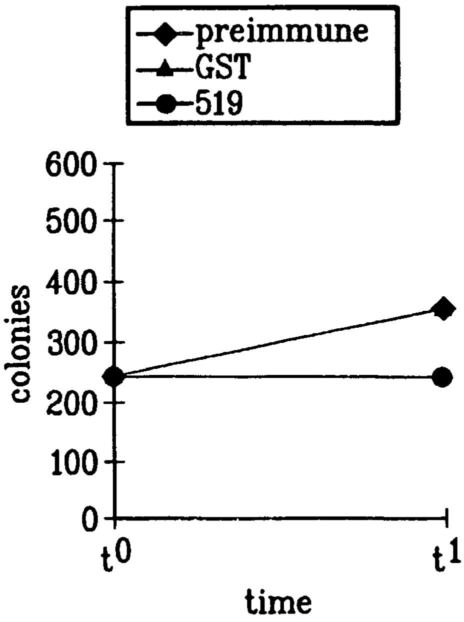


FIG. 4E

519 (33 kDa)
ELISA assay: positive

FIG. 5A

121 (40 kDa)

PURIFICATION

M1 121

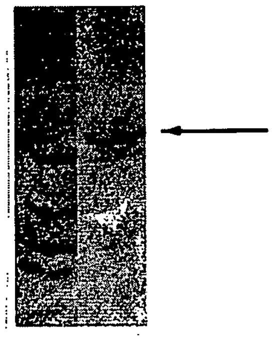

FIG. 5B

121 (40 kDa)

WESTERN BLOT

TP OMV

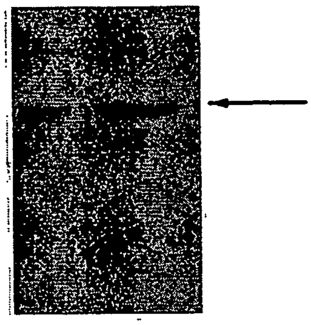

FIG. 5C

121 (40 kDa)

FACS

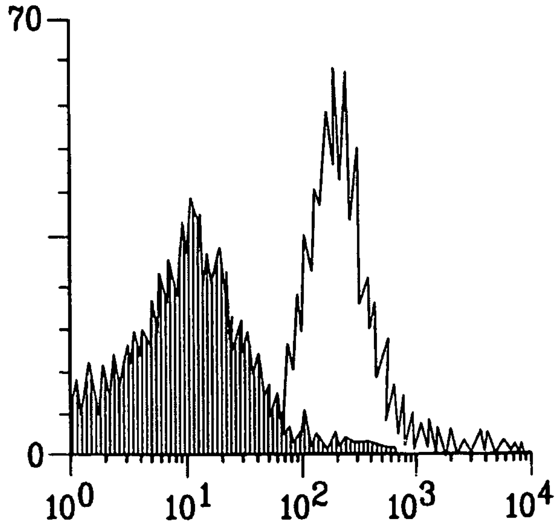


FIG. 5D

121 (40 kDa)

BACTERICIDAL ASSAY

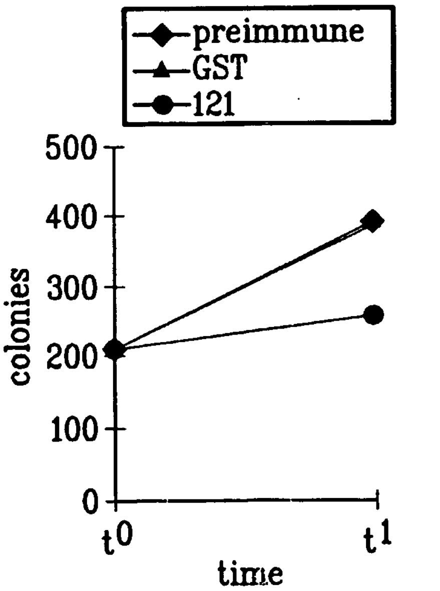


FIG. 5E

121 (40 kDa)

ELISA assay: <u>positive</u>

FIG. 6A

128 (101 kDa)

PURIFICATION

M1 128

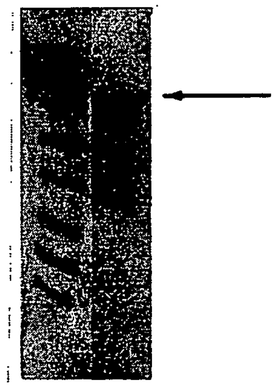

FIG. 6B

128 (101 kDa)

WESTERN BLOT

TP OMV

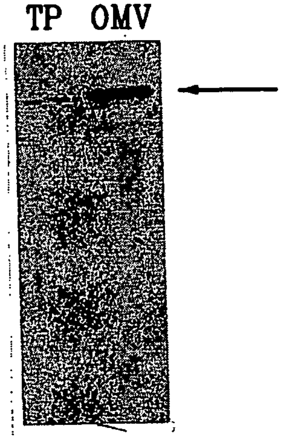

FIG. 6C

128 (101 kDa)

FACS

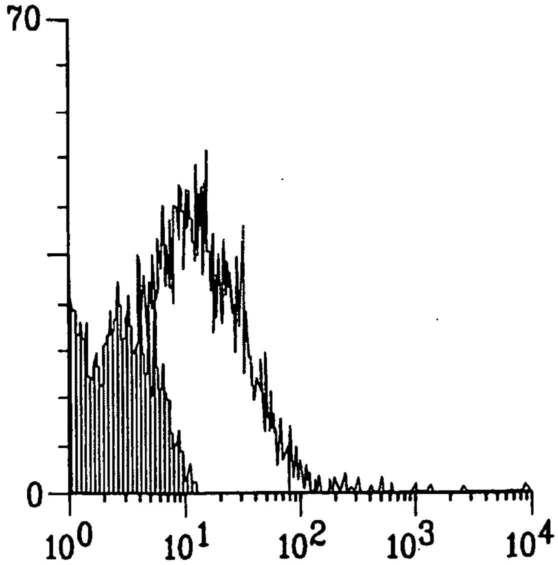


FIG. 6D

128 (101 kDa)

BACTERICIDAL ASSAY

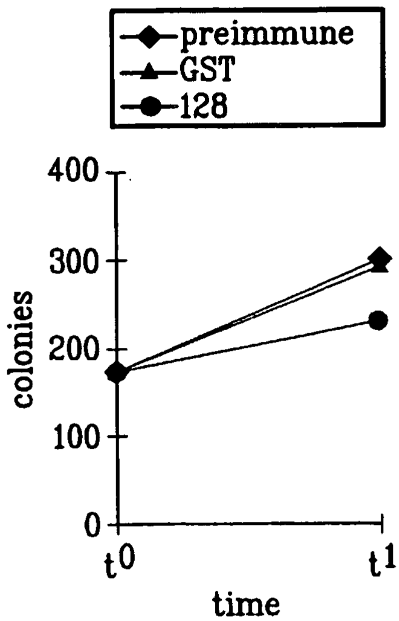


FIG. 6E

128 (101 kDa)

ELISA assay: positive

FIG. 7A

206 (17 kDa)

PURIFICATION

M1 206

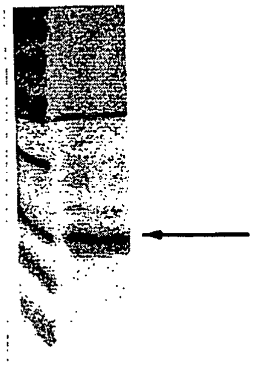

FIG. 7B

206 (17 kDa)

WESTERN BLOT

TP OMV

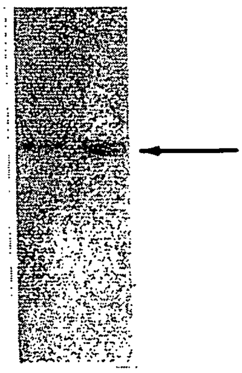

FIG. 7C

206 (17 kDa)

FACS

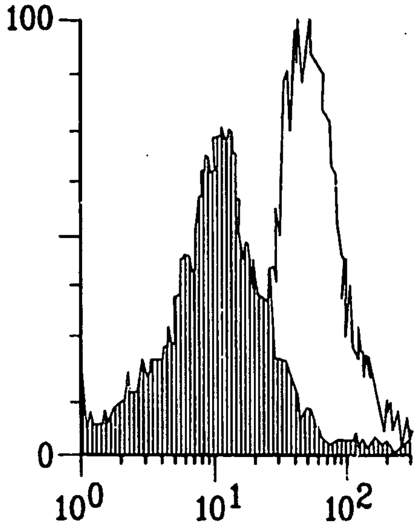


FIG. 7D

206 (17 kDa)

BACTERICIDAL ASSAY

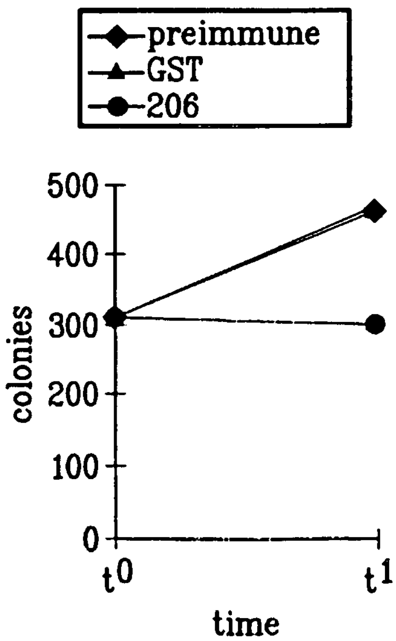


FIG. 7E

206 (17 kDa)

ELISA assay: <u>positive</u>

FIG. 8A

287 (78 kDa)
PURIFICATION

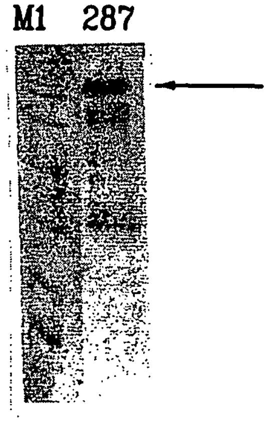


FIG. 8B

287 (78 kDa)
FACS

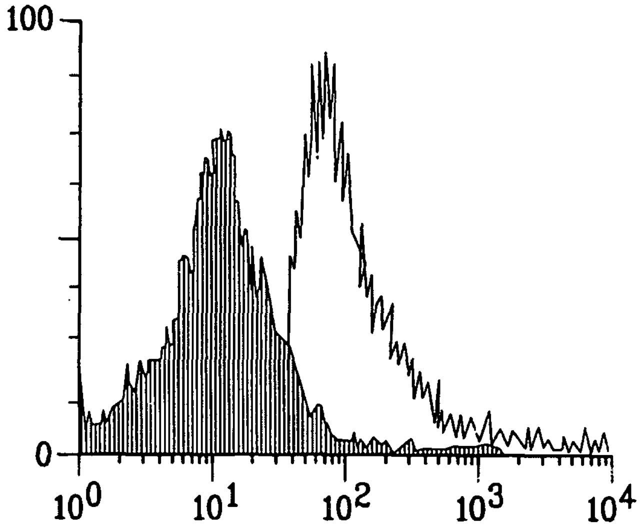


FIG. 8C

287 (78 kDa)
BACTERICIDAL ASSAY

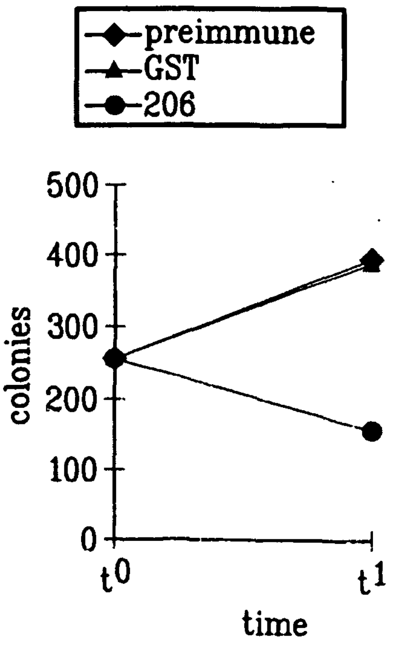


FIG. 8D

287 (78 kDa)
ELISA assay: positive

FIG. 9A

406 (33 kDa)

PURIFICATION

M1 406

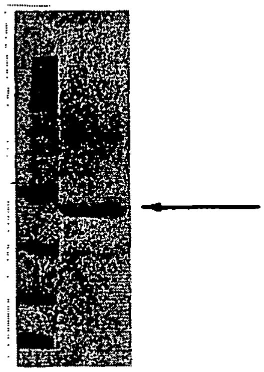

FIG. 9B

406 (33 kDa)

WESTERN BLOT

TP OMV

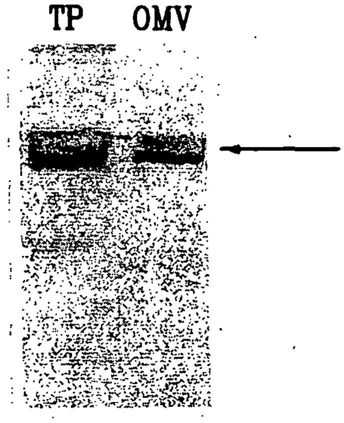

FIG. 9C

406 (33 kDa)

FACS

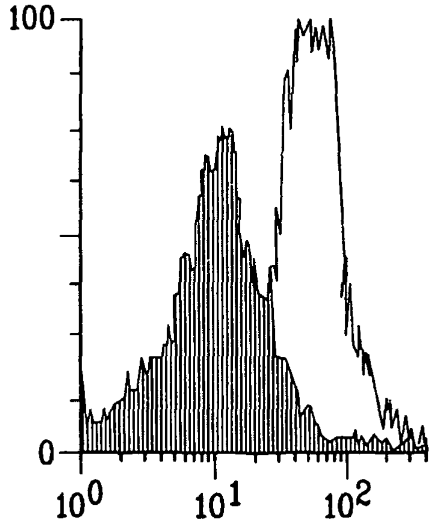


FIG. 9D

406 (33 kDa)

BACTERICIDAL ASSAY

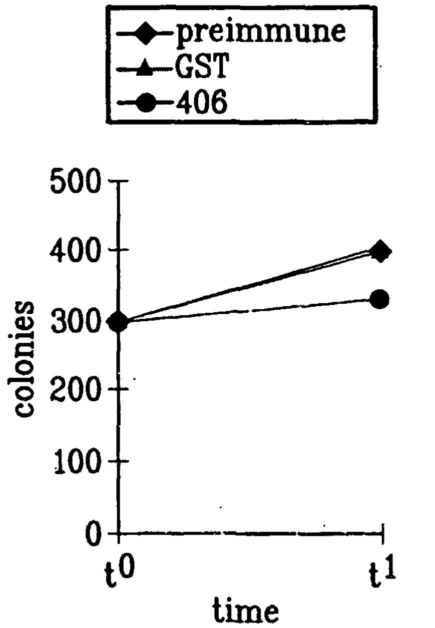


FIG. 9E

406 (33 kDa)

ELISA assay: positive

## 919
## Hydrophilicity Plot, Antigenic Index and AMPHI Regions

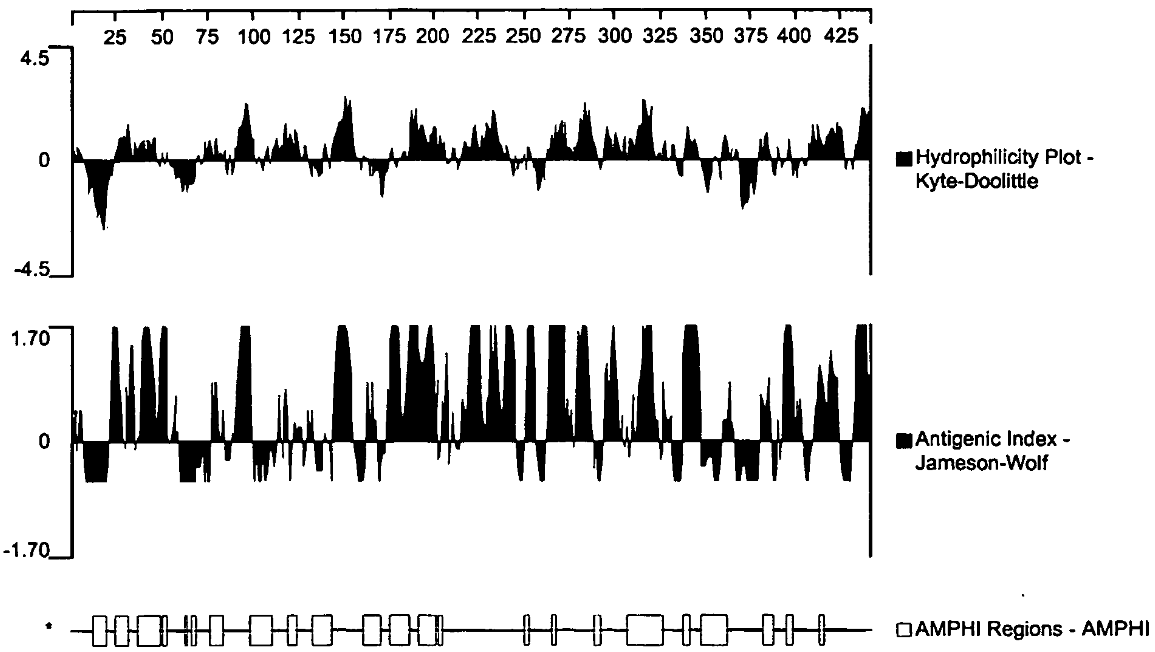


Fig. 10

## 279
## Hydrophilicity Plot, Antigenic Index and AMPHI Regions

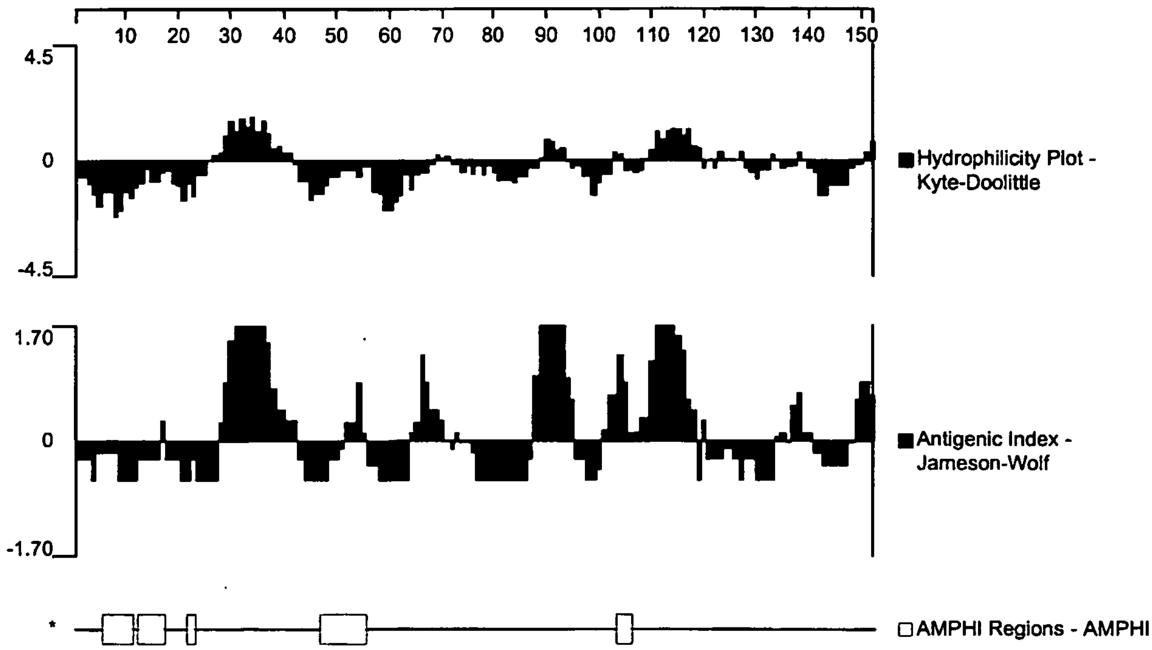


Fig. 11

## 576-1
## Hydrophilicity Plot, Antigenic Index and AMPHI Regions

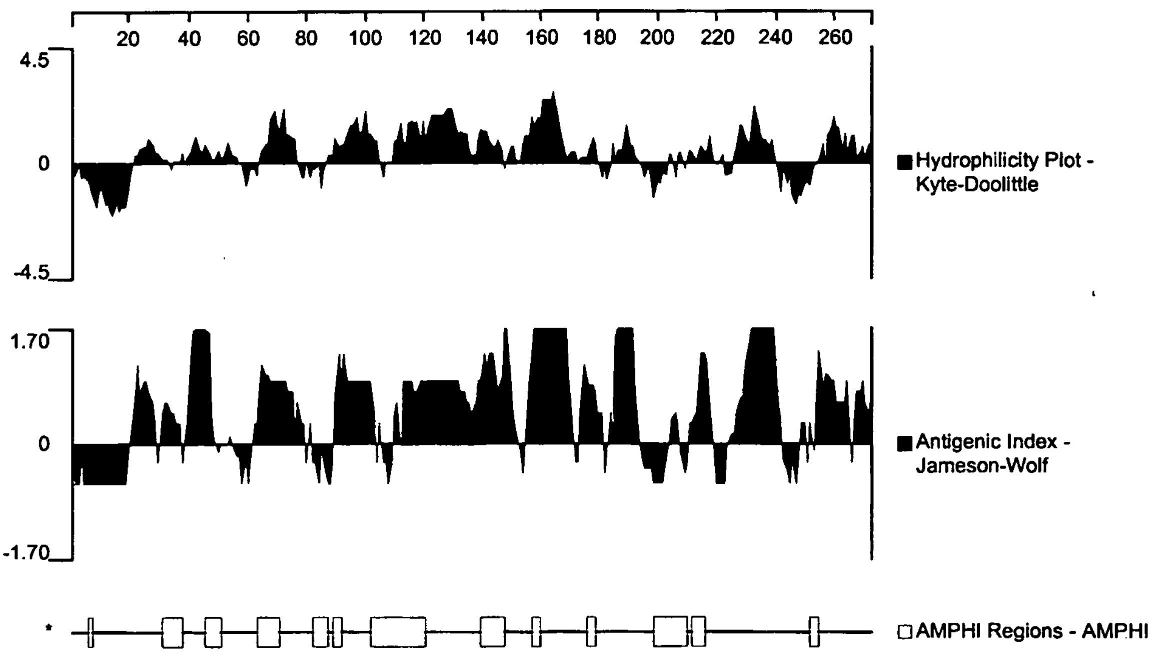


Fig. 12

# 519-1
# Hydrophilicity Plot, Antigenic Index and AMPHI Regions

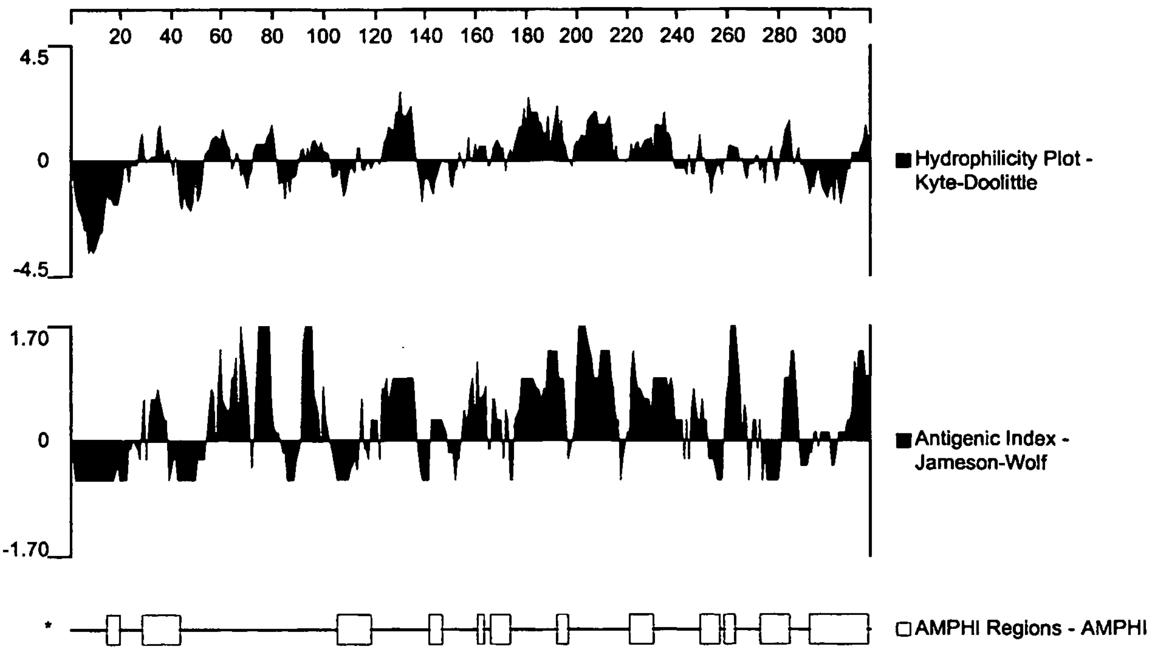


Fig. 13

## 121-1
## Hydrophilicity Plot, Antigenic Index and AMPHI Regions

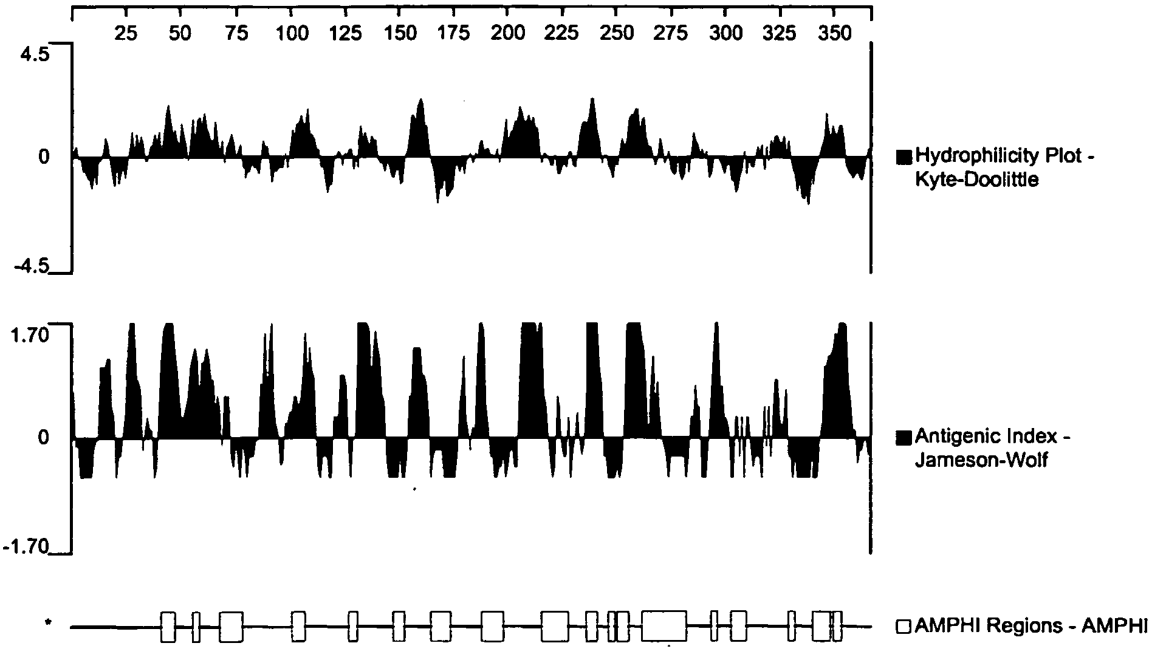


Fig. 14

# 128-1
# Hydrophilicity Plot, Antigenic Index and AMPHI Regions

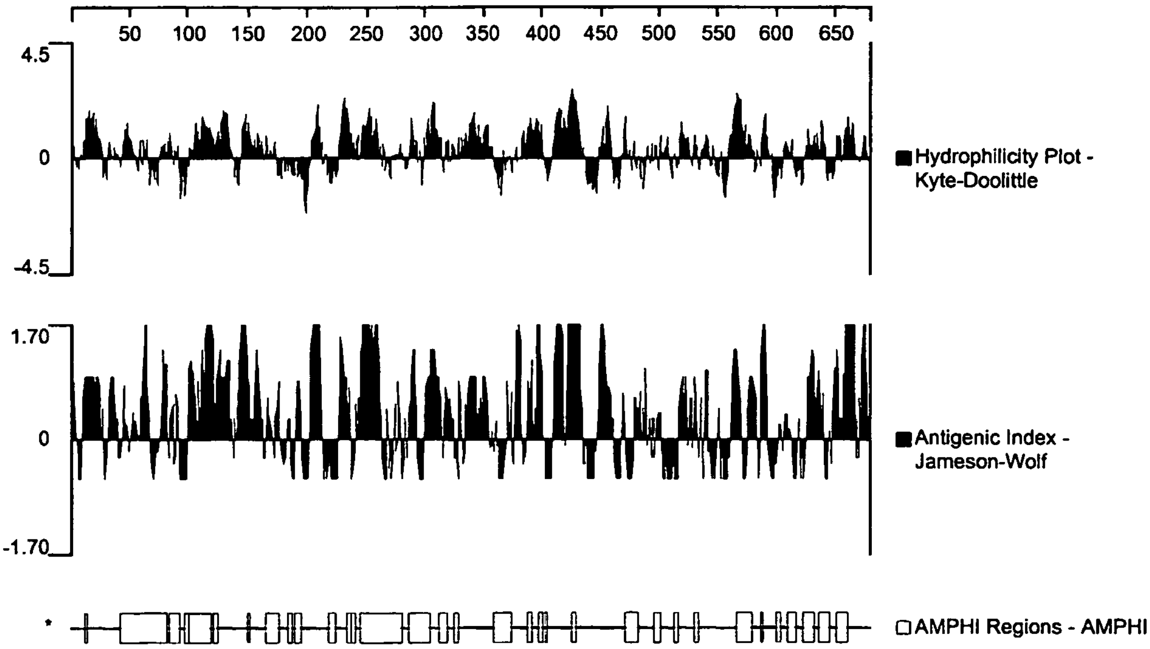


Fig. 15

## 206
## Hydrophilicity Plot, Antigenic Index and AMPHI Regions

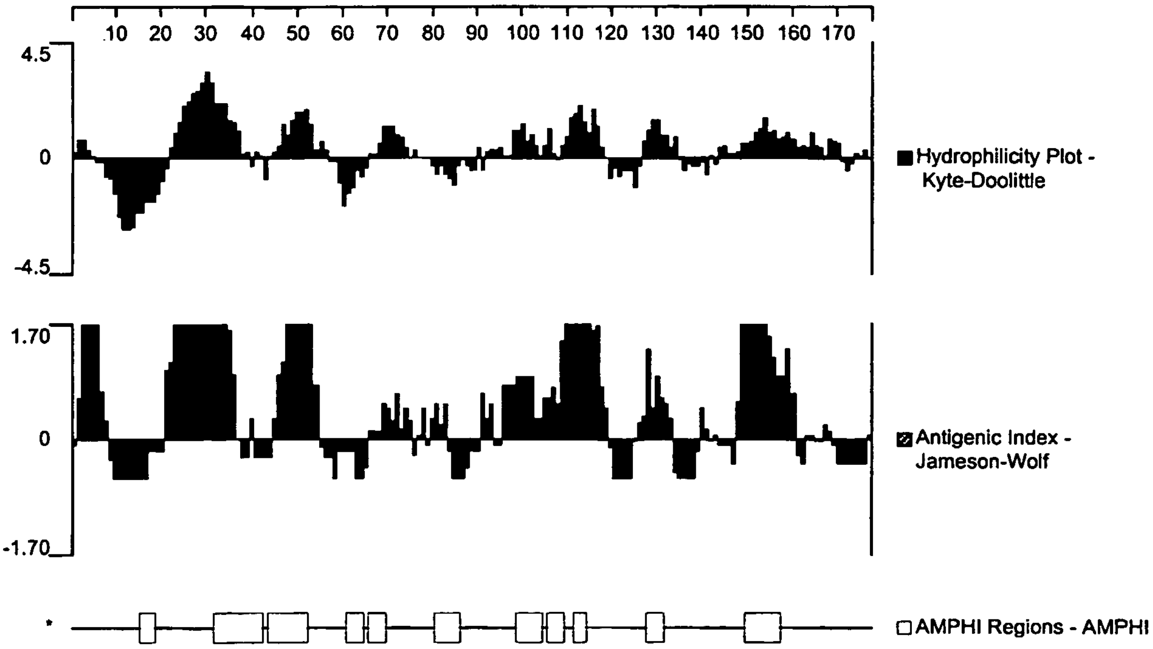


Fig. 16

**287**
**Hydrophilicity Plot, Antigenic Index and AMPHI Regions**

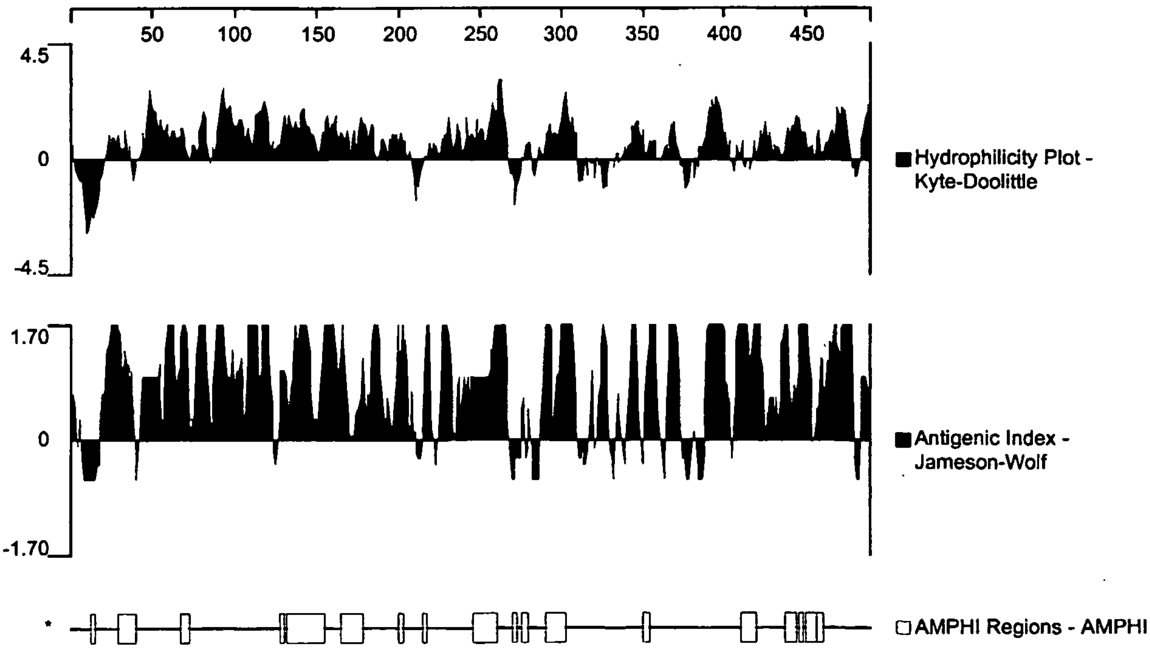


Fig. 17

# 406
# Hydrophilicity Plot, Antigenic Index and AMPHI Regions

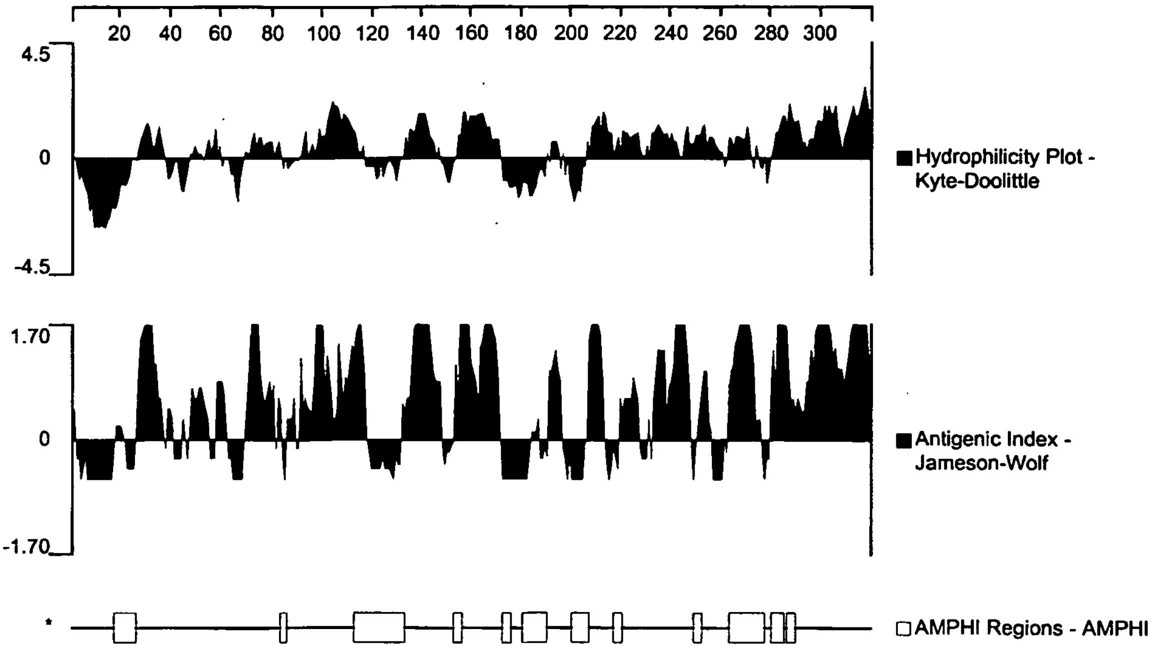


Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0467714 A
- WO 9629412 A
- US 9923573 W
- US 9801665 W, Chiron SpA
- US 9900103 W
- US 5783681 A
- US 5693506 A
- US 5659122 A
- US 5608143 A
- EP 127839 A
- EP 155476 A
- WO 89046699 A
- US 4738921 A
- EP 036776 A
- EP 121775 A
- US 4689406 A
- US 4551433 A
- EP 267851 A
- EP 219237 A
- EP 324647 A
- US 4336336 A
- EP 244042 A
- EP 127328 A
- EP 036259 A
- EP 063953 A
- WO 8404541 A
- EP 136829 A
- EP 136907 A
- US 4745056 A
- EP 284044 A
- EP 329203 A
- US 4876197 A
- US 4880734 A
- EP 164556 A
- EP 196056 A
- WO 88024066 A
- EP 012873 A
- JP 62096086 A
- US 4588684 A
- EP 060057 A
- US 4546083 A
- US 4870008 A
- EP 324274 A
- WO 8902463 A
- US 4837148 A
- US 4929555 A
- US 5753235 A
- WO 9014837 A
- WO 9313302 A
- WO 9219265 A
- US 5591624 A
- WO 9637626 A
- WO 9530763 A
- WO 9205266 A
- GB 2200651 A
- EP 0415731 A
- EP 0345242 A
- EP 0334301 A
- WO 8902468 A
- WO 8905349 A
- WO 8909271 A
- WO 9002806 A
- WO 9007936 A
- WO 9403622 A
- WO 9325698 A
- WO 9325234 A
- WO 9311230 A
- WO 9310218 A
- WO 9102805 A
- WO 9102825 A
- WO 9507994 A
- US 5219740 A
- US 4405712 A
- US 4861719 A
- US 4980289 A
- US 4777127 A
- WO 9307283 A
- WO 9306223 A
- WO 9307282 A
- WO 9412649 A
- WO 9303769 A
- WO 9319191 A
- WO 9428938 A
- WO 9511984 A
- WO 9500655 A
- WO 9527071 A
- WO 9529993 A
- WO 9534671 A
- WO 9605320 A
- WO 9408026 A
- WO 9411506 A
- WO 9424299 A
- WO 9514102 A
- WO 9524297 A
- WO 9502697 A
- WO 942815 A
- WO 9509241 A
- WO 9525807 A

- WO 9505835 A
- WO 9418922 A
- WO 9509654 A
- WO 9309239 A, Srivastava
- US 5478745 A
- US 4797368 A, Carter
- US 5139941 A, Muzyczka
- US 5474935 A, Chartejee
- WO 94288157 A, Kotin
- US 5354678 A
- US 5173414 A
- US 5252479 A
- US 5288641 A
- EP 0176170 A, Roizman
- WO 9504139 A
- WO 9009441 A
- WO 9207945 A
- EP 0453242 A
- US 5091309 A
- US 5217879 A
- WO 9210578 A
- US 08405627 B
- WO 9421792 A
- US SN08679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US SN60023867 P
- US 4762915 A
- EP 0524968 A
- WO 9820734 A
- WO 9314778 A
- US 9714465 W
- WO 9202526 A
- US 5124246 A
- US 4683195 A
- US 4683202 A

**Non-patent literature cited in the description**


- **LIEBERMAN et al.** Safety and Immunogenicity of a Serogroups A/C Neisseria meningitidis Oligosaccharide-Protein Conjugate Vaccine in Young Children. *JAMA,* 1996, vol. 275 (19), 1499-1503
- **SCHUCHAT et al.** Bacterial Meningitis in the United States in 1995. *N Engl J Med,* 1997, vol. 337 (14), 970-976
- *Morbidity and Mortality weekly report,* 1997, vol. 46 (RR-5
- New and Improved Vaccines Against Meningococcal Disease. **ZOLLINGER WD.** New Generation Vaccines. 469-488
- *Vaccine,* vol. 10, 691-698
- **ROMERO ; OUTSCHOORN.** Current status of Meningococcal group B vaccine candidates: capsular or non-capsular?. *Clin Microbiol Rev,* 1994, vol. 7 (4), 559-575
- **POOLMAN JT.** Development of a meningococcal vaccine. *Infect. Agents Dis.,* 1992, vol. 4, 13-28
- **ALA'ALDEEN ; BORRIELLO.** The meningococcal transferrin-binding proteins 1 and 2 are both surface exposed and generate bactericidal antibodies capable of killing homologous and heterologous strains. *Vaccine,* 1996, vol. 14 (1), 49-53
- **G.S. SUTTON et al.** TIGR Assembler: A New Tool for Assembling Large Shotgun Sequencing Projects. *Genome Science and Technology,* 1995, vol. 1, 9-19
- **BORODOVSKY ; MCININCH.** *Computers Chem,* 1993, vol. 17, 122-133
- **SALZBERG et al.** *Nucl Acids Res,* 1998, vol. 26, 544-548
- **ALTSCHUL et al.** Gapped BLAST and PSI-BLAST: new generation of protein database search programs. *Nucleic Acids Research,* 1997, vol. 25, 2289-3402
- **ESPOSTI et al.** Critical evaluation of the hydropathy of membrane proteins. *Eur JBiochem,* 1990, vol. 190, 207-219
- **SAMBROOK.** Molecular Cloning; A Laboratory Manual. 1989
- DNA Cloning. 1985, vol. I, ii
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. PERBAL.** A Practical Guide to Molecular Cloning. 1984

- Methods in Enzymology series. Academic Press, Inc, vol. 154 & 15
- Gene Transfer Vectors for Mammalian Cells. Cold Spring Harbor Laboratory, 1987
- Immunochemical Methods in Cell and Molecular Biology. Academic Press, 1987
- **SCOPES.** Protein Purification: Principles and Practice. Springer-Verlag, 1987
- Handbook of Experimental Immunology. 1986, vol. I-IV
- Expression of Cloned Genes in Mammalian Cells. **SAMBROOK et al.** Molecular Cloning: A Laboratory Manual. 1989
- **MANIATIS et al.** *Science,* 1987, vol. 236, 1237
- **ALBERTS et al.** Molecular Biology of the Cell. 1989
- **DIJKEMA et al.** *EMBO J.,* 1985, vol. 4, 761
- **GORMAN et al.** *Proc. Natl. Acad. Sci.,* 1982, vol. 79, 6777
- **BOSHART et al.** *Cell,* 1985, vol. 41, 521
- **SASSONE-CORSI ; BORELLI.** *Trends Genet.,* 1986, vol. 2, 215
- **BIRNSTIEL et al.** *Cell,* 1985, vol. 41, 349
- Termination and 3' end processing of eukaryotic RNA. **PROUDFOOT ; WHITELAW.** Transcription and splicing. 1988
- **PROUDFOOT.** *Trends Biochem. Sci.,* 1989, vol. 14, 105
- **SAMBROOK et al.** Expression of cloned genes in cultured mammalian cells. *Molecular Cloning: A Laboratory Manual,* 1989
- **GLUZMAN.** *Cell,* 1981, vol. 23, 175
- **KAUFMAN et al.** *Mol. Cell. Biol.,* 1989, vol. 9, 946
- **SHIMIZU et al.** *Mol. Cell. Biol.,* 1986, vol. 6, 1074
- **ZENK.** *Phytochemistry,* 1991, vol. 30, 3861-3863
- **VAULCOMBE et al.** *Mol. Gen. Genet.,* 1987, vol. 209, 33-40
- **CHANDLER et al.** *Plant Molecular Biology,* 1984, vol. 3, 407-418
- **ROGERS.** *J. Biol. Chem.,* 1985, vol. 260, 3731-3738
- **ROTHSTEIN et al.** *Gene,* 1987, vol. 55, 353-356
- **WHITTIER et al.** *Nucleic Acids Research,* 1987, vol. 15, 2515-2535
- **WIRSEL et al.** *Molecular Microbiology,* 1989, vol. 3, 3-14
- **YU et al.** *Gene,* 1992, vol. 122, 247-253
- **R.L. JONES ; J. MACMILLIN.** Gibberellins: in: Advanced Plant Physiology. Pitman Publishing Limited, 1984, 21-52
- **SHEEN.** *Plant Cell,* 1990, vol. 2, 1027-1038
- **MAAS et al.** *EMBO J.,* 1990, vol. 9, 3447-3452
- **BENKEL ; HICKEY.** *Proc. Natl. Acad. Sci.,* 1987, vol. 84, 1337-1339
- **WILMINK ; DONS.** *Plant Mol. Biol. Reptr,* 1993, vol. 11 (2), 165-185
- **REED ; MANIATIS.** *Cell,* 1985, vol. 41, 95-105
- **CROSSWAY.** *Mol. Gen. Genet,* 1985, vol. 202, 179-185
- **KRENS et al.** *Nature,* 1982, vol. 296, 72-74
- **KLEIN et al.** *Nature,* 1987, vol. 327, 70-73
- **KNUDSEN ; MULLER.** *Planta,* 1991, vol. 185, 330-336
- **FRALEY et al.** *Proc. Natl. Acad. Sci. USA,* 1982, vol. 79, 1859-1863
- **FROMM et al.** *Proc. Natl Acad. Sci. USA,* 1985, vol. 82, 5824
- **LUCKOW ; SUMMERS.** *Virology,* 1989, vol. 17, 31
- **MILLER et al.** *Ann. Rev. Microbiol.,* 1988, vol. 42, 177
- The Regulation of Baculovirus Gene Expression. **FRIESEN et al.** The Molecular Biology of Baculoviruses. 1986
- **VLAK et al.** *J. Gen. Virol.,* 1988, vol. 69, 765
- **CARBONELL et al.** *Gene,* 1988, vol. 73, 409
- **MAEDA et al.** *Nature,* 1985, vol. 315, 592
- **LEBACQ-VERHEYDEN et al.** *Molec. Cell. Biol.,* 1988, vol. 8, 3129
- **SMITH et al.** *Proc. Nat Acad. Sci. USA,* 1985, vol. 82, 8404
- **MIYAJIMA et al.** *Gene,* 1987, vol. 58, 273
- **MARTIN et al.** *DNA,* 1988, vol. 7, 99
- **SMITH et al.** *Mol. Cell. Biol.,* 1983, vol. 3, 2156
- **MILLER et al.** *Bioessays,* 1989, vol. 4, 91
- Current Protocols in Microbiology. 1990, vol. 2, 16.8
- **CARBONELL et al.** *J. Virol.,* 1985, vol. 56, 153
- **WRIGHT.** *Nature,* 1986, vol. 321, 718
- **FRASER et al.** *In Vitro Cell. Dev. Biol.,* 1989, vol. 25, 225
- **RAIBAUD et al.** *Annu. Rev. Genet.,* 1984, vol. 18, 173
- **CHANG et al.** *Nature,* 1977, vol. 198, 1056
- **GOEDDEL et al.** *Nuc. Acids Res.,* 1980, vol. 8, 4057
- **YELVERTON et al.** *Nucl. Acids Res.,* 1981, vol. 9, 731
- **SHIMATAKE et al.** *Nature,* 1981, vol. 292, 128
- **AMANN et al.** *Gene,* 1983, vol. 25, 167
- **DE BOER et al.** *Proc. Natl. Acad. Sci.,* 1983, vol. 80, 21
- **STUDIER et al.** *J. Mol. Biol.,* 1986, vol. 189, 113
- **TABOR et al.** *Proc Natl. Acad. Sci.,* 1985, vol. 82, 1074

- **SHINE et al.** *Nature,* 1975, vol. 254, 34
- Genetic signals and nucleotide sequences in messenger RNA. **STEITZ et al.** Biological Regulation and Development: Gene Expression. 1979
- Expression of cloned genes in Escherichia coli. **SAMBROOK et al.** Molecular Cloning: A Laboratory Manual. 1989
- **NAGAI et al.** *Nature,* 1984, vol. 309, 810
- **JIA et al.** *Gene,* 1987, vol. 60, 197
- **ALLEN et al.** *J. Biotechnol.,* 1987, vol. 5, 93
- **MAKOFF et al.** *J. Gen. Microbiol.,* 1989, vol. 135, 11
- **MILLER et al.** *BiolTechnology,* 1989, vol. 7, 698
- **MASUI et al.** *Experimental Manipulation of Gene Expression,* 1983
- **GHRAYEB et al.** *EMBO J.,* 1984, vol. 3, 2437
- **OKA et al.** *Proc. Natl. Acad. Sci.,* 1985, vol. 82, 7212
- **PALVA et al.** *Proc. Natl. Acad. Sci. USA,* 1982, vol. 79, 5582
- **DAVIES et al.** *Annu. Rev. Microbiol.,* 1978, vol. 32, 469
- **AMANN et al.** *Gene,* 1985, vol. 40, 183
- **POWELL et al.** *Appl. Environ. Microbiol.,* 1988, vol. 54, 655
- **MASSON et al.** *FEMS Microbiol. Lett.,* 1989, vol. 60, 273
- **MILLER et al.** *Proc. Natl. Acad. Sci.,* 1988, vol. 85, 856
- **WANG et al.** *J. Bacteriol.,* 1990, vol. 172, 949
- **COHEN et al.** *Proc. Natl. Acad. Sci.,* 1973, vol. 69, 2110
- **DOWER et al.** *Nucleic Acids Res.,* 1988, vol. 16, 6127
- An improved method for transformation of Escherichia coli with ColE1-derived plasmids. **KUSHNER.** Genetic Engineering: Proceedings of the International Symposium on Genetic Engineering. 1978
- **MANDEL et al.** *J. Mol. Biol.,* 1970, vol. 53, 159
- **TAKETO.** *Biochim. Biophys. Acta,* 1988, vol. 949, 318
- **CHASSY et al.** *FEMS Microbiol. Lett.,* 1987, vol. 44, 173
- **FIEDLER et al.** *Anal. Biochem,* 1988, vol. 170, 38
- **AUGUSTIN et al.** *FEMS Microbiol. Lett.,* 1990, vol. 66, 203
- **BARANY et al.** *J. Bacteriol.,* 1980, vol. 144, 698
- Transformation of Streptococcus lactis by electroporation. **HARLANDER.** Streptococcal Genetics. 1987
- **PERRY et al.** *Infect. Immun.,* 1981, vol. 32, 1295
- **SOMKUTI et al.** *Proc. 4th Evr. Cong. Biotechnology,* 1987, vol. 1, 412
- **MYANOHARA et al.** *Proc. Natl. Acad. Sci. USA,* 1983, vol. 80, 1
- **COHEN et al.** *Proc. Natl. Acad Sci. USA,* 1980, vol. 77, 1078
- **HENIKOFF et al.** *Nature,* 1981, vol. 283, 835
- **HOLLENBERG et al.** *Curr. Topics Microbiol. Immunol.,* 1981, vol. 96, 119
- The Expression of Bacterial Antibiotic Resistance Genes in the Yeast Saccharomyces cerevisiae. **HOLLENBERG et al.** Plasmids of Medical, Environmental and Commercial Importance. 1979
- **MERCERAU-PUIGALON et al.** *Gene,* 1980, vol. 11, 163
- **PANTHIER et al.** *Curr. Genet.,* 1980, vol. 2, 109
- **BOTSTEIN et al.** *Gene,* 1979, vol. 8, 17-24
- **BRAKE et al.** *Proc. Natl. Acad. Sci USA,* 1984, vol. 81, 4642-4646
- **STINCHCOMB et al.** *J. Mol. Biol.,* 1982, vol. 158, 157
- **ORR-WEAVER et al.** *Methods in Enzymol.,* 1983, vol. 101, 228-245
- **RINE et al.** *Proc. Natl. Acad. Sci. USA,* 1983, vol. 80, 6750
- **BUTT et al.** *Microbiol, Rev.,* 1987, vol. 51, 351
- **KURTZ et al.** *Mol. Cell. Biol.,* vol. 6, 142
- **KUNZE et al.** *J. Basic Microbiol.,* 1985, vol. 25, 141
- **GLEESON et al.** *J. Gen. Microbiol.,* vol. 132, 3459
- **ROGGENKAMP et al.** *Mol. Gen. Genet.,* 1986, vol. 202, 302
- **DAS et al.** *J. Bacteriol.,* 1984, vol. 158, 1165
- **DE LOUVENCOURT et al.** *J. Bacteriol.,* 1983, vol. 154, 737
- **VAN DEN BERG et al.** *Bio/Technology,* 1990, vol. 8, 135
- **CREGG et al.** *Mol. Cell. Biol.,* 1985, vol. 5, 3376
- **HINNEN et al.** *Proc. Natl. Acad. Sci. USA,* 1978, vol. 75, 1929
- **ITO et al.** *J. Bacteriol.,* 1983, vol. 153, 163
- **BEACH ; NURSE.** *Nature,* 1981, vol. 300, 706
- **DAVIDOW et al.** *Curr. Genet.,* 1985, vol. 10, 380471
- **GAILLARDIN et al.** *Curr. Genet.,* 1985, vol. 10, 49
- **KURTZ et al.** *Mol. Cell. Biol.,* 1986, vol. 6, 142
- **GLEESON et al.** *J. Gen. Microbiol.,* 1986, vol. 132, 3459
- **DE LOUVENCOURT et al.** *J. Bacteriol.,* 1983, vol. 154, 1165
- **KUNZE.** *J. Basic Microbiol.,* 1985, vol. 25, 141
- **DAVIDOW et al.** *Curr. Genet.,* 1985, vol. 10, 39

- **KOHLER ; MILSTEIN.** *Nature,* 1975, vol. 256, 495-96
- Remington's Pharmaceutical Sciences. Mack Pub. Co, 1991
- **ROBINSON ; TORRES.** *Seminars in Immunology,* 1997, vol. 9, 271-283
- **DONNELLY et al.** *Annu Rev Immunol,* 1997, vol. 15, 617-648
- **JOLLY.** *Cancer Gene Therapy,* 1994, vol. 1, 51-64
- **KIMURA.** *Human Gene Therapy,* 1994, vol. 5, 845-852
- **CONNELLY.** *Human Gene Therapy,* 1995, vol. 6, 185-193
- **KAPLITT.** *Nature Genetics,* 1994, vol. 6, 148-153
- **O'NEILL.** *J. Virol.,* 1985, vol. 53, 160
- **KELLY.** *J. Virol.,* 1983, vol. 45, 291
- RNA Tumor Viruses. Cold Spring Harbor Laboratory, 1985
- **HARTLEY ; ROWE.** *J Virol,* 1976, vol. 19, 19-25
- **VILE.** *Cancer Res,* 1993, vol. 53, 3860-3864
- **VILE.** *Cancer Res,* 1993, vol. 53, 962-967
- **RAM.** *Cancer Res,* 1993, vol. 53, 83-88
- **TAKAMIYA.** *J Neurosci Res,* 1992, vol. 33, 493-503
- **BABA.** *J Neurosurg,* 1993, vol. 79, 729-735
- **MANN.** *Cell,* 1983, vol. 33, 153
- **CANE.** *Proc Natl Acad Sci,* 1984, vol. 81, 6349
- **MILLER.** *Human Gene Therapy,* 1990, 1
- **BERKNER.** *Biotechniques,* 1988, vol. 6, 616
- **ROSENFELD.** *Science,* 1991, vol. 252, 431
- **CURIEL.** *Hum. Gene Ther.,* 1992, vol. 3, 147-154
- **NAHREINI.** *Gene,* 1993, vol. 124, 257-262
- **SAMULSKI.** *J. virol.,* 1987, vol. 61, 3096
- **SU.** *Human Gene Therapy,* 1996, vol. 7, 463-470
- **GELLER.** *Science,* 1988, vol. 241, 1667-1669
- **FINK.** *Human Gene Therapy,* 1992, vol. 3, 11-19
- **EVANS.** *Nature,* 1989, vol. 339, 385
- **SABIN.** *J. Biol. Standardization,* 1973, vol. 1, 115
- **ARNOLD.** *J Cell Biochem,* 1990, L401
- **FISHER-HOCH.** *Proc Natl Acad Sci,* 1989, vol. 86, 317
- **FLEXNER.** *Ann NY Acad Sci,* 1989, vol. 569, 86
- **FLEXNER.** *Vaccine,* 1990, vol. 8, 17
- **MULLIGAN.** *Nature,* 1979, vol. 277, 108
- **MADZAK.** *J Gen Virol,* 1992, vol. 73, 1533
- **ENAMI.** *Proc Natl Acad Sci,* 1990, vol. 87, 3802-3805
- **ENAMI ; PALESE.** *J Virol,* 1991, vol. 65, 2711-2713
- **LUYTJES.** *Cell,* 1989, vol. 59, 110
- **MCMICHAEL.** *NEJ Med,* 1983, vol. 309, 13
- **YAP.** *Nature,* 1978, vol. 273, 238
- *Nature,* 1979, vol. 277, 108
- **BUCHSCHACHER.** *J. Virol.,* 1992, vol. 66, 2731
- **HAMRE.** *Proc Soc Exp Biol Med,* 1966, vol. 121, 190
- **CURIEL.** *Hum Gene Ther,* 1992, vol. 3, 147-154
- **WU.** *J Biol Chem,* 1989, vol. 264, 16985-16987
- **PHILIP.** *Mol Cell Biol,* 1994, vol. 14, 2411-2418
- **WOFFENDIN.** *Proc Natl Acad Sci,* 1994, vol. 91, 1581-1585
- **WU ; WU.** *J. Biol. Chem.,* 1987, vol. 262, 4429-4432
- **HUCKED.** *Biochem Pharmacol,* 1990, vol. 40, 253-263
- **PLANK.** *Bioconjugate Chem,* 1992, vol. 3, 533-539
- **WOFFENDIN et al.** *Proc. Natl. Acad Sci. USA,* 1994, vol. 91 (24), 11581-11585
- **STRYER.** Biochemistry. W.H. Freeman, 1975, 236-240
- **SZOKA.** *Biochem Biophys Acta,* 1980, vol. 600, 1
- **BAYER.** *Biochem Biophys Acta,* 1979, vol. 550, 464
- **RIVNAY.** *Meth Enzymol,* 1987, vol. 149, 119
- **WANG.** *Proc Natl Acad Sci,* 1987, vol. 84, 7851
- *Plant (1989) Anal Biochem,* 1989, vol. 176, 420
- **HUG ; SLEIGHT.** *Biochim. Biophys. Acta,* 1991, vol. 1097, 1-17
- **STRAUBINGER.** *Meth. Enzymol.,* 1983, vol. 101, 512-527
- **FELGNER.** *Proc. Natl. Acad. Sci. USA,* 1987, vol. 84, 7413-7416
- **MALONE.** *Proc. Natl. Acad. Sci. USA,* 1989, vol. 86, 6077-6081
- **DEBS.** *J. Biol. Chem.,* 1990, vol. 265, 10189-10192
- **SZOKA.** *Proc. Natl. Acad. Sci. USA,* 1978, vol. 75, 4194-4198
- **STRAUBINGER.** *Meth. Immunol.,* 1983, vol. 101, 512-527
- **SZOKA.** *Proc. Natl. Acad Sci. USA,* 1978, vol. 75, 4194-4198
- **PAPAHADJOPOULOS.** *Biochim. Biophys. Acta,* 1975, vol. 394, 483
- **WILSON.** *Cell,* 1979, vol. 17, 77
- **DEAMER ; BANGHAM.** *Biochim. Biophys. Acta,* 1976, vol. 443, 629
- **OSTRO.** *Biochem. Biophys. Res. Commun.,* 1977, vol. 76, 836

- **FRALEY.** *Proc. Natl. Acad. Sci. USA,* 1979, vol. 76, 3348
- **ENOCH ; STRITTMATTER.** *Proc. Natl. Acad. Sci. USA,* 1979, vol. 76, 145
- **FRALEY.** *J. Biol. Chem.,* 1980, vol. 255, 10431
- **SZOKA ; PAPAHADJOPOULOS.** *Proc. Natl. Acad. Sci. USA,* 1978, vol. 75, 145
- **SCHAEFER-RIDDER.** *Science,* 1982, vol. 215, 166
- **BRESLOW.** *Annu Rev. Biochem,* 1985, vol. 54, 699
- **LAW.** *Adv. Exp Med. Biol.,* 1986, vol. 151, 162
- **CHEN.** *J Biol Chem,* 1986, vol. 261, 12918
- **KANE.** *Proc Natl Acad Sci USA,* 1980, vol. 77, 2465
- **UTERMANN.** *Hum Genet,* 1984, vol. 65, 232
- *Meth. Enzymol.,* 1986, 128
- **PITAS.** *J. Biochem.,* 1980, vol. 255, 5454-5460
- **MAHEY.** *J Clin. Invest,* 1979, vol. 64, 743-750
- **ATKINSON.** *Annu Rev Biophys Chem,* 1986, vol. 15, 403
- **RADDING.** *Biochim Biophys Acta,* 1958, vol. 30, 443
- **MEINKOTH ; WAHL.** *Anal. Biochem.,* 1984, vol. 138, 267-284
- **MATTEUCCI et al.** *J. Am. Chem. Soc.,* 1981, vol. 103, 3185
- **URDEA et al.** *Proc. Natl. Acad. Sci. USA,* 1983, vol. 80, 7461
- **AGRAWAL ; IYER.** *Curr Opin Biotechnol,* 1995, vol. 6, 12-19
- **AGRAWAL.** *TIBTECH,* 1996, vol. 14, 376-387
- **COREY.** *TIBTECH,* 1997, vol. 15, 224-229
- **BUCHARDT et al.** *TIBTECH,* 1993, vol. 11, 384-386
- **MULLIS et al.** *Meth. Enzymol.,* 1987, vol. 155, 335-350
- **R. D. FLEISCHMANN et al.** *Science,* 1995, vol. 269, 496-512
- **C. M. FRASER et al.** *Science,* 1995, vol. 270, 397-403
- **C. J. BULT et al.** *Science,* 1996, vol. 273, 1058-73
- **C. M. FRASER.** *Nature,* 1997, vol. 390, 580-586
- **J.-F. TOMB.** *Nature,* 1997, vol. 388, 539-547
- **H. P. KLENK et al.** *Nature,* 1997, vol. 390, 364-70
- **C. M. FRASER et al.** *Science,* 1998, vol. 281, 375-88
- **M. J. GARDNER et al.** *Science,* 1998, vol. 282, 1126-1132
- **K. E. NELSON et al.** *Nature,* 1999, vol. 399, 323-9
- **GAO et al.** *J. Immunol,* 1989, vol. 143, 3007
- **ROBERTS et al.** *AIDS Res Human Retroviruses,* 1996, vol. 12, 593
- **QUAKYI et al.** *Scand J Immunol,* 1992, 9
- **QUAKYI et al.** *Scand JImmunol,* 1992, vol. 11, 9